# EUROPEAN PATENT SPECIFICATION

(11) **EP 1 373 510 B1**
(45) Date of publication and mention of the grant of the patent: **19.11.2008**
(21) Application number: 01996610.0
(22) Date of filing: 16.11.2001
(51) Int. Cl.: C12N 15/29, C12N 15/12, C07K 14/415, C07K 14/435, A61K 39/35, A61K 39/36, C12N 5/10, A61P 37/08

(54) **MUTANT ALLERGENS**
MUTANTE ALLERGENE
ALLERGENES MUTANTS

(30) Priority: 16.11.2000 DK 200001718; 16.11.2000 US 249361 P; 14.06.2001 US 298170 P
(43) Date of publication of application: 02.01.2004
(73) Proprietor: Alk-Abelló A/S, 2970 Hørsholm (DK)
(72) Inventor: HOLM, Jens, DK-3480 Fredensborg (NO); IPSEN, Henrik, DK-3400 Hilleröd (DK); NEDERGAARD LARSEN, Jörgen, DK-3230 Grästed (DK); SPANGFORT, Michael, Dho, S-260 40 Viken (SE)
(74) Representative: Olsen, Lars Pallisgaard
(86) International application number: PCT/DK2001/000764
(87) International publication number: WO 2002/040676

(56) References cited:
- WO-A-98/43657
- WO-A-99/38978
- WO-A-99/47680
- MIRZA O ET AL: "Dominant epitopes and allergic cross-reactivity: complex formation between a Fab fragment of a monoclonal murine IgG antibody and the major allergen from birch pollen Bet v 1." JOURNAL OF IMMUNOLOGY (BALTIMORE, MD.: 1950) UNITED STATES 1 JUL 2000, vol. 165, no. 1, 1 July 2000 (2000-07-01), pages 331-338, XP002233501 ISSN: 0022-1767
- KING TE PIAO ET AL: "Structure and biology of stinging insect venom allergens." INTERNATIONAL ARCHIVES OF ALLERGY AND IMMUNOLOGY, vol. 123, no. 2, October 2000 (2000-10), pages 99-106, XP008014036 ISSN: 1018-2438
- PUNNONEN J: "MOLECULAR BREEDING OF ALLERGY VACCINES AND ANTIALLERGIC CYTOKINES" INTERNATIONAL ARCHIVES OF ALLERGY AND IMMUNOLOGY, vol. 121, no. 3, 2000, pages 173-182, XP000979180 ISSN: 1018-2438 cited in the application
- SPANGFORT MICHAEL D ET AL: "The potential use of recombinant allergens for immunotherapy." JOURNAL OF ALLERGY AND CLINICAL IMMUNOLOGY., vol. 105, no. 1 part 2, January 2000 (2000-01), page S169 XP002233502 Abstract 514 ISSN: 0091-6749 & 56th Annual Meeting of the American Academy of Allergy, Asthma and Immunology.;San Diego, California, USA; March 03-08, 2000
- LARSEN JORGEN NEDERGAARD ET AL: "Toward a unifying theory for the mechanism of Specific Allergy Vaccination." JOURNAL OF ALLERGY AND CLINICAL IMMUNOLOGY., vol. 105, no. 1 part 2, January 2000 (2000-01), page S311 XP002233503 Abstract 915 ISSN: 0091-6749 & 56th Annual Meeting of the American Academy of Allergy, Asthma and Immunology.;San Diego, California, USA; March 03-08, 2000
- SCHWEIMER K ET AL: "NMR spectroscopy reveals common structural features of the birch pollen allergen Bet v 1 and the cherry allergen Pru a 1" APPLIED MAGNETIC RESONANCE, (JUL 1999) VOL. 17, NO. 2-3, PP. 449-464., XP001010259
- FERREIRA F ET AL: "Modulation of IgE reactivity of allergens by site-directed mutagenesis: potential use of hypoallergenic variants for immunotherapy" FASEB JOURNAL, vol. 12, no. 2, 1 February 1998 (1998-02-01), pages 231-242, XP002085249 ISSN: 0892-6638 cited in the application
- SMITH A M AND CHAPMAN M D: "Localization of antigenic sites on Der p 2 using oligonucleotide-directed mutagenesis targeted to predicted surface residues" CLINICAL AND EXPERIMENTAL ALLERGY, vol. 27, no. 5, 1 May 1997 (1997-05-01), pages 593-599, XP002085251 ISSN: 0954-7894
- SPANGFORT M D ET AL: "Three-dimensional structure and epitopes of Bet v 1" INTERNATIONAL ARCHIVES OF ALLERGY AND IMMUNOLOGY, vol. 113, no. 1/03, 1997, pages 243-245, XP002085253 ISSN: 1018-2438
- SINGH MOHAN B ET AL: "Genetically engineered plant allergens with reduced anaphylactic activity." INTERNATIONAL ARCHIVES OF ALLERGY AND IMMUNOLOGY, vol. 119, no. 2, June 1999 (1999-06), pages 75-85, XP001010229 ISSN: 1018-2438
- HENRIKSEN ANETTE ET AL: "Major venom allergen of yellow jackets, Ves v 5: Structural characterization of a pathogenesis-related protein superfamily." PROTEINS, vol. 45, no. 4, 1 December 2001 (2001-12-01), pages 438-448, XP008014513 ISSN: 0887-3585

## Description

### FIELD OF THE INVENTION

The present invention relates to novel recombinant allergens, which are mutants of naturally occurring allergens. Also, the invention relates to a composition comprising a mixture of the novel recombinant mutant allergens. Further, the invention relates to a method of preparing such recombinant mutant allergens as well as to pharmaceutical compositions, including vaccines, comprising the recombinant mutant allergens. The present invention relates to methods of generating immune responses in a subject, vaccination or treatment of a subject as well as processes for preparing the compositions of the invention.

### BACKGROUND OF THE INVENTION

Genetically predisposed individuals become sensitised (allergic) to antigens originating from a variety of environmental sources, to the allergens of which the individuals are exposed. The allergic reaction occurs when a previously sensitised individual is re-exposed to the same or a homologous allergen. Allergic responses range from hay fever, rhinoconductivitis, rhinitis and asthma to systemic anaphylaxis and death in response to e.g. bee or hornet sting or insect bite. The reaction is immediate and can be caused by a variety of atopic allergens such as compounds originating from grasses, trees, weeds, insects, food, drugs, chemicals and perfumes.

However, the responses do not occur when an individual is exposed to an allergen for the first time. The initial adaptive response takes time and does usually not cause any symptoms. But when antibodies and T cells capable of reacting with the allergen have been produced, any subsequent exposure may provoke symptoms. Thus, allergic responses demonstrate that the immune response itself can cause significant pathological states, which may be life threatening.

The antibodies involved in atopic allergy belong primarily to immunoglobulins of the IgE class. IgE binds to specific receptors on the surface of mast cells and basophils. Following complex formation of a specific allergen with IgE bound to mast cells, receptor cross-linking on the cell surface results in signalling through the receptors and the physiological response of the target cells. Degranulation results in the release of i.a. histamine, heparin, a chemotactic factor for eosinophilic leukocytes, leukotrienes C4, D4 and E4, which cause prolonged constriction of the bronchial smooth muscle cells. The resulting effects may be systemic or local in nature.

The antibody-mediated hypersensitivity reactions can be divided into four classes, namely type I, type II, type III and type IV. Type I allergic reactions is the classic immediate hypersensitivity reaction occurring within seconds or minutes following antigen exposure. These symptoms are mediated by allergen specific IgE.

Commonly, allergic reactions are observed as a response to protein allergens present e.g. in pollens, house dust mites, animal hair and dandruff, venoms, and food products.

In order to reduce or eliminate allergic reactions, carefully controlled and repeated administration of allergy vaccines is commonly used. Allergy vaccination is traditionally performed by parenteral, intranasal, or sublingual administration in increasing doses over a fairly long period of time, and results in desensitisation of the patient. The exact immunological mechanism is not known, but induced differences in the phenotype of allergen specific T cells is thought to be of particular importance.

### Allergy vaccination

The concept of vaccination is based on two fundamental characteristics of the immune system, namely specificity and memory. Vaccination will prime the immune system of the recipient, and upon repeated exposure to similar proteins the immune system will be in a position to respond more rigorously to the challenge of for.example a microbial infection. Vaccines are mixtures of proteins intended to be used in vaccination for the purpose of generating such a protective immune response in the recipient. The protection will comprise only components present in the vaccine and homologous antigens.

Compared to other types of vaccination allergy vaccination is complicated by the existence of an ongoing immune response in allergic patients. This immune response is characterised by the presence of allergen specific IgE mediating the release of allergic symptoms upon exposure to allergens. Thus, allergy vaccination using allergens from natural sources has an inherent risk of side effects being in the utmost consequence life threatening to the patient.

Approaches to circumvent this problem may be divided in three categories. In practise measures from more than one category are often combined. First category of measures includes the administration of several small doses over prolonged time to reach a substantial accumulated dose. Second category of measures includes physical modification of the allergens by incorporation of the allergens into gel substances such as aluminium hydroxide. Aluminium hydroxide formulation has an adjuvant effect and a depot effect of slow allergen release reducing the tissue concentration of active allergen components. Third category of measures include chemical modification of the allergens for the purpose of reducing allergenicity, i.e. IgE binding.

The detailed mechanism behind successful allergy vaccination remains controversial. It is, however, agreed that T cells play a key role in the overall regulation of immune responses. According to current consensus the relation between two extremes of T cell phenotypes, Th1 and Th2, determine the allergic status of an individual. Upon stimulation with allergen Th1 cells secrete interleukines dominated by interferon-γ leading to protective immunity and the individual is healthy. Th2 cells on the other hand secrete predominantly interleukin 4 and 5 leading to IgE synthesis and eosinophilia and the individual is allergic. In vitro studies have indicated the possibility of altering the responses of allergen specific T cells by challenge with allergen derived peptides containing relevant T cell epitopes. Current approaches to new allergy vaccines are therefore largely based on addressing the T cells, the aim being to silence the T cells (anergy induction) or to shift the response from the Th2 phenotype to the Th1 phenotype.

### Antibody-binding epitopes (B-cell epitopes)

X-ray crystallographic analyses of F_{ab}-antigen complexes has increased the understanding of antibody-binding epitopes. According to this type of analysis antibody-binding epitopes can be defined as a section of the surface of the antigen comprising atoms from 15-25 amino acid residues, which are within a distance from the atoms of the antibody enabling direct interaction. The affinity of the antigen-antibody interaction can not be predicted from the enthalpy contributed by van der Waals interactions, hydrogen bonds or ionic bonds, alone. The entropy associated with the almost complete expulsion of water molecules from the interface represent an energy contribution similar in size. This means that perfect fit between the contours of the interacting molecules is a principal factor underlying antigen-antibody high affinity interactions.

In WO 97/30150 (ref. 1), a population of protein molecules is claimed, which protein molecules have a distribution of specific mutations in the amino acid sequence as compared to a parent protein. From the description, it appears that the invention is concerned with producing analogues which are modified as compared to the parent protein, but which are taken up, digested and presented to T cells in the same manner as the parent protein (naturally occurring allergens). Thereby, a modified T cell response is obtained. Libraries of modified proteins are prepared using a technique denoted PM (Parsimonious Mutagenesis).

In WO 92/02621 (ref. 2), recombinant DNA molecules are described, which molecules comprise a DNA coding for a polypeptide having at least one epitope of an allergen of trees of the order *Fagales*, the allergen being selected from *Aln g* 1, *Cor a* 1 and *Bet v* 1. The recombinant molecules described herein do all have an amino acid sequence or part of an amino acid sequence that corresponds to the sequence of a naturally occurring allergen.

WO 90/11293 (ref. 3) relates i.a. to isolated allergenic peptides of ragweed pollen and to modified ragweed pollen peptides. The peptides disclosed therein have an amino acid sequence corresponding either to the sequence of the naturally occurring allergen or to naturally occurring isoforms thereof.

### Chemical modification of allergens

Several approaches to chemical modification of allergens have been taken. Approaches of the early seventies include chemical coupling of allergens to polymers, and chemical cross-linking of allergens using formaldehyde, etc., producing the so-called 'allergoids'. The rationale behind these approaches was random destruction of IgE binding epitopes by attachment of the chemical ligand thereby reducing IgE-binding while retaining immunogenicity by the increased molecular weight of the complexes. Inherent disadvantages of `allergoid' production are linked to difficulties in controlling the process of chemical cross-linking and difficulties in analysis and standardisation of the resulting high molecular weight complexes. 'Allergoids' are currently in clinical use and due to the random destruction of IgE binding epitopes higher doses can be administered as compared to conventional vaccines, but the safety and efficacy parameters are not improved over use of conventional vaccines.

More recent approaches to chemical modification of allergens aim at a total disruption of the tertiary structure of the allergen thus eliminating IgE binding assuming that the essential therapeutic target is the allergen specific T cell. Such vaccines contain allergen sequence derived synthetic peptides representing minimal T cells epitopes, longer peptides representing linked T cells epitopes, longer allergen sequence derived synthetic peptides representing regions of immunodominant T cell epitopes, or allergen molecules cut in two halves by recombinant technique. Another approach based on this rationale has been the proposal of the use of "low IgE binding" recombinant isoforms. In recent years it has become clear that natural allergens are heterogeneous containing isoallergens and variants having up to approximately 25% of their amino acids substituted. Some recombinant isoallergens have been found to be less efficient in IgE binding possibly due to irreversible denaturation and hence total disruption of tertiary structure.

### In vitro mutagenesis and allergy vaccination

Attempts to reduce allergenicity by *in vitro* site directed mutagenesis have been performed using several allergens including Der f 2 (Takai *et al*, ref. 4), Der p 2 (Smith *et al*, ref. 5), a 39 kDa *Dermatophagoides farinae* allergen (Aki *et al,* ref. 6), bee venom phospholipase A2 (Förster *et al*, ref. 7), Ara h 1 (Burks *et al,* ref. 8), Ara h 2 (Stanley *et al*, ref. 9), *Bet v* 1 (Ferreira *et al,* ref. 10 and 11), birch profilin (Wiedemann *et al,* ref. 12), and Ory s 1 (Alvarez *et al,* ref. 13).

The rationale behind these approaches, again, is addressing allergen specific T cells while at the same time reducing the risk of IgE mediated side effects by reduction or elimination of IgE binding by disruption of the tertiary structure of the recombinant mutant allergen. The rationale behind these approaches does not include the concept of dominant IgE binding epitopes and it does not include the concept of initiating a new protective immune response which also involves B-cells and antibody generation.

The article by Ferreira *et al* (ref. 11) describes the use of site directed mutagenesis for the purpose of reducing IgE binding.. Although the three-dimensional structure of *Bet v* 1 is mentioned in the article the authors do not use the structure for prediction of solvent exposed amino acid residues for mutation, half of which have a low degree of solvent exposure. Rather they use a method developed for prediction of functional residues in proteins different from the concept of structure based identification of conserved surface areas described here. Although the authors do discuss conservation of α-carbon backbone tertiary structure this concept is not a part of the therapeutic strategy but merely included to assess *in vitro* IgE binding. Furthermore, the evidence presented is not adequate since normalisation of CD-spectra prevents the evaluation of denaturation of a proportion of the sample, which is a common problem. The therapeutic strategy described aim at inducing tolerance in allergen specific T cells and initiation of a new immune response is not mentioned.

The article by Wiedemann *et al*. (ref. 12) describes the use of site directed mutagenesis and peptide synthesis for the purpose of monoclonal antibody epitope characterisation. The authors have knowledge of the tertiary structure of the antigen and they use this knowledge to select a surface exposed amino acid for mutation. The algorithm used can be said to be opposite to the one described by the present inventors since an amino acid differing from homologous sequences is selected. The study demonstrates that substitution of a surface exposed amino acid has the capacity to modify the binding characteristics of a monoclonal antibody, which is not surprising considering common knowledge. The experiments described are not designed to assess modulation in the binding of polyclonal antibodies such as allergic patients' serum IgE. One of the experiments contained do apply serum IgE and although this experiment is not suitable for quantitative assessment, IgE binding does not seem to be affected by the mutations performed.

The article by Smith *et al.* (ref. 5) describes the use of site directed mutagenesis for the purpose of monoclonal antibody epitope mapping and reduction of IgE binding. The authors have no knowledge of the tertiary structure and make no attempt to assess the conservation of α-carbon backbone tertiary structure. The algorithm used does not ensure that amino acids selected for mutation are actually exposed to the molecular surface. Only one of the mutants described lead to a substantial reduction in IgE binding. This mutant is deficient in binding of all antibodies tested indicating that the tertiary structure is disrupted. The authors do not define a therapeutic strategy and initiation of a new immune response is not mentioned.

The article by Colombo *et al.* (ref. 14) describes the study of an IgE binding epitope by use of site directed mutagenesis and peptide synthesis. The authors use a three dimensional computer model structure based on the crystal structure of a homologous protein to illustrate the presence of the epitope on the molecular surface. The further presence of an epitope on a different allergen showing primary structure homology is addressed using synthetic peptides representing the epitope. The therapeutic strategy is based on treatment using this synthetic peptide representing a monovalent IgE binding epitope. Conserved surface areas between homologous allergens as well as the therapeutic concept of initiating a new protective immune response are not mentioned.

The article by Spangfort *et al*. (ref. 15) describes the three-dimensional structure and conserved surface exposed patches of the major birch allergen. The article does not mention major IgE binding epitopes nor site directed mutagenesis, neither is therapeutic application addressed.

In none of the studies described above is.IgE binding reduced by substitution of surface exposed amino acids while conserving α-carbon backbone tertiary structure. The rationale behind above-mentioned approaches does not include the concept of dominant IgE binding epitopes and it does not include the therapeutic concept of initiating a new protective immune response.

WO 99/47680 discloses the introduction of artificial amino acid substitutions into defined critical positions while retaining the α-carbon backbone tertiary structure of the allergen. In particular, WO 99/47680 discloses a recombinant allergen, which is a non-naturally occurring mutant derived from a naturally occurring allergen, wherein at least one surface-exposed, conserved amino acid residue of a B cell epitope is substituted by another residue which does not occur in the same position in the amino acid sequence of any known homologous protein within the taxonomic order from which said naturally occurring allergen originates, said mutant allergen having essentially the same α-carbon backbone tertiary structure as said naturally occurring allergen, and the specific IgE binding to the mutated allergen being reduced as compared to the binding to said naturally occurring allergen.

The recombinant allergen disclosed in WO 99/47680 is obtainable by a) identifying amino acid residues in a naturally occurring allergen which are conserved with more than 70% identity in all known homologous proteins within the taxonomic order from which said naturally occurring allergen originates, b) defining at least one patch of conserved amino acid residues being coherently connected over at least 400 Å² of the surface of the three-dimensional of the allergen molecule as defined by having a solvent accessibility of at least 20%, said at least one patch comprising at least one B cell epitope, and c) substituting at least one amino acid residue in said at least one patch by another amino acid being non-conservative in the particular position while essentially preserving the overall α-carbon backbone tertiary structure of the allergen molecule.

### BRIEF DESCRIPTION OF THE FIGURES

Figure 1 shows mutant-specific oligonucleotide primers used for *Bet v* 1 mutant number 1. Mutated nucleotides are underlined.
Figure 2 shows two generally applicable primers (denoted "all-sense" and "all non-sense"), which were synthesised and used for all mutants.
Figure 3 shows the DNA and amino acid sequence of the naturally occurring allergen *Bet v* 1 as well as a number of *Bet v* 1 mutations.
Figure 4 shows the inhibition of the binding of biotinylated recombinant *Bet v* 1 to serum IgE from a pool of allergic patients by non-biotinylated *Bet v* 1 and by *Bet v* 1 Glu45Ser mutant.
Figure 5 shows the inhibition of the binding of biotinylated recombinant *Bet v* 1 to serum IgE from a pool of allergic patients by non-biotinylated *Bet v* 1 and by *Bet v* 1 mutant Asn28Thr+Lys32Gln.
Figure 6 shows the inhibition of the binding of biotinylated recombinant *Bet v* 1 to serum IgE from a pool of allergic patients by non-biotinylated *Bet v* 1 and by *Bet v* 1 Pro108Gly mutant.
Figure 7 shows the inhibition of the binding of biotinylated recombinant *Bet v* 1 to serum IgE from a pool of allergic patients by non-biotinylated *Bet v* 1 and by *Bet v* 1 Glu60Ser mutant.
Figure 8 shows the CD spectra of recombinant and Triple-patch mutant, recorded at close to equal concentrations.
Figure 9 shows the inhibition of the binding of biotinylated recombinant *Bet v* 1 to serum IgE from a pool of allergic patients by non-biotinylated *Bet v* 1 and by *Bet v* 1 Triple-patch mutant.
Figure 10 shows solvent accessibility of individually aligned antigen 5 residues and alignment of *Vespula* antigen 5 sequences (left panel). On the right panel of Figure 10 is shown the molecular surface of antigen 5 with conserved areas among *Vespula* antigen 5:*s*.
Figure 11 shows the sequence of the primer corresponding to the amino terminus of *Ves v* 5 derived from the sense strand. The sequence of the downstream primer is derived from the non-sense strand.
Figure 12 shows two generally applicable primers (denoted "all sense" and "all non-sense", which were synthesised and used for all mutants.
Figure 13 shows the DNA and amino acid sequence of the naturally occurring allergen *Ves v* 5 as well as two *Ves v 5* mutations.
Figure 14 shows the inhibition of the binding of biotinylated recombinant *Ves v* 5 to serum IgE from a pool of allergic patients by non-biotinylated *Ves v* 5 and by *Ves v* 5 Lys72Ala mutant.
Figure 15 shows a theoretical model of the reaction between an allergen and mast cells by IgE cross-linking.
Figure 16 shows the DNA and amino acid sequence of the naturally occurring allergen *Der p* 2.
Figure 17 shows schematically the primers used to create the mutations. (I) shows the sense and antisense primers. (II) shows the final recombinant protein harbouring mutations at the indicated positions.
Figure 18 shows an illustration of the construction of Bet v 1 mutants and a listing of the primers used. The mutants contain from five to nine amino acids.
Figure 19 shows introduced point mutations at the surface of Bet v 1 (2628) and Bet v 1 (2637). In mutant Bet v 1 (2628), five primary mutations were introduced in one half of Bet v 1 leaving the other half unaltered. In mutant Bet v 1 (2637), five primary and three secondary mutations were introduced in the other half, leaving the first half unaltered.
Figure 20 shows the circular dichroism (CD) spectra of recombinant Bet v 1.2801 (wild type) and the Bet v 1 (2637) mutant recorded at nearly identical concentrations.
Figure. 21 shows the inhibition of the binding of biotinylated recombinant Bet v 1.2801 (wild type) to serum IgE from a pool of allergic patients by non-biotinylated Bet v 1.2801 and by Bet v 1 (2628), Bet v 1 (2637), and a 1:1 mix of Bet v 1 (2628) and Bet v1 (2637).
Figure 22 shows histamine release in human basophil cells of Bet v 1.2801 (wild type), Bet v 1 (2628), and Bet v 1 (2637).
Figure 23 shows histamine release in human basophil cells of Bet v 1.2801 (wild type), Bet v 1 (2628), and Bet v 1 (2637).
Figure 24 shows point mutations at the surface of Bet v1 (2744).
Figure 25 shows point mutations at the surface of Bet v 1 (2753).
Figure 26 shows point mutations at the surface of Bet v 1 (2744) and Bet v 1 (2753).
Figur 27 shows circular dichroism (CD) spectra of Bet v 1.2801 (wild type) and Bet v 1 (2744), recorded at nearly equal concentrations.
Figur 28 shows histamine release in human basophil cells of Bet v 1.2801 (wild type), and mutant Bet v 1 (2744).
Figur 29 shows histamine release in human basophil cells of Bet v 1.2801 (wild type), and mutant Bet v 1 (2744).
Figur 30 shows point mutations at the surface of Bet v 1 (2733).
Figure 31 shows primers used for site-directed mutagenesis of Der p 2.
Figure 32 shows a sequence alignment of Der p 2 with other group 2 house dust mite allergens.
Figure 33 shows surface contours of Der p 2 from four different angles.
Figure 34 shows surface contours of a Der p 2 mutant from four different angles.
Figure 35A and B shows a sequence alignment of Der p 1 with other group 1 house dust mite allergens.
Figure 36 shows surface contours of Der p 1 from four different angles.
Figure 37 shows surface contours of a Der p 1 mutant from four different angles.
Figure 38A-D shows a sequence alignment of Phl p 5 with other group 5 grass allergens.
Figure 39A and B shows surface contours of Phl p 5 Model A and Model B, respectively, from four different angles.
Figure 40A and B shows surface contours of a Phl p 5 mutant Model A and B, respectively, from four different angles.
Figure 41 shows the proliferation of Peripheral Blood Lymphocytes expressed as Stimulation Index (SI) for various Bet v 1 preparations.
Figure 42-44 show the cytokine profile of T cells stimulated with various Bet v preparations. Figure 42 shows a patient with a Th0 profile, Figure 43 a Th1 profile and Figure 44 a Th2 profile.

### OBJECT OF THE INVENTION

### Rationale behind the present invention

The current invention is based on a unique rationale. According to this rationale the mechanism of successful allergy vaccination is not an alteration of the ongoing Th2-type immune response, but rather a parallel initiation of a new immune response involving tertiary epitope recognition by B-cells and antibody formation. It is believed that this new immune response is partly a Th1-type immune response. This model is supported by the observation that levels of specific IgE are unaffected by successful vaccination treatment, and that successful treatment is often accompanied by a substantial rise in allergen specific IgG4. In addition, studies of nasal biopsies before and after allergen challenge do not show a reduction in T cells with the Th2-like phenotype, but rather an increase in Th1-like T cells are observed. When the vaccine (or pharmaceutical compositions) is administered through another route than the airways, it is hypothesised, that the new immune response evolves in a location physically separated from the ongoing Th2 response thereby enabling the two responses to exist in parallel.

Another important aspect of the immunological system is the assertion of the existence of so-called dominant IgE binding epitopes. It is proposed that these dominant IgE binding epitopes are constituted by tertiary structure dependent coherent surface areas large enough to accommodate antibody binding and conserved among isoallergens, variants, and/or homologous allergens from related species. The existence of cross-reactive IgE capable of binding similar epitopes on homologous allergens is supported by the clinical observation that allergic patients often react to several closely related species, e.g. alder, birch, and hazel, multiple grass species, or several species of the house dust mite genus Dermatophagoides. It is furthermore supported by laboratory experiments demonstrating IgE cross-reactivity between homologous allergens from related species and the capacity of one allergen to inhibit the binding of IgE to homologous allergens (Ipsen *et al*. 1992, ref. 16). It is well known that exposure and immune responses are related in a dose dependent fashion. Based on the combination of these observations it is hypothesised that conserved surface areas are exposed to the immune system in higher doses than non-conserved surface areas resulting in the generation of IgE antibodies with higher affinities, hence the term 'dominant IgE binding epitopes'.

According to this rationale it is essential that the allergen has an α-carbon backbone tertiary structure which essentially is the same as that of the natural allergen, thus ensuring conservation of the surface topology of areas surrounding conserved patches representing targets for mutagenesis aimed at reducing IgE binding. By fulfilling these criteria the allergen has the potential to be administered in relatively higher doses improving its efficacy in generating a protective immune response without compromising safety.

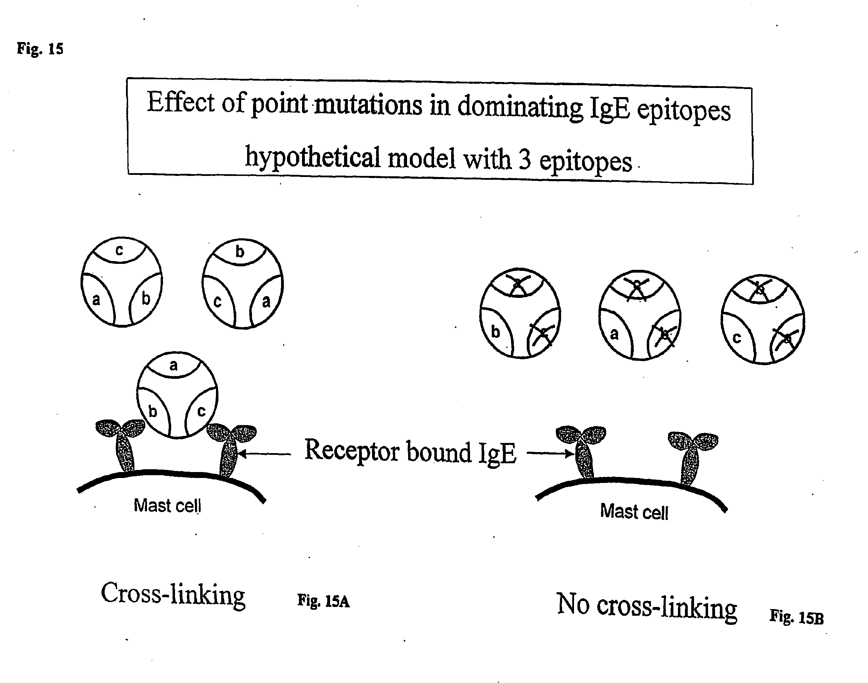

Furthermore, the invention is based on the finding that allergic symptoms are triggered by the cross-linking of allergen with two specific IgE's bound to the surface of effector cells, i.e. mast cells and basophils, via the high affinity IgE receptor, FceRI. For illustration, we refer to Fig. 15, which depicts a theoretical model of an allergen with IgE binding epitopes. Induction of mediator release from the mast cell and hence allergic symptoms is effected by allergen-mediated cross-linking of IgE bound to the surface of the mast cell, cf. Fig 15A. In the situation shown in Fig. 15B two of the epitopes have been mutated so as to reduce their IgE binding ability, and hence the allergen-mediated cross-linking is prevented. In this connection it should be noted that allergens usually comprise more than three B cell epitopes. However, from the theoretical situation depicted in Fig. 15 it may be assumed that the more epitopes, which have been mutated so as to eliminate or reduce their IgE binding ability, the lower the risk of allergen-mediated cross-linking and resulting allergic symptoms.

However, in order for a mutated allergen to be able to raise the new immune response, including the IgG response, the allergen must comprise at least one intact epitope. Preferably, the intact epitope is a dominant epitope, since such a mutated allergen will provide an improved protection when used for vaccination.

In conclusion, the inventive idea of the present invention is based on the recognition that a mutated allergen having IgE binding reducing mutations in multiple B cell epitopes, and at least one intact epitope, would on the one hand reduce the allergen-mediated cross-linking and on the other hand allow the raising of an IgG response with a binding ability competitive with that of IgE. Thus, the said mutated allergen would constitute a highly advantageous allergen in that the risk of anaphylactic reactions would be strongly reduced.

Also, the present invention is based on the recognition that a vaccine comprising a mixture of different such mutated allergens, wherein ideally many or all epitopes are represented as intact, would be equally efficient in its ability to induce protection against allergic symptoms as the natural occurring allergen from which the mutated allergens are derived.

### SUMMARY OF THE INVENTION

The present invention relates to the introduction of artificial amino acid substitutions into a number of defined critical positions, i.e. IgE binding epitopes, with the object of reducing the specific IgE binding capability of each mutated epitope.

The invention provides claim 1.

Without being bound by theory it is believed that the B cell epitopes can be distributed over almost the entire surface of the allergen. Furthermore, there is experimental evidence that at least some epitopes constitute a part of a cluster of epitopes comprising a large number of overlapping epitopes. Therefore, the theoretical basis for the present invention is that any surface-exposed amino acid constitutes a potential site of mutation, which may result in a reduced capability to bind specific IgE.

Accordingly, the primary mutations are defined by their location in respect to each other, i.e. they are spaced apart, to ensure that they are mutations in separate clusters of epitopes.

The present invention also provides a composition comprising two or more recombinant mutant allergen variants according to claim 1, wherein each variant is defined by having at least one principal mutation, which is absent in at least one of the other variants, wherein for each variant no secondary mutation is present within a radius of 15 Å from each absent primary mutation. The composition preferably comprises 2-12, more preferably 3-10, more preferably 4-8 and most preferably 5-7 variants.

The present invention also provides a method of preparing the recombinant allergen according to claim 1, characterised in
a) identifying a number of amino acid residues in a naturally occurring allergen, which has a solvent accessibility of at least 20 %;
b) selecting at least four of the identified amino acid residues in such a manner that each selected amino acid is spaced from each other selected amino acid by at least 15 Å, and that the selected amino acids are placed in such a manner that at least one circular surface region with a area of 800 Å² comprises no selected amino acid; and
c) effecting for each of the selected amino acids a primary mutation, which reduce the specific IgE binding capability of the mutated allergen as compared to the binding capability of the said naturally occurring allergen, wherein each primary mutation is a substitution of a selected amino acid residue with another amino acid, which does not occur in the same position in the amino acid sequence of any known homologous protein within the taxonomic species from which said naturally occurring allergen originates.

In an alternative aspect the invention relates to a method of preparing a recombinant allergen according to the invention, characterised in that the allergen is produced from a DNA sequence obtained by DNA shuffling (molecular breeding) of the DNA encoding the corresponding naturally occurring.

Furthermore, the invention relates to a recombinant allergen according to claim 1 for use as a pharmaceutical.

Also, the invention relates to use of the recombinant allergen according to claim 1 for preparing a pharmaceutical for preventing and/or treating allergy.

Furthermore, the invention relates to the composition according to claim 39 for use as a pharmaceutical.

Also, the invention relates to the use of a composition according to claim 39 for preparing a pharmaceutical for preventing and/or treating allergy.

Further, the invention relates to a pharmaceutical composition, characterised in that it comprises a recombinant allergen according to claim 1 or a composition according to claim 39, optionally in combination with a pharmaceutically acceptable carrier and/or excipient, and optionally an adjuvant. The pharmaceutical composition according to the invention may be in the form of a vaccine against allergic reactions elicited by a naturally occurring allergen in patients suffering from allergy.

Also, the invention relates to a method of generating an immune response in a subject comprising administering to the subject a recombinant allergen according to claim 1, a composition according to claim 39 or a pharmaceutical composition according to claim 43-44 or 48.

Further, the invention relates to vaccination or treatment of a subject comprising administering to the subject a recombinant allergen according to claim 1, a composition according to claim 39 or a pharmaceutical composition according to claim 43-44 or 48.

Also, the invention relates to a process for preparing a pharmaceutical composition according to claim 43 or 44 comprising mixing a recombinant allergen according to claim 1 or a composition according to claim 39 with pharmaceutically acceptable substances and/or excipients.

Further, the invention relates to a pharmaceutical composition obtainable by the process according to claim 45.

Also, the invention relates to a method for the treatment, prevention or alleviation of allergic reactions in a subject comprising administering to a subject a recombinant allergen according to claim 1, a composition according to claim 39 or a pharmaceutical composition according to claims 43-44 or 48.

Further, the invention relates to a DNA sequence encoding an allergen according to invention, a derivative thereof, a partial sequence thereof, a degenerated sequence thereof or a sequence, which hybridises thereto under stringent conditions, wherein said derivative, partial sequence, degenerated sequence or hybridising sequence encodes a peptide having at least one B cell epitope.

Also, the invention relates to an expression vector comprising the DNA according to the invention.

Furthermore, the invention relates to a host cell comprising the expression vector according to the invention.

Additionally, the invention relates to a method of producing a recombinant mutant allergen comprising the step of cultivating the host cell according to the invention.

Finally, the invention relates to a recombinant allergen according to the invention or encoded by the DNA sequence according to the invention comprising at least one T cell epitope capable of stimulating a T cell clone or T cell line specific for the naturally occurring allergen. The mutants according to invent should preferable be able to stimulate allergen specific T-cell lines in a similar manner/degree as measured by the T-cell stimulation index.

### DETAILED DESCRIPTION OF THE INVENTION

In a preferred embodiment of the invention, the primary mutations are spaced 20 Å, preferably 25 Å and most preferably 30 Å.

It is believed that an allergen comprises a number of potential binding regions for specific IgE's, wherein each region approximately has a size of 800 Å², each surface region comprising a large number of overlapping epitopes. Thus, an allergen has a number of potential primary mutations of the surface area divided by 800 Å²

Preferably, the recombinant allergen according to the invention comprises from 5 to 20, preferably from 6 to 15, more preferably from 7 to 12, and most preferably from.8 to 10 primary mutations.

In a preferred embodiment of the invention, the surface region comprising no mutation has an area of 700 Å², preferably 600 Å², more preferably 500 Å² and most preferably 400 Å².

In a preferred embodiment of the invention, the recombinant allergen comprises a number of secondary mutations, which each reduce the specific IgE binding capability of the mutated allergen as compared to the binding capability of the said naturally occurring allergen, wherein each secondary mutation is a substitution of one surface-exposed amino acid residue with another residue, which does not occur in the same position in the amino acid sequence of any known homologous protein within the taxonomic species from which said naturally occurring allergen originates,
wherein the secondary mutations are placed outside the said circular region.

The secondary mutations may be located close to a primary mutation, i.e. a secondary mutation may well be an additional mutation for the same epitope, which is mutated by the primary mutation.

In a preferred embodiment of the invention, at least one of the surface-exposed amino acids to be substituted in the naturally occurring allergen has a solvent accessibility of above 20 %, preferably above 30 %, more preferably above 40 % and most preferably above 50 %.

In another preferred embodiment of the invention, at least one of the surface-exposed amino acids to be substituted in the naturally occurring allergen is conserved with more than 70 %, preferably 80 % and most preferably 90 % identity in all known homologous proteins within the species from which said naturally occurring allergen originates.

Preferably, the recombinant allergen according to invention essentially has the same α-carbon backbone tertiary structure as said naturally occurring allergen.

When comparing the α-carbon backbone tertiary structures of the mutant and the naturally occurring allergen molecules, the average root mean square deviation of the atomic coordinates is preferably below 2Å.

In a preferred embodiment of the recombinant allergen of the invention, each amino acid residue to be incorporated into the mutant allergen does not occur in the same position in the amino acid sequence of any known homologous protein within the taxonomic genus, preferably the subfamily, more preferably the family, more preferably the superfamily, more preferably the legion, more preferably the suborder and most preferably the order from which said naturally occurring allergen originates.

In a preferred embodiment of the invention the recombinant mutant allergen according to the invention is a non-naturally occurring allergen.

Specific IgE binding to the mutated allergen is preferably reduced by at least 5%, preferably at least 10% in comparison to naturally-occurring isoallergens or similar recombinant proteins in an immuno assay with sera from source-specific IgE reactive allergic patients or pools thereof.

Another way of assessing the reduced IgE binding and the reduced ability of mediating cross-linking of the mutant are the capability of the mutant to initiate Histamine Release (HR). The release of Histamine can be measured in several Histamine releasing assays. The reduced Histamine release of the mutants originates from reduced affinity toward the specific IgE bound to the cell surface as well as their reduced ability to facilitate cross-linking. HR is preferably reduced by 5-100%, more preferably 25-100%, more preferably 50-100% and most preferably 75-100% for the mutants of the invention in comparison to the naturally occurring allergens.

Typically, the circular surface region with an area of 800 Å² comprising no mutation comprises atoms of 15-25 amino acid residues.

A preferred recombinant allergen according to the invention is characterised in that the surface-exposed amino acid residues are ranked with respect to solvent accessibility, and that one or more amino acids among the more solvent accessible ones are substituted.

A further preferred recombinant allergen according to the invention is characterised in that the surface-exposed amino acid residues are ranked with respect to degree of conversation in all known homologous proteins within the species from which said naturally occurring allergen originates, and that one or more amino acids among the more conserved ones are substituted.

Preferably, the recombinant allergen according to the invention comprises from 1 to 4 secondary mutations per primary mutation.

A preferred embodiment of the invention is characterised in that one or more of the substitutions is carried out by site-directed mutagenesis.

Another preferred embodiment of the invention is characterised in that one or more of the substitutions is carried out by random mutagenesis.

A further preferred embodiment of the invention is characterised in that one or more of the substitutions is carried out by DNA shuffling.

Recombinant allergens according to the invention may suitably be a mutant of an inhalation allergen originating i.a. from trees, grasses, herbs, fungi, house dust mites, cockroaches and animal hair and dandruff. Important pollen allergens from trees, grasses and herbs are such originating from the taxonomic orders of *Fagales, Oleales* and *Pinales* including i.a. birch (*Betula*), alder (*Alnus*)*,* hazel (*Corylus*)*,* hornbeam (*Carpinus*) and olive (*Olea*) *,* the order of *Poales* including i.a. grasses of the genera *Lolium, Phleum, Poa, Cynodon, Dactylis* and *Secale,* the orders of *Asterales* and *Urticales* including i.a. herbs of the genera *Ambrosia* and *Artemisia.* Important inhalation allergens from fungi are i.a. such originating from the genera *Alternaria* and *Cladosporium.* Other important inhalation allergens are those from house dust mites of the genus *Dermatophagoides,* those from cockroaches and those from mammals such as cat, dog and horse. Further, recombinant allergens according to the invention may be mutants of venom allergens including such originating from stinging or biting insects such as those from the taxonomic order of *Hymenoptera* including bees (superfamily Apidae), wasps (superfamily Vespidea), and ants (superfamily Formicoidae).

Specific allergen components include e.g. *Bet v* 1 (*B. verrucosa,* birch), *Aln g* 1 (*Alnus glutinosa*, alder), *Cor* a 1 (*Corylus avelana,* hazel) and *Car b* 1 (*Carpinus* betulus, hornbeam) of the *Fagales* order. Others are *Cry j* 1 (*Pinales*)*, Amb a* 1 and 2, Art *v* 1 (*Asterales*)*, Par j* 1 (*Urticales*)*, Ole e* 1 (*Oleales*), *Ave e* 1, *Cyn d* 1, *Dac g* 1, *Fes p* 1, *Hol 1* 1, *Lol p* 1 and 5, *Pas n* 1, *Phl p* 1 and 5, *Poa p* 1, 2 and 5, *Sec c* 1 and 5, and *Sor h* 1 (various grass pollens), *Alt a* 1 and *Cla h* 1 (fungi), *Der f* 1 and *2, Der p* 1 and 2 (house dust mites, *D. farinae* and *D*. *pteronyssinus,* respectively), *Lep d* 1 and 2 (Lepidoglyphus destructor; storage mite), *Bla g* 1 and 2, Per a 1 (cockroaches, *Blatella germanica* and *Periplaneta* americana, respectively), *Fel d* 1 (cat), *Can f* 1 (dog), *Equ c* 1, 2 and 3 (horse), *Apis m* 1 and 2 (honeybee), *Ves v* 1, 2 and 5, *Pol a* 1, 2 and 5 (all wasps) and *Sol i* 1, 2, 3 and 4 (fire ant).

In one embodiment, the recombinant allergen is a mutant of *Bet v* 1. Amino acids potentially suitable for substitution comprise amino acids V2, D72, E87, K-129, E-60, N-47, K-65, P-108, N-159, D-93, K-123, K-32, D-125, R-145, D-109, E-127, Q-36, E-131, L-152, E-6, E-96, D-156, P-63, H-76, E-8, K-134, E-45, T-10, V-12, K-20, S-155, H-126, P-50, N-78, K-119, V-2, L-24, E-42, N-4, A-153, I-44, E-138, G-61, A-130, R-70, N-28, P-35, S-149, K-103, Y-150, H-154, N-43, A-106, K-115, P-14, Y-5, K-137, E-141, E-87 and E-73. One or more of the primary and secondary substitutions may be selected from the group consisting of V2F, V2L, V2I, V2M, Y5V, T10P, T10A, K20N, D25E, N28T, K32Q, Q36A, Q36K, E42S, E45S, N47S, K55N, K65N, D72H, D72Q, D72N, T77A, N78K, E87G, E96L, K97S, K103V, P108G, D109N, K123I, D125Y, K129N, K134E, R145E, S149R, S149T, D156H and +160N, wherein + means that an additional amino acid is incorporated.

Examples of *Bet v* 1 mutants according to the present invention are as follows (parentheses, when used, indicate primary and secondary mutations):
Mutant A:
   (Asn28Thr, Lys32Gln), (Asn78Lys, Lys103Val), Arg145Glu, (Asp156His, +160Asn).
Mutant B:
   Tyr5Val, Glu42Ser, Glu45Ser, Asn78Lys, Lysl03Val, Lys123Ile Lys134Glu, Asp156His.
Mutant 2595 (Example 2):
   N28T, K32Q, E45S, P108G
Mutant 2628 (Example 4):
   Tyr5Val, Glu45Ser, Lys65Asn, Lys97Ser, Lys134Glu.
Mutant 2637 (Example 4):
   Ala16Pro, (Asn28Thr, Lys32Gln), Lys103Thr, Pro108Gly, (Leu152Lys, Ala153Gly, Ser155Pro).
Mutant 2724:
   N28T, K32Q, N78K, K103V, P108G, R145E, D156H, +160N.
Mutant 2733 (Example 4):
   (Tyr5Val, Lys134Glu), (Asn28Thr, Lys32Gln), Glu45Ser, Lys65Asn, (Asn78Lys, Lys103Val), Lys97Ser, Pro108Gly Arg145Glu, (Asp156His, +160Asn)
Mutant 2744: (Tyr5Val, Lys134Glu), (Glu42Ser, Glu45Ser),
   (Asn78Lys, Lys103Val), Lys123Ile, (Asp156His, +160Asn).
Mutant 2753 (Example 4):
   (Asn28Thr, Lys32Gln), Lys65Asn, (Glu96Leu, Lys97Ser), (Pro108Gly, Asp109Asn), (Asp125Tyr, Glu127Ser), Arg145Glu.
Mutant 2744 + 2595:
   Y5V, N28T, K32Q, E42S, E45S, N78K, K103V, P108G, K123I, K134E, D156H, +160N.
Mutant 2744 + 2628:
   Y5V, E42S, E45S, K65N, N78K, K97S, K103V, K123I, K134E, D156H, +160N.
Mutant 2744 + 2595 + 2628:
   Y5V, N28T, K32Q, E42S, E45S, K65N, N78K, K97S, K103V, P108G, K123I, K134E, D156H, +160N.

Furthermore, all of the above mutants comprising one or more of the following substitutions: V2F, V2L, V2I, V2M, T10A, K20N, Q36A or Q36K, D72H, D72Q, D72N, E87G, K129N and S149R or S149T.

In another embodiment, the recombinant allergen is derived from a venom allergen from the taxonomic order of Vespidae, Apidae and Formicoidae.

In a further embodiment, the recombinant allergen is derived from *Ves v* 5. Amino acids potentially suitable for substitution comprise amino acids Amino acids potentially suitable for substitution comprise amino acids K-16, K-185, K-11, K-44, K-210, R-63, K-13, F-6, K-149, K-128, E-184, K-112, F-157, E-3, K-29, N-203, N-34, K-78, K-151, L-15, L-158, Y-102, W-186, K-134, D-87, K-52, T-67, T-125, K-150, Y-40, Q-48, L-65, K-81, Q-101, Q-208, K-144, N-8, N-70, H-104, Q-45, K-137, K-159, E-205, N-82, A-111, D-131, K-24, V-36, N-7, M-138, T-209, V-84, K-172, V-19, D-56, P-73, G-33, T-106, N-170, L-28, T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85 and I-182. One or more of the primary and secondary substitutions may be selected from the group consisting of K29A, T67A, K78A, V84S, Y102A, K112S, K144A, K202M and N203G.

In a further embodiment, the recombinant allergen is derived from Der p 2. Amino acids potentially suitable for substitution comprise amino acids R-128, D-129, H-11, H-30, S-1, K-77, Y-75, R-31, K-82, K-6, K-96, K-48, K-55, K-89, Q-85, W-92, 1-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 and R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109, K-15. One or more of the primary and secondary substitutions may be selected from the group consisting of K6A, N10S, K15E, S24N, H30N, K48A, E62S, H74N, K77N, K82N, K100N and R128Q.

Examples of *Bet v* 1 mutants according to the present invention are as follows:
Mutant A:
   K6A, K15E, H30N, E62S.
Mutant B:
   K6A, K15E, H30N, E62S, H74N, K82N.
Mutant C:
   K6A, N10S, K15E, S24N, H30N, K48A, E62S, H74N, K77N, K82N, K100N and R128Q

### Vaccines

Preparation of vaccines is generally well known in the art. Vaccines are typically prepared as injectables either as liquid solutions or suspensions. Such vaccine may also be emulsified or formulated so as to enable nasal administration as well as oral, including buccal and sublingual, administration. The immunogenic component in question (the recombinant allergen as defined herein) may suitably be mixed with excipients which are pharmaceutically acceptable and further compatible with the active ingredient. Examples of suitable excipients are water, saline, dextrose, glycerol, ethanol and the like as well as combinations thereof. The vaccine may additionally contain other substances such as wetting agents, emulsifying agents, buffering agents or adjuvants enhancing the effectiveness of the vaccine.

Vaccines are most frequently administered parenterally by subcutaneous or intramuscular injection. Formulations which are suitable for administration by another route include oral formulations and suppositories. Vaccines for oral administration may suitably be formulated with excipients normally employed for such formulations, e.g. pharmaceutical grades of mannitol, lactose, starch, magnesium stearate, sodium saccharine, cellulose, magnesium carbonate and the like. The composition can be formulated as solutions, suspensions, emulsions, tablets, pills, capsules, sustained release formulations, aerosols, powders, or granulates.

The vaccines are administered in a way so as to be compatible with the dosage formulation and in such amount as will be therapeutically effective and immunogenic. The quantity of active component contained within the vaccine depends on the subject to be treated, i.a. the capability of the subject's immune system to respond to the treatment, the route of administration and the age and weight of the subject. Suitable dosage ranges can vary within the range from about 0.0001 µg to 1000 µg.

As mentioned above, an increased effect may be obtained by adding adjuvants to the formulation. Examples of such adjuvants are aluminum hydroxide and phosphate (alum) or calcium phosphate as a 0.05 to 0.1 percent solution in phosphate buffered saline, synthetic polymers of sugars or polylactid glycolid (PLG) used as 0.25 percent solution. Mixture with bacterial cells such as *C*. *parvum,* endotoxins or lipopolysaccharide components of gram-negative bacteria, emulsion in physiologically acceptable oil vehicles such as mannide monoaleate (Aracel A) or emulsion with 20 percent solution of a perfluorocarbon (e.g. Fluosol-DA) used as a block substitute may also be employed. Oil emulsions, such as MF-59 may also be used. Other adjuvants such as Freund's complete and incomplete adjuvants as well as saponins, such as QuilA, Qs-21 and ISCOM, and RIBI may also be used.

Most often, multiple administrations of the vaccine will be necessary to ensure an effect. Frequently, the vaccine is administered as an initial administration followed by subsequent inoculations or other administrations. The number of vaccinations will typically be in the range of from 1 to 50, usually not exceeding 35 vaccinations. Vaccination will normally be performed from biweekly to bimonthly for a period of 3 months to 5 years. This is contemplated to give desired level of prophylactic or therapeutic effect.

The recombinant allergen may be used as a pharmaceutical preparation, which is suitable for providing a certain protection against allergic responses during the period of the year where symptoms occur (prophylaxis). Usually, the treatment will have to be repeated every year to maintain the protective effect. Preparations formulated for nasal, oral and sublingual application are particular suited for this purpose.

### Method of preparing a recombinant allergen according to the invention

As mentioned above, the present invention also relates to a method of preparing the recombinant mutant allergen of the invention, cf. claim 50.

The surface-exposed amino acids suitable for substitution in accordance with the present invention may be identified on the basis of information of their solvent (water) accessibility, which expresses the extent of surface exposure. A preferred embodiment of the method of the invention is characterised in ranking the said identified amino acid residues with respect to solvent accessibility and substituting one or more amino acids among the more solvent accessible ones.

A second parameter, which may contribute to the identification of surface-exposed amino acids suitable for substitution in accordance with the present invention is the extent in which an amino acid is conserved in all known homologous proteins within the species from which said naturally occurring allergen originates. Alternatively, the extent in which in all known homologous proteins within the taxonomic genus, subfamily, family, superfamily, legion suborder or order from which said naturally occurring allergen originates is used as such a second parameter.

Accordingly, a preferred embodiment of the method of the invention is characterised in selecting identified amino acid residues, which are conserved with more than 70 %, preferably more than 80 % and most preferably more than 90 % identity in all known homologous proteins within the species from which said naturally occurring allergen originates.

Furthermore, a particularly preferred embodiment of the method of the invention is characterised in ranking the said identified amino acid residues with respect to degree of conversation in all known homologous proteins within the species from which said naturally occurring allergen originates and substituting one or more amino acids among the more conserved ones.

A further preferred embodiment of the method of the invention comprises selecting the identified amino acids so as to form a mutant allergen, which has essentially the same α-carbon backbone tertiary structure as said naturally occurring allergen.

Another preferred embodiment of the method of the invention is characterised in that the substitution of amino acid residues is carried out by site-directed mutagenesis.

An alternative preferred embodiment of the method of the invention is characterised in that the substitution of amino acid residues is carried out by DNA shuffling.

### Criteria for substitution

For molecules for which the tertiary structure has been determined (e.g. by x-ray crystallography, or NMR electron microscopy), the mutant carrying the substituted amino acid(s) should preferably fulfil the following criteria:
1. The overall α-carbon backbone tertiary structure of the molecule is preferably conserved. Conserved is defined as an average root mean square deviation of the atomic coordinates comparing the structures below 2Å. This is important for two reasons: a) It is anticipated that the entire surface of the natural allergen constitutes an overlapping continuum of potential antibody-binding epitopes. The majority of the surface of the molecule is not affected by the substitution (s), and thus retain its antibody-binding inducing properties, which is important for the generation of new protective antibody specificities being directed at epitopes present also on the natural allergen. b) Stability, both concerning shelf-life and upon injection into body fluids.
2. The amino acids to be substituted are preferably located at the surface, and thus accessible for antibody-binding. Amino acids located on the surface in the three-dimensional structure usually have a solvent (water) accessibility of at least 20%, suitably 20-80%, more suitably 30-80%. Solvent accessibility is defined as the area of the molecule accessible to a sphere with a radius comparable to a solvent (water, r = 1.4 A) molecule.
3. Each of the substituted amino acids is preferably located in conserved patches having an area larger than 400 Å². Conserved patches are defined as coherently connected areas of surface-exposed conserved amino acid residues and backbone. Conserved amino acid residues are defined by sequence alignment of all known (deduced) amino acid sequences of homologues proteins within the same taxonomic species, genus, subfamily, family, superfamily, legion, suborder or order. Amino acid positions having identical amino acid residues in more than 70% of the sequences are considered conserved. Conserved patches are expected to contain epitopes to which the IgE of the majority of patients is directed.
   Conservation of α-carbon backbone tertiary structure is best determined by obtaining identical structures by x-ray crystallography or NMR before and after mutagenesis. In absence of structural data describing the mutant indistinguishable CD-spectra or immunochemical data, e.g. antibody reactivity, may render conservation of α-carbon backbone tertiary structure probable, if compared to the data obtained by analysis of a structurally determined molecule.
4. Within the conserved patches amino acids for mutagenesis should.preferentially be selected among the most solvent (water) accessible ones located preferably near the centre of the conserved patch.
5. Preferentially, a polar amino acid residue is substituted by another polar residue, and a non-polar amino acid residue is substituted by another non-polar residue.

With an object of essentially retaining the three-dimensional structure of the allergen, the amino acid to be incorporated may be selected on the basis of a comparison with a protein, which is a structural homologue to the allergen, e.g. a protein, which belongs to the same taxonomic order as the allergen, and which does not have any cross-reactivity with the allergen.

### DNA according to the invention

In a preferred embodiment, the DNA sequence of the invention is a derivative of the DNA sequence encoding the naturally occurring allergen.

Preferably, the DNA derivative is obtained by site-directed or random mutagenesis of the DNA encoding the naturally occurring allergen.

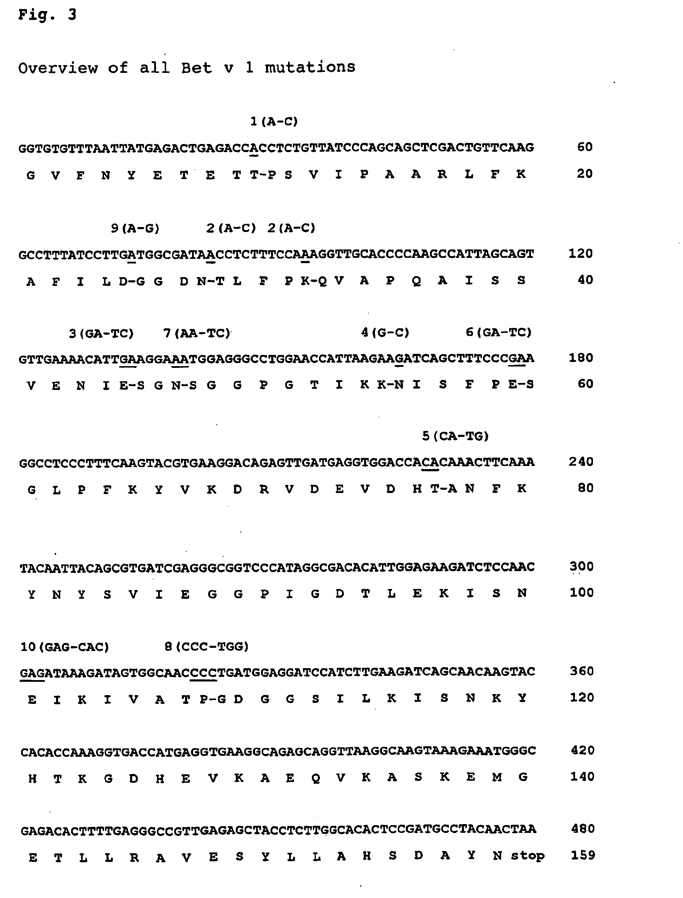

In a first particularly preferred embodiment, the DNA sequence is a derivative of the sequence shown in Fig. 3, wherein the DNA sequence is mutated so as to encode an allergen having at least four mutations selected from the group consisting of K-129, E-60, N-47, K-65, P-108, N-159, D-93, K-123, K-32, D-125, R-145, D-109, E-127, Q-36, E-131, L-152, E-6, E-96, D-156, P-63, H-76, E-8, K-134, E-45, T-10, V-12, K-20, S-155, H-126, P-50, N-78, K-119, V-2, L-24, E-42, N-4, A-153, I-44, E-138, G-61, A-130, R-70, N-28, P-35, S-149, K-103, Y-150, H-154, N-43, A-106, K-115, P-14, Y-5, K-137, E-141, E-87, E-73.

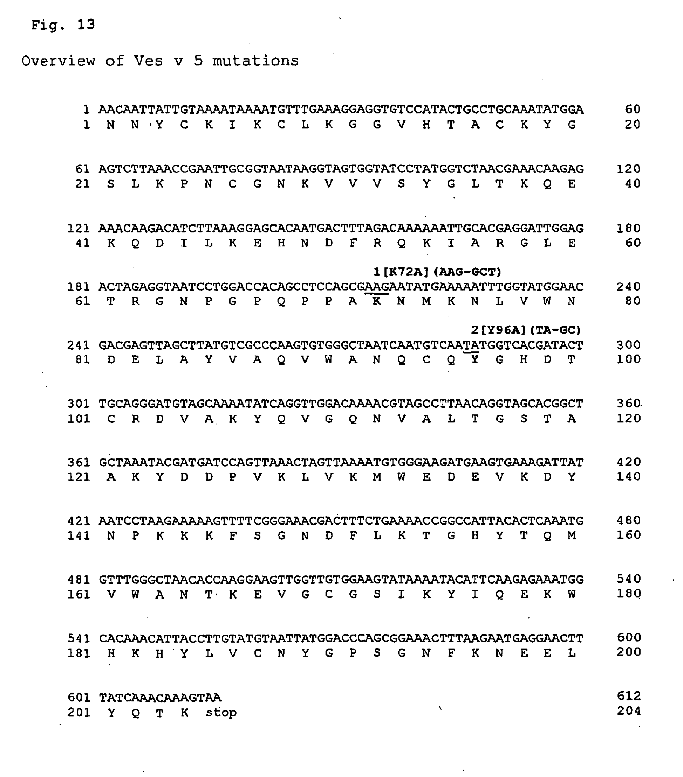

In a second particularly preferred embodiment, the DNA sequence is a derivative of the sequence shown in Fig. 13, wherein the DNA sequence is mutated so as to encode an allergen having at least four mutations selected from the group consisting of K-16, K-185, K-11, K-44, K-210, R-63, K-13, F-6, K-149, K-128, E-184, K-112, F-157, E-3, K-29, N-203, N-34, K-78, K-151, L-15, L-158, Y-102, W-186, K-134, D-87, K-52, T-67, T-125, K-150, Y-40, Q-48, L-65, K-81, Q-101, Q-208, K-144, N-8, N-70, H-104, Q-45, K-137, K-159, E-205, N-82, A-111, D-131, K-24, V-36, N-7, M-138, T-209, V-84, K-172, V-19, D-56, P-73, G-33, T-106, N-170, L-28, T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85 and I-182.

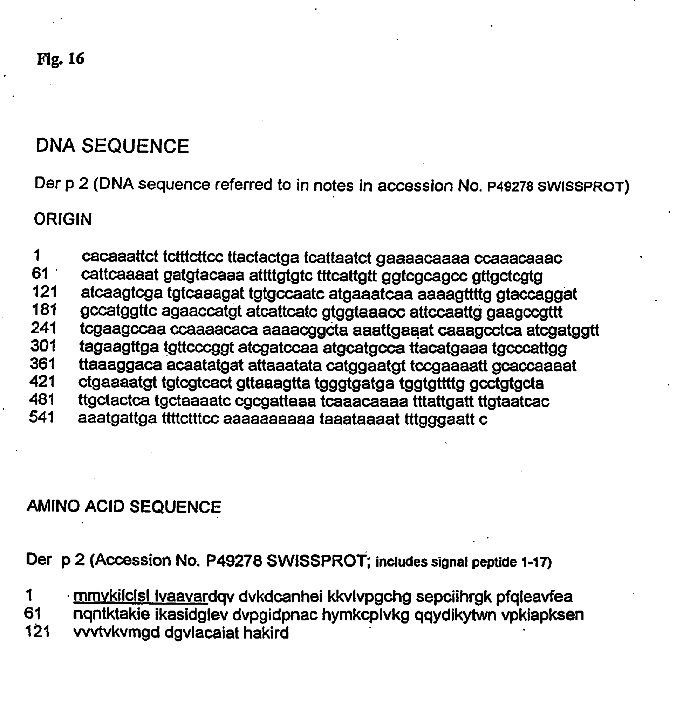

In a third particularly preferred embodiment, the DNA sequence is a derivative of the sequence shown in Fig. 16, wherein the DNA sequence is mutated so as to encode an allergen having at least four mutations selected from the group consisting of R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 and R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109, K-15.

### DNA shuffling

The recombinant mutant allergen according to the present invention may be produced using a DNA sequence obtained by DNA shuffling (molecular breeding) of the corresponding naturally DNA. DNA shuffling may be carried out according to the procedures disclosed in the article by Punnonen et al. (ref. 25) as well as the procedures disclosed in the articles mentioned therein, which are all included herein by this reference.

### Diagnostic assay

Furthermore, the recombinant mutant allergens according to the invention have diagnostic possibilities and advantages. Prior art allergy vaccines are based on extracts of the naturally occurring allergen source, and thus represent a wide variety of isoforms. The allergic individual has initially been sensitised and has IgE to one or some of the isoforms present. Some of the isoforms may be relevant with respect to the allergic reactions of the allergic individual due to homology and subsequent cross-reactivity with the isoform to which the individual is allergic, whereas other isoforms may be irrelevant as they do not harbour any of the IgE binding epitopes to which the allergic individual has specific IgE. Due to this heterogeneity of the specificities of the IgE population, some isoforms may therefore be safe to administer, i.e. they do not result in an allergic response via IgE, whereas other isoforms may be harmful causing undesirable side-effects.

Thus, the mutants of the invention and the compositions of the invention intended to be administered therapeutically may also be used for an in vivo or in vitro diagnostic assay to monitor the relevance, safety or outcome of a treatment with such mutants or compositions. Diagnostic samples to be applied include body samples, such as sera. Thus, the invention also relates to a diagnostic assay for assessing relevance, safety or outcome of therapy of a subject using a recombinant mutant allergen according to the invention or a composition according to the invention, wherein an IgE containing sample of the subject is mixed with said mutant or said composition and assessed for the level of reactivity between the IgE in said sample and said mutant. The assessing of the level of reactivity between the IgE in the sample and the mutant may be carried out using any known immunoassay.

### Definitions

In connection with the present invention the expression "reduce the specific IgE binding capability as compared to the IgE binding capability of the said natural-occurring allergen" means that the reduction is measurable in a statistically significant manner (p <0.05) in at least one immunoassay using serum from a subject allergic to the natural-occurring allergen. Preferably, the IgE binding capability is reduced by at least 5 %, more preferably at least 10 %.

The expression "surface-exposed amino acid" means that the amino acid residue is located at the surface of the three-dimensional structure in such a manner that when the allergen is in solution at least a part of at least one atom of the amino acid residue is accessible for contact with the surrounding solvent. Preferably, the amino acid residue in the three-dimensional structure has a solvent (water) accessibility of at least 20%, suitably at least 30 %, more suitably at least 40 % and most preferably at least 50 %.

The expression "solvent accessibility" is defined as the area of the molecule accessible to a sphere with a radius comparable to a solvent (water, r = 1.4 Å) molecule.

The expressions "surface-exposed" and "solvent-exposed" are used interchangeably.

The expression "the taxonomic species from which said naturally occurring allergen originates" means species in the taxonomic system.

Furthermore, the expression "said mutant allergen having essentially the same α-carbon backbone tertiary structure as said naturally occurring allergen" means that when comparing the structures, the average root mean square deviation of the atomic coordinates is below 2 Å.

In connection with the present invention the expression "substitution" means the deletion, substitution or addition of an amino acid in comparison to the amino acid sequence of the naturally occurring allergen.

The present invention is further illustrated by the following non-limiting examples.

### EXAMPLES

### EXAMPLE 1

Example 1 describes the preparation of recombinant mutant allergens with one and three primary mutations. Recombinant mutant allergens according to the invention, i.e. allergens comprising at least four primary mutations, may be prepared using the same procedures.

### Identification of common epitopes within Fagales pollen allergens

The major birch pollen allergen *Bet v* 1 shows about 90% amino acid sequence identity with major allergens from pollens of taxonomically related trees, i.e *Fagales* (for instance hazel and hornbeam) and birch pollen allergic patients often show clinical symptoms of allergic cross-reactivity towards these *Bet v* 1 homologous proteins.

*Bet v* 1 also shows about 50-60% sequence identity with allergic proteins present in certain fruits (for instance apple and cherry) and vegetables (for instance celery and carrot) and there are clinical evidence for allergic cross-reactivity between Bet *v* 1 and these food related proteins.

In addition, *Bet v* 1 shares significant sequence identity (20-40%) with a group of plant proteins called pathogenesis-related proteins (PR-10), however there are no reports of allergic cross-reactivity towards these PR-10 proteins.

Molecular modelling suggests that the structures of *Fagales* and food allergens and PR-10 proteins are close to being identical with the *Bet v* 1 structure.

The structural basis for allergic *Bet v* 1 cross-reactivity was reported in (Gajhede et al 1996, ref. 17) where three patches on the molecular surface of *Bet v* 1 could be identified to be common for the known major tree pollen allergens. Thus, any IgE recognising these patches on *Bet v* 1 would be able to cross-react and bind to other *Fagales* major pollen allergens and give rise to allergic symptoms. The identification of these common patches was performed after alignment of all known amino acid sequences of the major tree pollen allergens in combination with an analysis of the molecular surface of *Bet v* 1 revealed by the α-carbon backbone tertiary structure reported in ref. 17. In addition, the patches were defined to have a certain minimum size (>400 Å²) based on the area covered by an antibody upon binding.

### Selection of amino acid residues for site-directed mutagenesis

Amino acid residues for site-directed mutagenesis were selected among residues present in *Bet v* 1 specific areas and the common patches since modifications of these is expected to affect the binding of serum IgE from the majority of patients showing clinical tree pollen allergic cross-reactivity.

The relative orientation and percentage of solvent-exposure of each amino acid residue within respective patch was calculated based on their atomic coordinates. Residues having a low degree of solvent exposure (<20%) were not regarded relevant for mutagenesis due to the possible disruption of the structure or lack of antibody interaction. The remaining residues were ranked according to their degree of solvent-exposure.

### Sequence alignment

Sequences homologous to the query sequence (*Bet v* 1 No. 2801, WHO IUIS Nomenclature Subcommittee on Allergens) were derived from GenBank and EMBL sequence databases by a BLAST search (Altschul et al., ref. 18). All sequences with BLAST reported probabilities less than 0.1 were taken into consideration and one list were constructed containing a non-redundant list of homologous sequences. These were aligned by CLUSTAL W (Higgins et al., ref. 19) and the percentage identity were calculated for each position in the sequence considering the complete list or taxonomically related species only. A total of 122 sequences were homologous to *Bet v 1 No. 2801* of which 57 sequences originates from taxonomically related species.

### Cloning of the gene encoding Bet v 1

RNA was prepared from *Betula verrucosa* pollen (Allergon, Sweden) by phenol extraction and LiCl precipitation. Oligo(dT)-cellulose affinity chromatography was performed batch-wise in Eppendorph tubes, and double-stranded cDNA was synthesised using a commercially available kit (Amersham). DNA encoding *Bet v* 1 was amplified by PCR and cloned. In brief, PCR was performed using cDNA as template, and primers designed to match the sequence of the cDNA in positions corresponding to the amino terminus of *Bet v* 1 and the 3'-untranslated region, respectively. The primers were extended in the 5'-ends to accommodate restriction sites (*Nco*I and *Hin*dIII) for directional cloning into pKK233-2.

### Subcloning into pMAL-c

The gene encoding *Bet v* 1 was subsequently subcloned into the maltose binding protein fusion vector pMAL-c (New England Biolabs). The gene was amplified by PCR and subcloned in frame with *malE* to generate maltose binding protein (MBP)-*Bet v* 1 protein fusion operons in which MBP and *Bet v* 1 were separated by a factor Xₐ protease clevage site positioned to restore the authentic aminoterminal sequence of *Bet v* 1 upon cleavage, as described in ref. 15. In brief, PCR was performed using pKK233-3 with *Bet v* 1 inserted as template and primers corresponding to the amino- and carboxyterminus of the protein, respectively. The promoter proximal primer was extended in the 5'-end to accommodate 4 codons encoding an in frame factor Xₐ protease cleavage site. Both primers were furthermore extended in the 5'-ends to accommodate restriction sites (*Kpn*I) for cloning. The *Bet v* 1 encoding genes were subcloned using 20 cycles of PCR to reduce the frequency of PCR artefacts.

### In vitro mutagenesis

*In vitro* mutagenesis was performed by PCR using recombinant pMAL-c with *Bet v* 1 inserted as template. Each mutant *Bet v* 1 gene was generated by 3 PCR reactions using 4 primers.

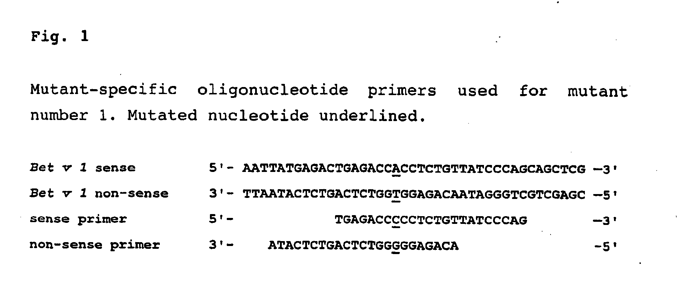

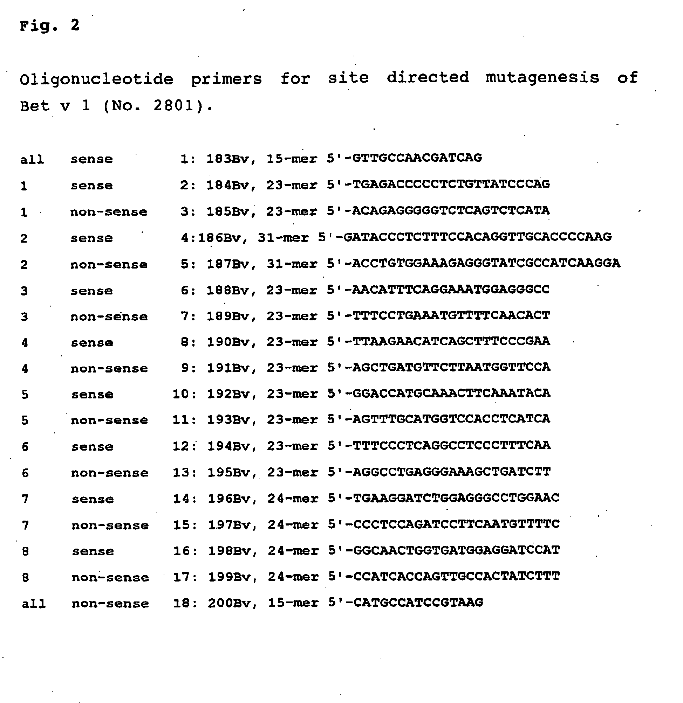

Two mutation-specific oligonucleotide primers were synthesised accommodating each mutation, one for each DNA strand, see Figs. 1 and 2. Using the mutated nucleotide(s) as starting point both primers were extended 7 nucleotides in the 5'-end and 15 nucleotides in the 3'-end. The extending nucleotides were identical in sequence to the *Bet v* 1 gene in the actual region.

Two generally applicable primers (denoted "all-sense" and "all non-sense" in Figure 2) were furthermore synthesised and used for all mutants. These primers were 15 nucleotides in length and correspond in sequence to regions of the pMAL-c vector approximately 1 kilobase upstream and downstream from the *Bet v 1*. The sequence of the upstream primer is derived from the sense strand and the sequence of the downstream primer is derived from the non-sense strand, see Fig. 2.

Two independent PCR reactions were performed essentially according to standard procedures (Saiki *et al* 1988, ref. 20) with the exception that only 20 temperature cycles were performed in order to reduce the frequency of PCR artefacts. Each PCR reaction used pMAL-c with *Bet v 1* inserted as template and one mutation-specific and one generally applicable primer in meaningful combinations.

Introduction of the four amino acid substitutions (Asn28Thr, Lys32Gln, Glu45Ser, Pro108Gly) in the Triple-patch mutant were performed like described above in a step by step process. First the Glu45Ser mutation then the Pro108Gly mutation and last the Asn28Thr, Lys32Gln mutations were introduced using pMAL-c with inserted Bet *v 1 No. 2801, Bet v 1 (Glu45Ser), Bet v 1 (Glu45Ser, Pro108Gly)* as templates, respectively.

The PCR products were purified by agarose gel electrophoresis and electro-elution followed by ethanol precipitation. A third PCR reaction was performed using the combined PCR products from the first two PCR reactions as template and both generally applicable primers. Again, 20 cycles of standard PCR were used. The PCR product was purified by agarose gel electrophoresis and electro-elution followed by ethanol precipitation, cut with restriction enzymes (*Bsi*WI/*Eco*RI), and ligated directionally into pMAL-c with *Bet v 1* inserted restricted with the same enzymes.

Figure 3 shows an overview of all 9 *Bet v* 1 mutations, which are as follows

Thr10Pro, Asp25Gly, Asn28Thr + Lys32Gln, Glu45Ser, Asn47Ser, Lys55Asn, Glu60Ser (non-patch), Thr77Ala and Pro108Gly. An additional mutant with four mutations was also prepared (Asn28Thr, Lys32Gln, Glu45Ser, Pro108Gly). Of these, five mutants were selected for further testing: Asn28Thr + Lys32Gln, Glu45Ser, Glu60Ser, Pro108Gly and the Triple-patch mutant Asn28Thr, Lys32Gln, Glu45Ser, Pro108Gly.

### Nucleotide sequencing

Determination of the nucleotide sequence of the *Bet v* 1 encoding gene was performed before and after subcloning, and following *in vitro* mutagenesis, respectively.

Plasmid DNA's from 10 ml of bacterial culture grown to saturation overnight in LB medium supplemented with 0.1 g/l ampicillin were purified on Qiagen-tip 20 columns and sequenced using the Sequenase version 2.0 DNA sequencing kit (USB) following the recommendations of the suppliers.

### Expression and purification of recombinant Bet v 1 and mutants

Recombinant *Bet v* 1 (*Bet v* 1 No. 2801 and mutants) were over-expressed in *Escherichia coli* DH 5a fused to maltose-binding protein and purified as described in ref. 15. Briefly, recombinant *E.coli* cells were grown at 37°C to an optical density of 1.0 at 436 nm, whereupon expression of the *Bet v* 1 fusion protein was induced by addition of IPTG. Cells were harvested by centrifugation 3 hours post-induction, re-suspended in lysis buffer and broken by sonication. After sonication and additional centrifugation, recombinant fusion protein was isolated by amylose affinity chromatography and subsequently cleaved by incubation with Factor Xa (ref. 15). After F Xa cleavage, recombinant *Bet v* 1 was isolated by gelfiltration and if found necessary, subjected to another round of amylose affinity chromatography in order to remove trace amounts of maltose-binding protein.

Purified recombinant *Bet v* 1 was concentrated by ultrafiltration to about 5 mg/ml and stored at 4 °C. The final yields of the purified recombinant *Bet v* 1 preparations were between 2-5 mg per litre *E*. *coli* cell culture.

The purified recombinant *Bet v* 1 preparations appeared as single bands after silver-stained SDS-polyacrylamide electrophoresis with an apparent molecular weight of 17.5 kDa. N-terminal sequencing showed the expected sequences as derived from the cDNA nucleotide sequences and quantitative amino acid analysis showed the expected amino acid compositions.

We have previously shown (ref. 15) that recombinant Bet *v* 1 No. 2801 is immunochemically indistinguishable from naturally occurring *Bet v* 1.

### Immunoelectrophoresis using rabbit polyclonal antibodies

The seven mutant *Bet v 1* were produced as recombinant *Bet v* 1 proteins and purified as described above and tested for their reactivity towards polyclonal rabbit antibodies raised against *Bet v* 1 isolated from birch pollen. When analysed by immunoelectrophoresis (rocket-line immunoelectrophoresis) under native conditions, the rabbit antibodies were able to precipitate all mutants, indicating that the mutants had conserved α-carbon backbone tertiary structure.

In order to analyse the effect on human polyclonal IgE-response, the mutants Glu45Ser, Pro108Gly Asn28Thr+Lys32Gln and Glu60Ser were selected for further analysis.

### Bet v 1 Glu45Ser mutant

Glutamic acid in position 45 show a high degree of solvent-exposure (40%) and is located in a molecular surface patch common for *Fagales* allergens (patch I). A serine residue was found to occupy position 45 in some of the *Bet v* 1 homologous PR-10 proteins arguing for that glutamic acid can be replaced by serine without distortion of the α-carbon backbone tertiary structure. In addition, as none of the known *Fagales* allergen sequences have serine in position 45, the substitution of glutamic acid with serine gives rise to a non-naturally occurring *Bet v* 1 molecule.

### T cell proliferation assay using recombinant Glu45Ser Bet v 1 mutant

The analysis was carried out as described in Spangfort *et al* 1996a. It was found that recombinant *Bet v* 1 Glu45Ser mutant was able to induce proliferation in T cell lines from three different birch pollen allergic patients with stimulation indices similar to recombinant and naturally occurring.

### Crystallisation and structural determination of recombinant Glu45Ser Bet v 1

Crystals of recombinant Glu45Ser *Bet v* 1 were grown by vapour diffusion at 25°C, essentially as described in (Spangfort *et al* 1996b, ref. 21). Glu45Ser *Bet v* 1, at a concentration of 5 mg/ml, was mixed with an equal volume of 2.0 M ammonium sulphate, 0.1 M sodium citrate, 1% (v/v) dioxane, pH 6.0 and equilibrated against 100x volume of 2.0 M ammonium sulfate, 0.1 M sodium citrate, 1% (v/v) dioxane, pH 6.0. After 24 hours of equilibration, crystal growth was induced by applying the seeding technique described in ref. 21, using crystals of recombinant wild-type *Bet v* 1 as a source of seeds.

After about 2 months, crystals were harvested and analysed using X-rays generated from a Rigaku rotating anode as described in ref. 21 and the structure was solved using molecular replacement.

### Structure of Bet v 1 Glu45Ser mutant

The structural effect of the mutation was addressed by growing three-dimensional *Bet v* 1 Glu45Ser protein crystals diffracting to 3.0 Å resolution when analysed by X-rays generated from a rotating anode. The substitution of glutamic acid to serine in position 45 was verified by the *Bet v* 1 Glu45Ser structure electron density map which also showed that the overall α-carbon backbone tertiary structure is preserved.

### IgE-binding properties of Bet v 1 Glu45Ser mutant

The IgE-binding properties of *Bet v* 1 Glu45Ser mutant was compared with recombinant *Bet v* 1 in a fluid-phase IgE-inhibition assay using a pool of serum IgE derived from birch allergic patients.

Recombinant *Bet v* 1 no. 2801 was biotinylated at a molar ratio of 1:5 (Bet *v* 1 no. 2801:biotin). The inhibition assay was performed as follows: a serum sample (25 µl) was incubated with solid phase anti IgE, washed, resuspended and further incubated with a mixture of biotinylated *Bet v* 1 no. 2801 (3.4 nM) and a given mutant (0-28.6 nM). The amount of biotinylated *Bet v* 1 no. 2801 bound to the solid phase was estimated from the measured RLU after incubation with acridinium ester labelled streptavidin. The degree of inhibition was calculated as the ratio between the RLU's obtained using buffer and mutant as inhibitor.

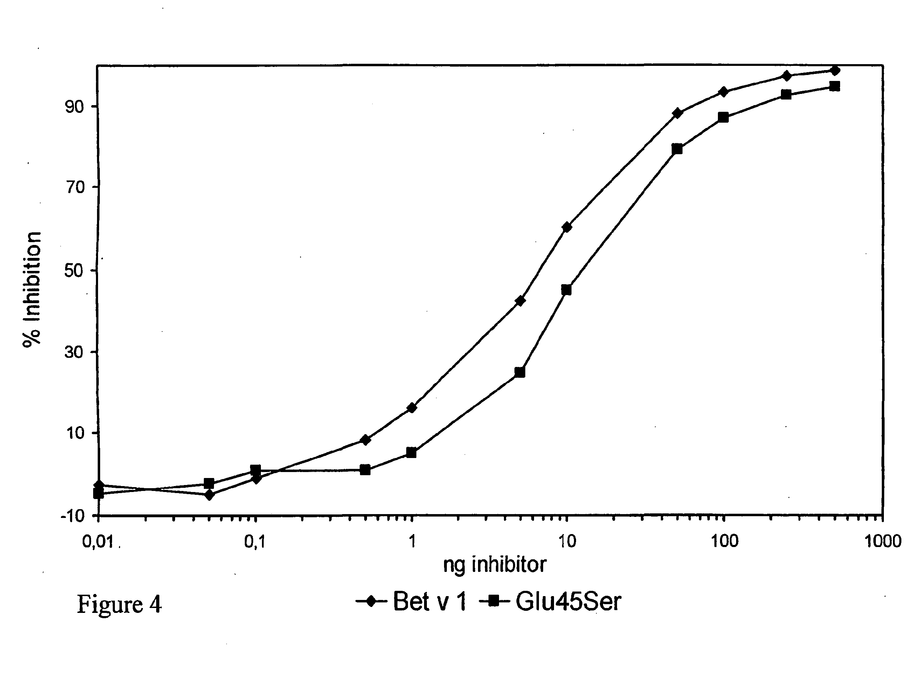

Figure 4 shows the inhibition of the binding of biotinylated recombinant *Bet v* 1 to serum IgE from a pool of allergic patients by non-biotinylated *Bet v* 1 and by *Bet v* 1 Glu45Ser mutant.

There is a clear difference in the amount of respective recombinant proteins necessary to reach 50% inhibition of the binding to serum IgE present in the serum pool. Recombinant *Bet v* 1 reaches 50% inhibition at about 6.5 ng whereas the corresponding concentration for *Bet v 1* Glu45Ser mutant is about 12 ng. This show that the point mutation introduced in *Bet v* 1 Glu45Ser mutant lowers the affinity for specific serum IgE by a factor of about 2. The maximum level of inhibition reached by the *Bet v 1* Glu45Ser mutant is clearly lower compared to recombinant *Bet v* 1. This may indicate that after the Glu45Ser substitution, some of the specific IgE present in the serum pool are unable to recognise the *Bet v* 1 Glu45Ser mutant.

### Bet v 1 mutant Asn28Thr+Lys32Gln

Aspartate and lysine in positions 28 and 32, respectively show a high degree of solvent-exposure (35% and 50%, respectively) and are located in a molecular surface patch common for *Fagales* allergens (patch II). In the structure, aspartate 28 and lysine 32 are located close to each other on the molecular surface and most likely interact via hydrogen bonds. A threonine and a gluatamate residue were found to occupy positions 28 and 32, respectively in some of the Bet *v* 1 homologous PR-10 proteins arguing for that aspartate and lysine can be replaced with threonine and glutamate, respectively without distortion of the α-carbon backbone tertiary structure. In addition, as none of the naturally occurring isoallergen sequences have threonine and glutamate in positions 28 and 32, respectively, the substitutions gives rise to a non-naturally occurring *Bet v* 1 molecule.

### IgE-binding properties of Bet v 1 mutant Asn28Thr+Lys32Gln

The IgE-binding properties of mutant Asn28Thr+Lys32Gln was compared with recombinant *Bet v* 1 in a fluid-phase IgE-inhibition assay using the pool of serum IgE derived from birch allergic patients described above.

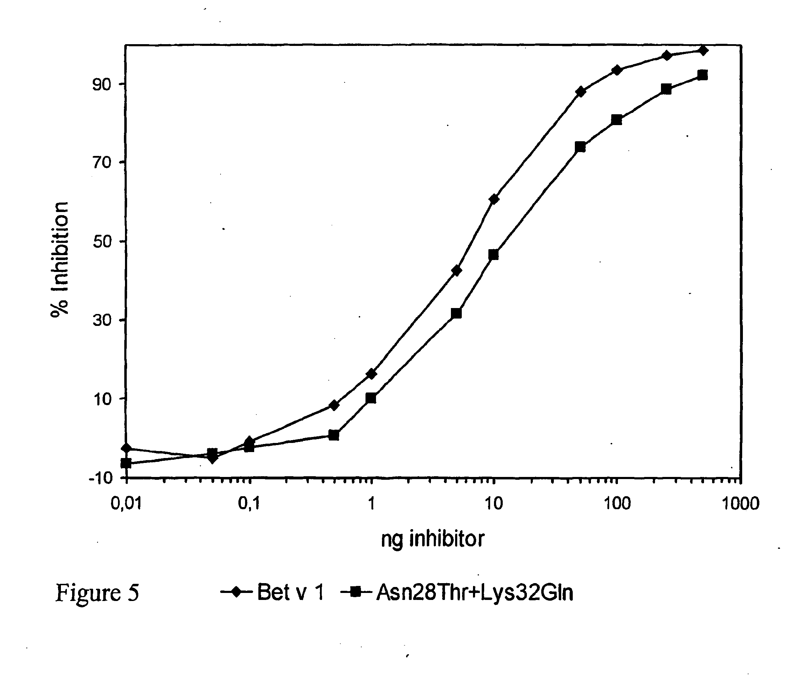

Figure 5 shows the inhibition of the binding of biotinylated recombinant *Bet v* 1 to serum IgE from a pool of allergic patients by non-biotinylated *Bet v* 1 and by *Bet v* 1 mutant Asn28Thr+Lys32Gln.

There is a clear difference in the amount of respective recombinant proteins necessary to reach 50% inhibition of the binding to serum IgE present in the serum pool. Recombinant *Bet v* 1 reaches 50% inhibition at about 6.5 ng whereas the corresponding concentration for *Bet v 1* mutant Asn28Thr+Lys32Gln is about 12 ng. This show that the point mutations introduced in *Bet v* 1 mutant Asn28Thr+Lys32Gln lowers the affinity for specific serum IgE by a factor of about 2.

The maximum level of inhibition reached by the *Bet v 1* mutant Asn28Thr+Lys32Gln mutant is clearly lower compared to recombinant *Bet v* 1. This may indicate that after the Asn28Thr+Lys32Gln substitutions, some of the specific IgE present in the serum pool are unable to recognise the *Bet v 1* mutant Asn28Thr+Lys32Gln.

### Bet v 1 mutant Pro108Gly

Proline in position 108 show a high degree of solvent-exposure (60%) and is located in a molecular surface patch common for *Fagales* allergens (patch III). A glycine residue was found to occupy position 108 in some of the *Bet v* 1 homologous PR-10 proteins arguing for that proline can be replaced with glycine without distortion of the α-carbon backbone tertiary structure. In addition, as none of the naturally occurring isoallergen sequences have glycine in position 108, the substitution of proline with glycine gives rise to a non-naturally occurring *Bet v* 1 molecule.

### IgE-binding properties of Bet v 1 Pro108Gly mutant

The IgE-binding properties of *Bet v* 1 Pro108Gly mutant was compared with recombinant *Bet v* 1 in a fluid-phase IgE-inhibition assay using the pool of serum IgE derived from birch allergic patients described above.

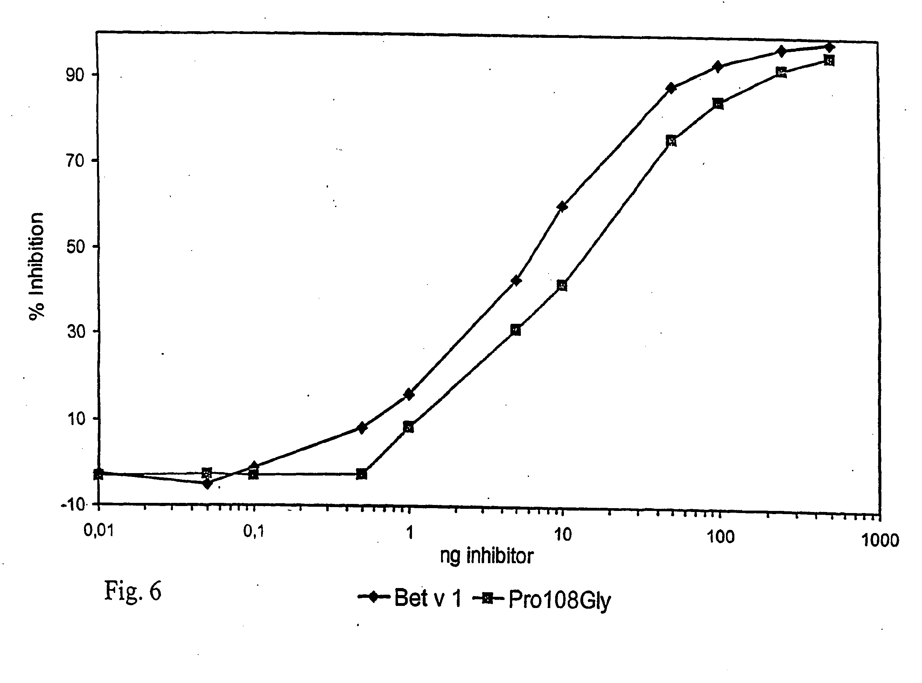

Figure 6 shows the inhibition of the binding of biotinylated recombinant *Bet v* 1 to serum IgE from a pool of allergic patients by non-biotinylated *Bet v* 1 and by *Bet v* 1 Pro108Gly mutant.

There is a clear difference in the amount of respective recombinant proteins necessary to reach 50% inhibition of the binding to serum IgE present in the serum pool. Recombinant *Bet v* 1 reaches 50% inhibition at about 6.5 ng whereas the corresponding concentration for *Bet v* 1 Pro108Gly is 15 ng. This show that the single point mutation introduced in *Bet v* 1 Pro108Gly lowers the affinity for specific serum IgE by a factor of about 2.

The maximum level of inhibition reached by the *Bet v* 1 Pro108Gly mutant is somewhat lower compared to recombinant *Bet v* 1. This may indicate that after the Pro108Gly substitution, some of the specific IgE present in the serum pool are unable to recognise the *Bet v* 1 Pro108Gly mutant.

### Bet v 1 mutant Glu60Ser (non-patch mutant)

Glutamic acid in position 60 show a high degree of solvent-exposure (60%) however, it is not located in a molecular surface patch common for *Fagales* allergens. A serine residue was found to occupy position 60 in some of the *Bet v* 1 homologous PR-10 proteins arguing for that glutamic acid can be replaced with serine without distortion of the α-carbon backbone tertiary structure. In addition, as none of the naturally occurring isoallergen sequences have serine in position 60, the substitution of glutamic acid with serine gives rise to a non-naturally occurring *Bet v* 1 molecule.

### IgE-binding properties of Bet v 1 Glu60Ser mutant

The IgE-binding properties of *Bet v* 1 Glu60Ser mutant was compared with recombinant *Bet v* 1 in a fluid-phase IgE-inhibition assay using the pool of serum IgE derived from birch allergic patients described above.

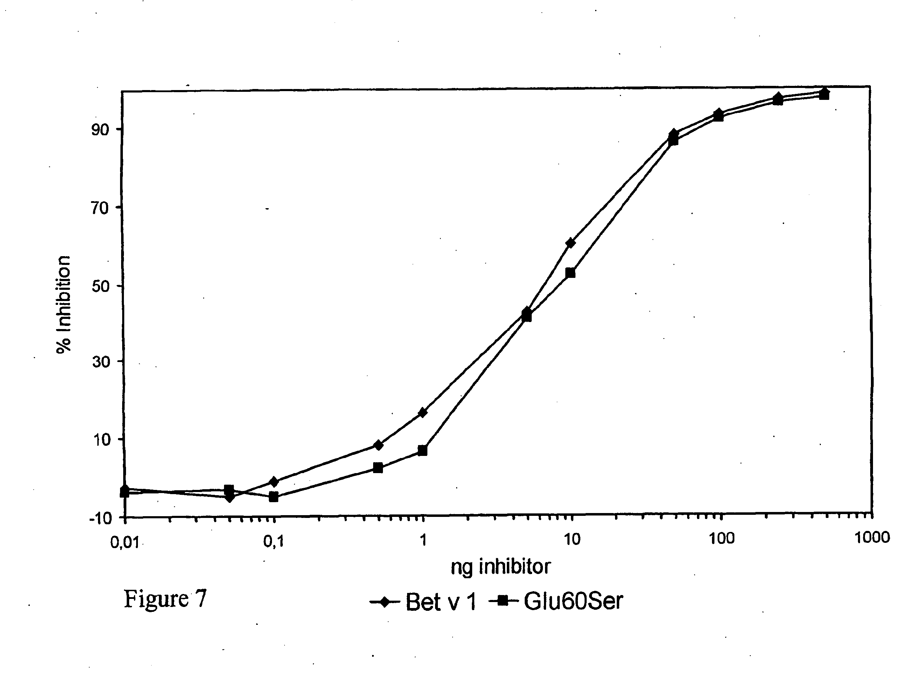

Figure 7 shows the inhibition of the binding of biotinylated recombinant *Bet v* 1 to serum IgE from a pool of allergic patients by non-biotinylated *Bet v* 1 and by *Bet v* 1 Glu60Ser mutant. In contrast to the Glu45Ser, Pro108Gly and Asn28Thr+Lys32Gln mutants, the substitution glutamic acid 60 to serine, does not shown any significant effect on the IgE-binding properties of. This indicates that substitutions outside the defined *Fagales* common patches only have a marginal effect on the binding of specific serum IgE supporting the concept that conserved allergen molecular surface areas harbours dominant IgE-binding epitopes.

### Bet v 1 Triple-patch mutant

In the Triple-patch mutant, the point mutations (Glu45Ser, Asn28Thr+Lys32Gln and Pro108Gly) introduced in the three different common *Fagales* patches, described above, were simultaneously introduced in creating an artificial mutant carrying four amino acid substitutions.

### Structural analysis of Bet v 1 Triple-patch mutant

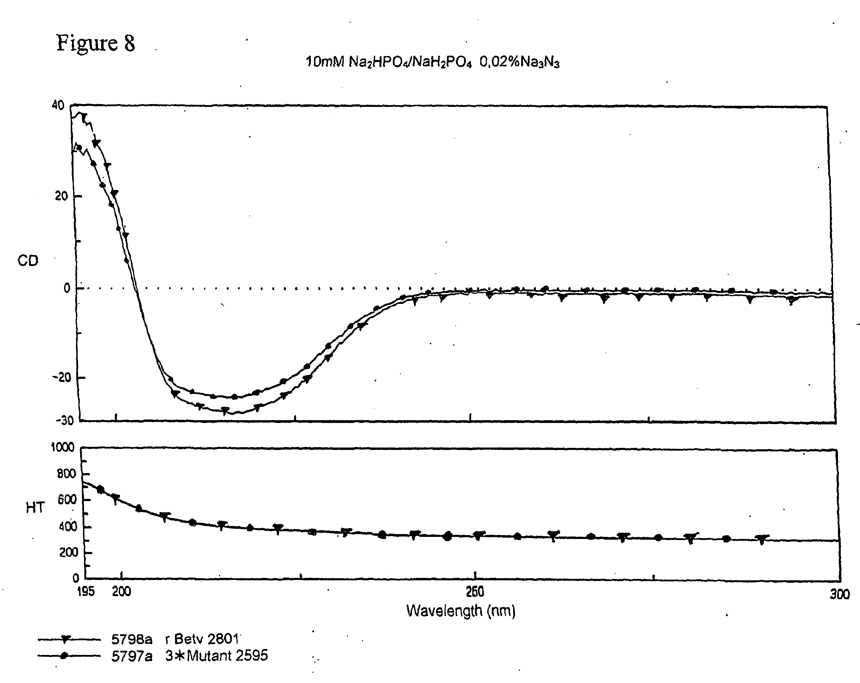

The structural integrity of the purified Triple-patch mutant was analysed by circular dichroism (CD) spectroscopy. Figure 8 shows the CD spectra of recombinant and Triple-patch mutant, recorded at close to equal concentrations. The overlap in peak amplitudes and positions in the CD spectra from the two recombinant proteins shows that the two preparations contain equal amounts of secondary structures strongly suggesting that the α-carbon backbone tertiary structure is not affected by the introduced amino acid substitutions.

### IgE-binding properties of Bet v 1 Triple-patch mutant

The IgE-binding properties of *Bet v* 1 Triple-patch mutant was compared with recombinant *Bet v* 1 in a fluid-phase IgE-inhibition assay using the pool of serum IgE derived from birch allergic patients described above.

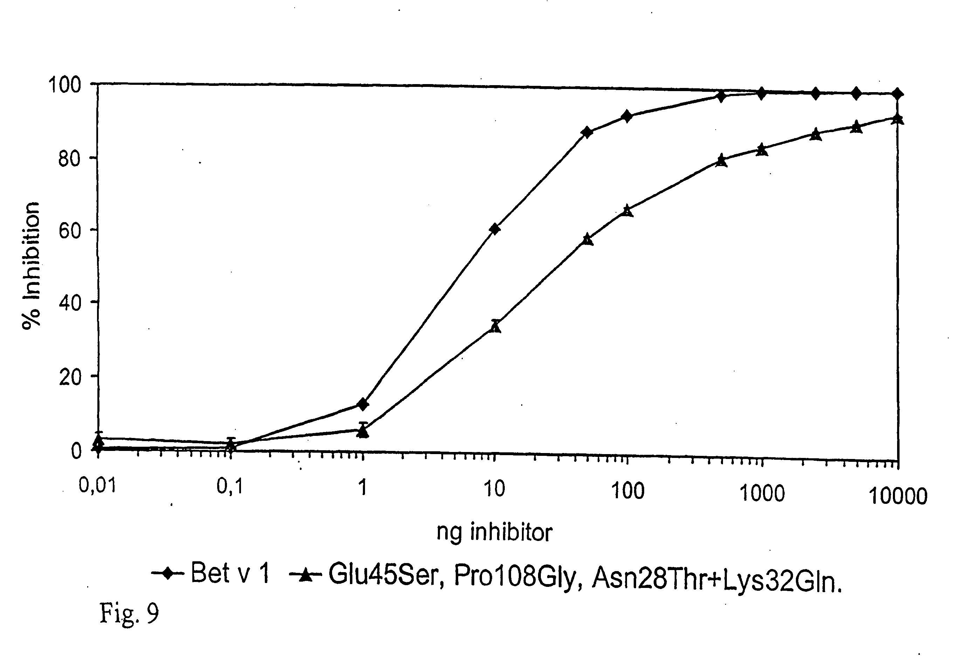

Figure 9 shows the inhibition of the binding of biotinylated recombinant *Bet v* 1 to serum IgE from a pool of allergic patients by non-biotinylated *Bet v* 1 and by *Bet v* 1 Triple-patch mutant. In contrast to the single mutants described above, the inhibition curve of the Triple-patch mutant is no longer parallel relative to recombinant. This shows that the substitutions introduced in the Triple-patch mutant has changed the IgE-binding properties and epitope profile compared to recombinant. The lack of parallellity makes it difficult to quantify the decrease of the Triple-patch mutant affinity for specific serum IgE.

Recombinant *Bet v* 1 reaches 50% inhibition at about 6 ng whereas the corresponding concentration for *Bet v* 1 Triple-patch mutant is 30 ng, i.e a decrease in affinity by a factor 5. However, in order to reach 80% inhibition the corresponding values are 20 ng and 400 ng, respectively, i.e a decrease by a factor 20.

### T cell proliferation assay using recombinant Bet v 1 Triple-patch mutant

The analysis was carried out as described in ref. 15. It was found that recombinant *Bet v* 1 Triple-patch mutant was able to induce proliferation in T cell lines from three different birch pollen allergic patients with stimulation indices similar to recombinant and naturally occurring. This suggests that the Triple-patch mutant can initiate the cellular immune response necessary for antibody production.

### EXAMPLE 2

Example 2 describes the preparation of recombinant mutant allergens with one primary mutation. Recombinant mutant allergens according to the invention, i.e. allergens comprising at least four primary mutations, may be prepared using the same procedures.

### Identification of common epitopes within Vespula vulgaris venom major allergen antigen 5

Antigen 5 is one of the three vespid venom proteins, which are known allergens in man. The vespids include hornets, yellow-jacket and wasps. The other two known allergens of vespid venoms are phospholipase A₁ and hyaluronidase. Antigen 5 from *Vespula vulgaris* (*Ves v* 5) has been cloned and expressed as recombinant protein in the yeast system (Monsalve et al. 1999, ref. 22). The three-dimensional crystal structure of recombinant *Ves v* 5 has recently been determined at 1.8 Å resolution (in preparation). The main features of the structure consist of four β-strands and four α-helices arranged in three stacked layers giving rise to a "α-β-α sandwich". The sequence identity between Antigen 5 homologous allergens from different *Vespula* species is about 90% suggesting presence of conserved molecular surface areas and B cell epitopes.

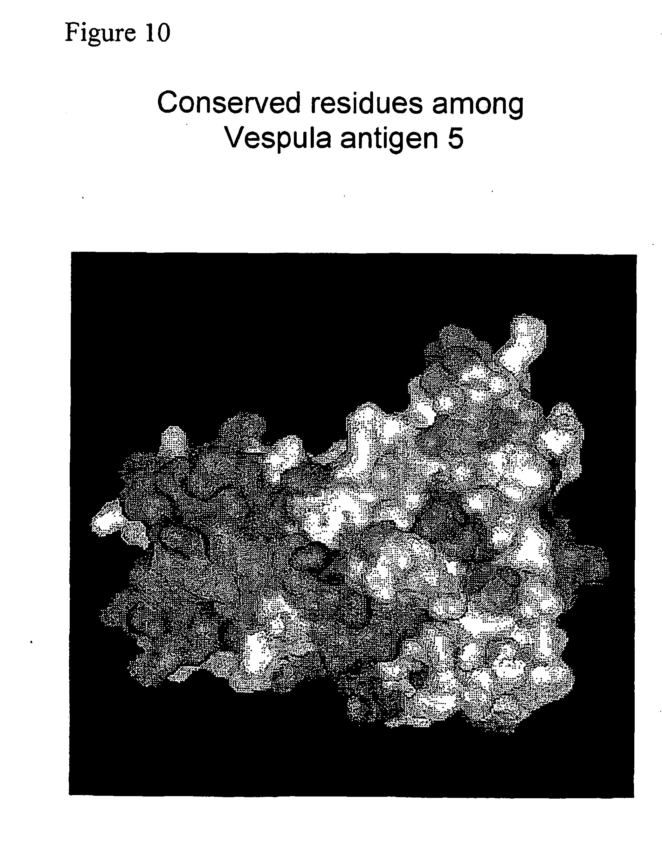

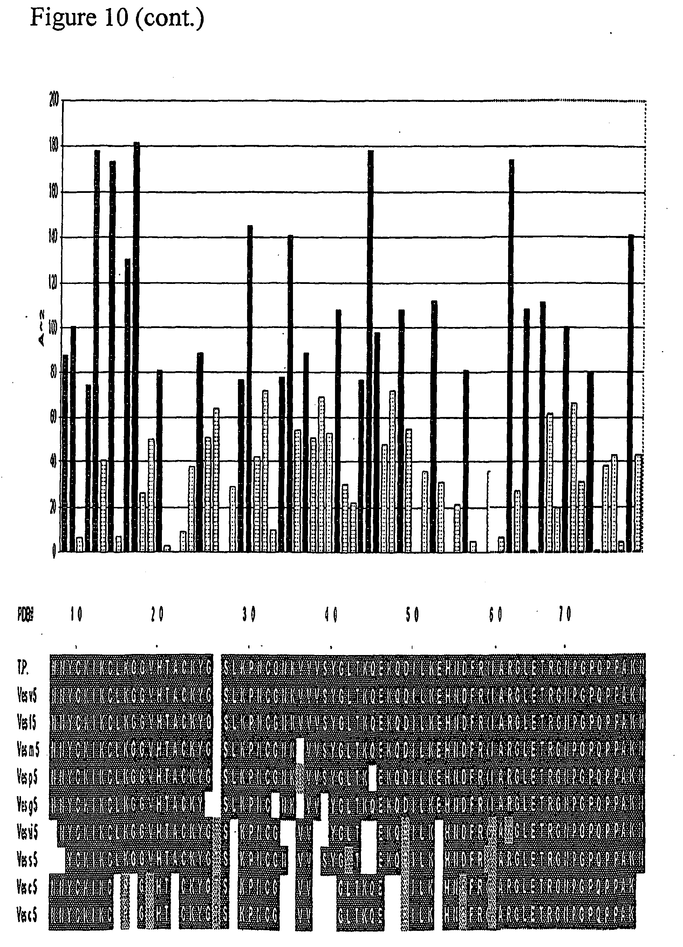

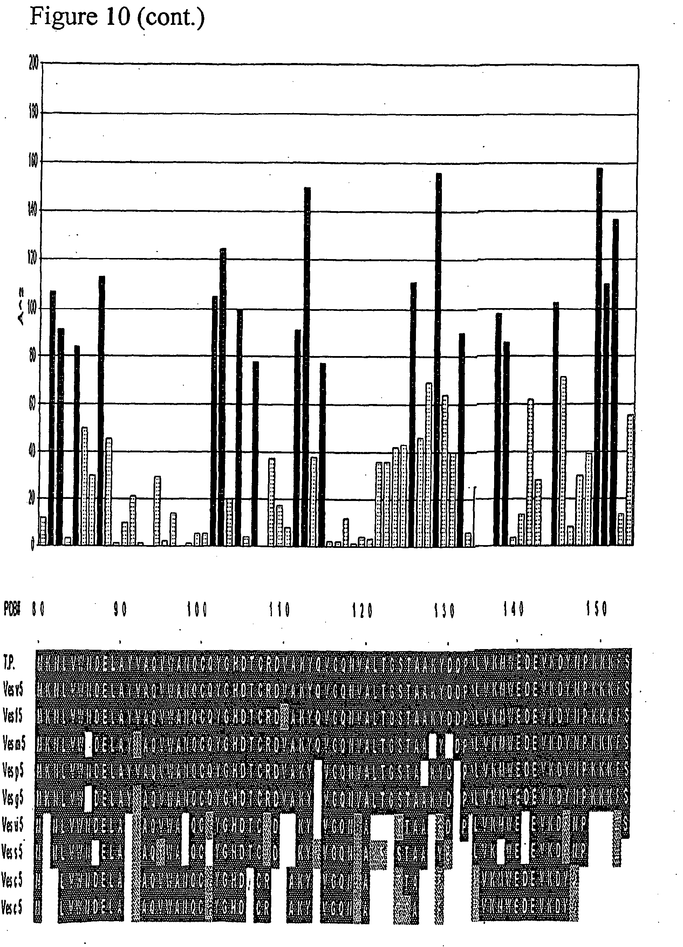

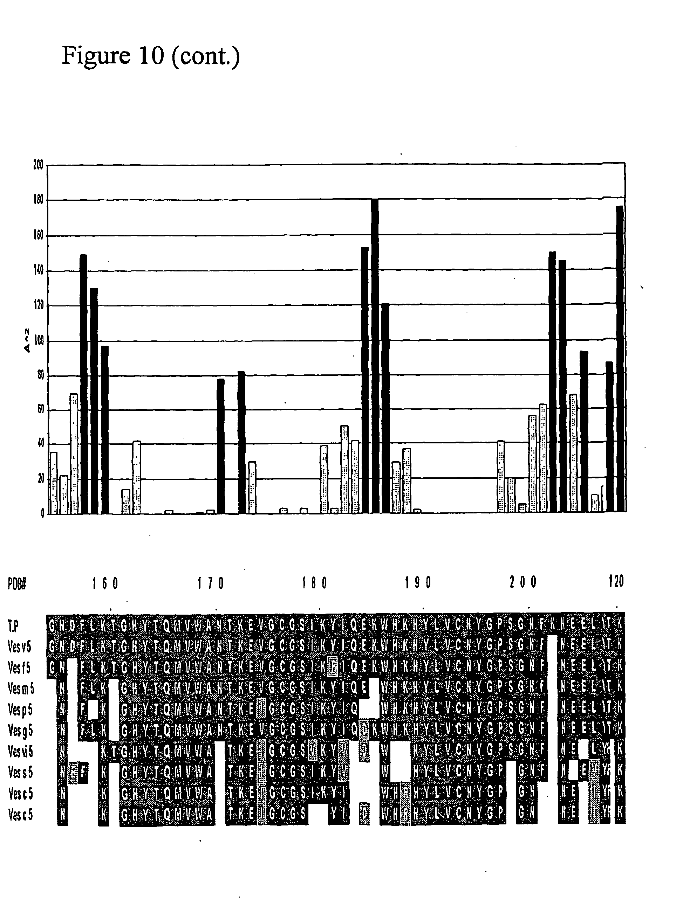

The presence and identification of common patches was performed after alignment of all known amino acid sequences, as previously described for tree pollen allergens, of the Vespula antigen 5 allergens in combination with an analysis of the molecular surface of Antigen 5 revealed by the three-dimensional structure of Ves v 5. Figure 10 shows solvent accessibility of individually aligned antigen 5 residues and alignment of *Vespula* antigen 5 sequences (left panel). On the right panel of figure 10 is shown the molecular surface of antigen 5 with conserved areas among *Vespula* antigen 5:s coloured.

### Selection of amino acid residues for site-directed mutagenesis

Amino acid residues for site-directed mutagenesis were selected among residues present the patches common for *Vespula* since modifications of these is expected to affect the binding of serum IgE from the majority of patients showing clinical *Vespula* allergic cross-reactivity.

The relative orientation and percentage of solvent-exposure of each amino acid residue within respective patch was calculated based on their atomic coordinates. Residues having a low degree of solvent exposure were not regarded suitable for mutagenesis due to the possible disruption of the structure or lack of antibody interaction. The remaining residues were ranked according to their degree of solvent-exposure.

### Cloning of the gene encoding Ves v 5

Total RNA was isolated from venom acid glands of *Vespula vulgaris* vespids as described in (Fang *et al.* 1988, ref. 23).

First-strand cDNA synthesis, PCR amplification and cloning of the *Ves v* 5 gene was performed as described in (Lu *et al*. 1993, ref. 24)

### Subcloning into pPICZαA

The gene encoding *Ves v* 5 was subsequently sub-cloned into the pPICZαA vector (Invitrogen) for secreted expression of *Ves* v 5 in *Pichia pastoris*. The gene was amplified by PCR and sub-cloned in frame with the coding sequence for the α-factor secretion signal of *Saccharomyces cerevisiae.* In this construct the α-factor is cleaved off, *in vivo,* by the *Pichia pastoris* Kex2 protease system during secretion of the protein.

In brief PCR was performed using *Ves v* 5 as template and primers corresponding to the amino- and carboxyterminus of the protein, respectively. The primers were extended in the 5'-end to accommodate restriction sites for cloning, EcoRI and XbaI, respectively. Nucleotides encoding the Kex2 cleavage site was in this construct positioned 18 nucleotides upstream to the amino terminus of the protein, resulting in the expression of *Ves v* 5 with six additional amino acids, Glu-Ala-Glu-Ala-Glu-Phe, at the amino terminus.

### Insertion of pPICZαA-Ves v 5 into P. pastoris

The pPICZαA vectors with the *Ves v* 5 gene inserted was linearised by Sac I restriction and inserted into the *AOX1* locus on the *Pichia pastoris* genome. Insertion was performed by homologous recombination on *Pichia pastoris* KM71 cells following the recommendations of Invitrogen.

### In vitro mutagenesis

*In vitro* mutagenesis was performed by PCR using recombinant pPICZαA with *Ves v* 5 inserted as template. Each mutant *Ves v* 5 gene was generated by 3 PCR reactions using 4 primers.

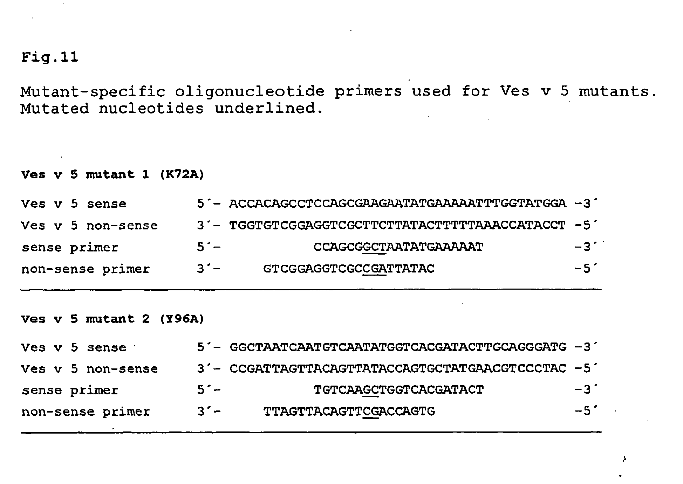

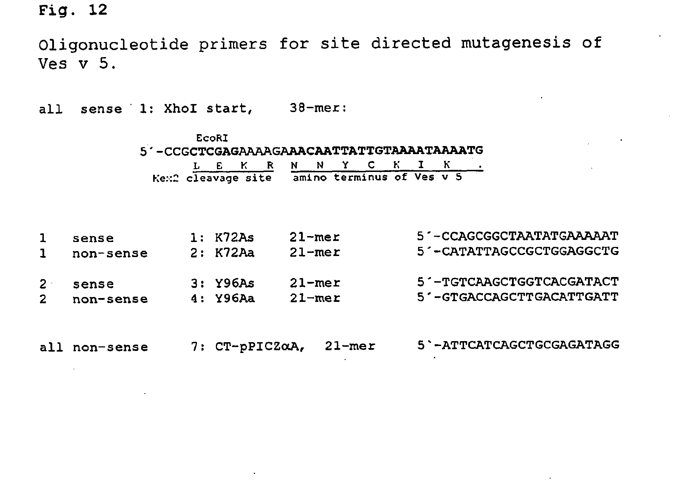

Two mutation-specific oligonucleotide primers were synthesised accommodating each mutation, one for each DNA strand, see Figures 11 and 12. Using the mutated nucleotide(s) as starting point both primers were extended 6-7 nucleotides in the 5'-end and 12-13 nucleotides in the 3'-end. The extending nucleotides were identical in sequence to the *Ves v* 5 gene in the actual region.

Two generally applicable primers (denoted "all sense" and "all non-sense" in Figure 12) were furthermore synthesised and used for all mutants. To insure expression of *Ves v* 5 mutants with authentic amino terminus, one primer corresponding to the amino terminus of the protein was extended in the 5'-end with a Xho I site. Upon insertion of the *Ves v* 5 mutant genes into the pPICZαA vector, the Kex2 protease cleavage site was regenerated directly upstream to the amino terminus of Ves v 5. The second primer was corresponding in sequence to a region of the pPICZαA vector positioned approximately 300 bp downstream from the *Ves v* 5 gene. The sequence of the primer corresponding to the amino terminus of *Ves v* 5 is derived from the sense strand and the sequence of the downstream primer is derived from the non-sense strand, see Figure 11.

Two independent PCR reactions were performed essentially according to standard procedures (Saiki *et al* 1988) with the exception that only 20 temperature cycles were performed in order to reduce the frequency of PCR artefacts. Each PCR reaction used pPICZαA with *Ves v* 5 inserted as template and one mutation-specific and one generally applicable primer in meaningful combinations.

The PCR products were purified by using "Concert, Rapid PCR Purification System" (Life Technologies). A third PCR reaction was performed using the combined PCR products from the first two PCR reactions as template and both generally applicable primers. Again, 20 cycles of standard PCR were used. The PCR product was purified with the "Concert, Rapid PCR Purification System" (Life Technologies), cut with restriction enzymes (Xho*I*/Xba*I*), and ligated directionally into pPICZαA vector restricted with the same enzymes. Figure 13 shows an overview of all Ves v 5 mutations.

### Insertion of pPICZαA-Ves v 5 mutants into P. pastoris

The pPICZαA vectors with the *Ves v* 5 *mutant* genes inserted were linearised by Sac I restriction and inserted into the AOX1 locus on the *Pichia pastoris* genome. Insertions were performed by homologous recombination on *Pichia pastoris* KM71 cells following the recommendations of Invitrogen.

### Nucleotide sequencing

Determination of the nucleotide sequence of the Ves v 5 encoding gene was performed before and after subcloning, and following *in vitro* mutagenesis, respectively.

Plasmid DNA's from 10 ml of bacterial culture grown to saturation overnight in LB medium supplemented with 0.1 g/l ampicillin were purified on Qiagen-tip 20 columns and sequenced using the Sequenase version 2.0 DNA sequencing kit (USB) following the recommendations of the suppliers.

### Expression and purification of recombinant Ves v 5

Recombinant yeast cells of *Pichia pastoris* strain KM71 were grown in 500 ml bottles containing 100 ml of pH 6.0 phosphate buffer containing yeast nitrogen base, biotin, glycerol and histidine at 30°C with orbital shaking at 225 rpm until A₆₀₀ nm of 4-6. Cells were collected by centrifugation and re-suspended in 10 ml of similar buffered medium containing methanol in place of glycerol. Incubation was continued at 30°C for 7 days with daily addition of 0.05 ml methanol.

Cells were harvested by centrifugation and the collected culture fluid was concentrated by ultrafiltration. After dialysis against 50 mM ammonium acetate buffer, pH 4.6, the sample was applied to a FPLC (Pharmacia) SE-53 cation exchange column equilibrated in the same buffer. The column was eluated with a 0-1.0 M NaCl, 50 mM ammonium acetate linear gradient. The recombinant Ves v 5 peak eluting at about 0.4 M NaCl was collected and dialysed against 0.02 N acetic acid. After concentration to about 10 mg/ml, the purified Ves v 5 was stored at 4°C.

### Crystallisation of recombinant Ves v 5

Crystals of *Ves v* 5 was grown by the vapour diffusion technique at 25°C. For crystallisation, 5 µl of 5 mg/ml Ves v 5 was mixed with 5 µl of 18% PEG 6000, 0.1 M sodium citrate, pH 6.0 and equilibrated against 1 ml of 18% PEG 6000, 0.1 M sodium citrate, pH 6.0.

X-ray diffraction data was collected at 100K from native *Ves* v 5 crystals and after incorporation of heavy-atom derivatives and used to solve the three-dimensional structure of *Ves v* 5, see Figure 10 (manuscript in preparation).

### Immunoelectrophoresis using rabbit polyclonal antibodies

The two *Ves v* 5 mutants were produced as recombinant *Ves v* 5 proteins and tested for their reactivity towards polyclonal rabbit antibodies raised against recombinant *Ves* v 5. When analysed by rocket immunoelectrophoresis under native conditions, the rabbit antibodies were able to precipitate recombinant *Ves v* 5 as well as both mutants, indicating that the mutants have conserved α-carbon backbone tertiary structure.

### Inhibition of specific serum IgE

The IgE-binding properties of *Ves v* 5 mutants were compared to recombinant *Ves v* 5 in a fluid-phase IgE-inhibition assay using a pool of serum IgE derived from vespid venom allergic patients.

The inhibition assay was performed as described above using biotinylated recombinant *Ves v* 5 instead of *Bet v* 1.

### Ves v 5 Lys72Ala mutant

Lysine in position 72 show a high degree of solvent-exposure (70%) and is located in a molecular surface patch common for *Vespula* antigen 5. The relative orientation and high degree of solvent exposure argued for that lysine 72 can be replaced by an alanine residue without distortion of the α-carbon backbone tertiary structure. In addition, as none of the naturally occurring isoallergen sequences have alanine in position 72, the substitution of lysine with alanine gives rise to a non-naturally occurring *Ves v* 5 molecule.

### IgE-binding properties of Ves v 5 Lys72Ala mutant

The IgE-binding properties of *Ves v* 5 Lys72Ala mutant was compared with recombinant *Ves v* 5 in a fluid-phase IgE-inhibition assay using the pool of serum IgE derived from birch allergic patients described above.

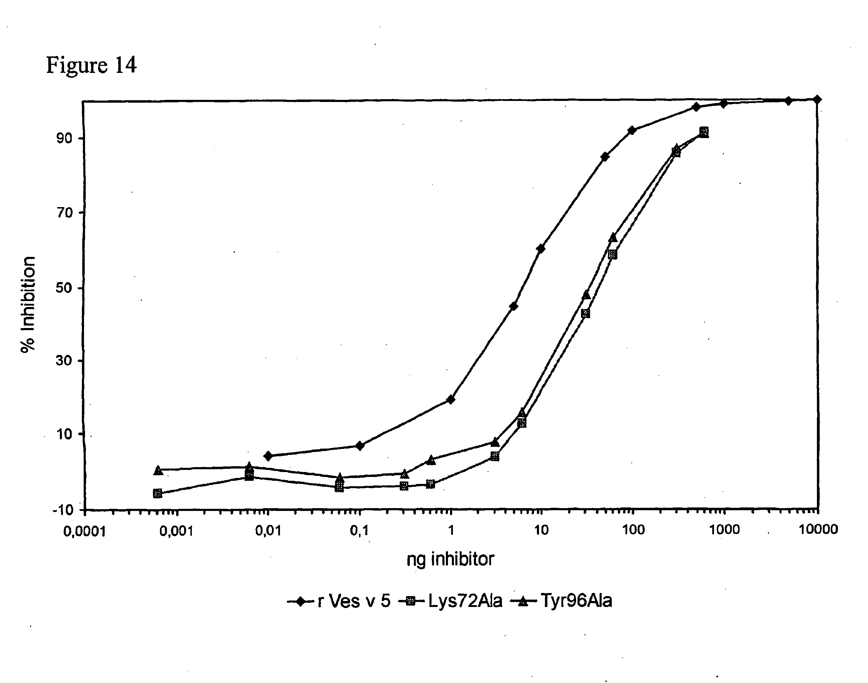

Figure 14 shows the inhibition of the binding of biotinylated recombinant *Ves v* 5 to serum IgE from a pool of allergic patients by non-biotinylated *Ves v* 5 and by *Ves v* 5 Lys72Ala mutant.

There is a clear difference in the amount of respective recombinant proteins necessary to reach 50% inhibition of the binding to serum IgE present in the serum pool. Recombinant *Ves v* 5 reaches 50% inhibition at about 6 ng whereas the corresponding concentration for *Ves v* 5 Lys72Ala mutant is 40 ng. This show that the single point mutation introduced in *Ves v* 5 Lys72Ala mutant lowers the affinity for specific serum IgE by a factor of about 6. The maximum level of inhibition reached by the *Ves v* 5 Lys72Ala mutant significantly lower compared to recombinant *Ves v* 5. This may indicate that after the Lys72Ala substitution, some of the specific IgE present in the serum pool are unable to recognise the *Ves v* 5 Lys72Ala mutant.

### Ves v 5 Tyr96Ala mutant

Tyrosine in position 96 show a high degree of solvent-exposure (65%) and is located in a molecular surface patch common for *Vespula* antigen 5. The relative orientation an high degree of solvent exposure argued for that tyrosine 96 can be replaced by an alanine residue without distortion of the three-dimensional structure. In addition, as none of the naturally occurring isoallergen sequences have alanine in position 96, the substitution of tyrosine with alanine gives rise to a non-naturally occurring *Ves v* 5 molecule.

### IgE-binding properties of Ves v 5 Tyr96Ala mutant

The IgE-binding properties of *Ves v* 5 Tyr96Ala mutant was compared with recombinant *Ves v* 5 in a fluid-phase IgE-inhibition assay using the pool of serum IgE derived from birch allergic patients described above.

Figure 14 shows the inhibition of the binding of biotinylated recombinant *Ves v* 5 to serum IgE from a pool of allergic patients by non-biotinylated *Ves v* 5 and by *Ves v* 5 Tyr96Ala mutant.

There is a clear difference in the amount of respective recombinant proteins necessary to reach 50% inhibition of the binding to serum IgE present in the serum pool. Recombinant *Ves v* 5 reaches 50% inhibition at about 6 ng whereas the corresponding concentration for *Ves v* 5 Tyr96Ala mutant is 40 ng.

This show that the single point mutation introduced in *Ves v* 5 Tyr96Ala mutant lowers the affinity for specific serum IgE by a factor of about 6.

The maximum level of inhibition reached by the *Ves v* 5 Tyr96Ala mutant significantly lower compared to recombinant *Ves v* 5. This may indicate that after the Tyr96Ala substitution, some of the specific IgE present in the serum pool are unable to recognise the *Ves v* 5 Tyr96Ala mutant.

### EXAMPLE 3

### Identification and selection of amino acids for substitution

The parameters of solvent accessibility and conservation degree were used to identify and select surface-exposed amino acids suitable for substitution for the allergens *Bet v* 1, Der p 2 and Ves v 5.

### Solvent accessibility

Solvent accessibility was calculated using the software InsightII, version 97.0 (MSI) and a probe radius of 1.4 Å (Connolly surface).

Internal cavities were excluded from the analyses by filling with probes using the software PASS (Putative Active Sites with Spheres). Probes on the surface were subsequently removed manually.

### Conservation

### Bet v 1:

3-D structure is based on accession number Z80104 (1bv1.pdb).

38 other *Bet v* 1 sequences included in the analysis of conserved residues comprise accession numbers: P15494=X15877=Z80106, Z80101, AJ002107, Z72429, AJ002108, Z80105, Z80100, Z80103, AJ001555, Z80102, AJ002110, Z72436, P43183=X77271, Z72430, AJ002106, P43178=X77267, P43179=X77268, P43177=X77266, Z72438, P43180=X77269, AJ001551, P43185=X77273, AJ001557, Z72434, AJ001556, Z72433=P43186, AJ001554, X81972, Z72431, P45431=X77200, P43184=X77272, P43176=X77265, 547250, S47251, Z72435, Z72439, Z72437, S47249.

### Der p 2:

3-D structure is based on accession number P49278 (1a9v.pdb).
6 other Der p 2 sequences included in the analysis of conserved residues comprise the following substitutions:
ALK-G: V40L, T47S, M111L, D114N.
ALK-101: M76V.
ALK-102: V40L, T47S.
ALK-104: T47S, M111I, D114N.
ALK-113: T47S.
ALK-120: V40L, T47S, D114N.

### Ves v 5:

3-D structure is based on accession number Q05110 (pdb coordinates unpublished).

Another Ves v 5 sequence in the analysis of conserved residues contains one amino acid substitution: M202K.

### Results

### Bet v 1

59 amino acids highly solvent exposed:
K-129, E-60, N-47, K-65, P-108, N-159, D-93, K-123, K-32, D-125, R-145, D-109, T-77, E-127, Q-36, E-131, L-152, E-6, E-96, D-156, P-63, H-76, E-8, K-134, E-45, T-10, V-12, K-20, L-62, S-155, H-126, P-50, N-78, K-119, V-2, L-24, E-42, N-4, A-153, 1-44, E-138, G-61, A-130, R-70, N-28, P-35, S-149, K-103, Y-150, H-154, N-43, A-106, K-115, P-14, Y-5, K-137, E-141, E-87, E-73.

57 amino acids highly solvent exposed and conserved (>70%) :
K-129, E-60, N-47, K-65, P-108, N-159, D-93, K-123, K-32, D-125, R-145, D-109, E-127, Q-36, E-131, L-152, E-6, E-96, D-156, P-63, H-76, E-8, K-134, E-45, T-10, V-12, K-20, S-155, H-126, P-50, N-78, K-119, V-2, L-24, E-42, N-4, A-153, I-44, E-138, G-61, A-130, R-70, N-28F P-35, S-149, K-103, Y-150, H-154, N-43, A-106, K-115, P-14, Y-5, K-137, E-141, E-87, E-73.

23 mutations performed:
Y5V, T10P, D25E, N28T, K32Q, E42S, E45S, N47S, K55N, K65N, T77A, N78K, E96L, K97S, K103V, P108G, D109N, K123I, D125Y, K134E, R145E, D156H, +160N.

Table 1 shows a listing in descending order of solvent exposure of Bet v 1 amino acids. Column 1 lists the amino acid number starting from the amino-terminal, column 2 lists the amino acid in one letter abbreviation, column 3 lists the normalised solvent exposure index, column 4 lists the percent of known sequences having the concerned amino acid in this position.

**Table 1: Bet v 1**

| | | |
|---|---|---|
| NO AA | Solv_exp | Cons % |
| 129K | 1,000 | 90 |
| 60E | 0,986 | 97 |
| 47N | 0,979 | 100 |
| 65 K | 0,978 | 100 |
| 108P | 0,929 | 100 |
| 159N | 0,869 | 100 |
| 93D | 0,866 | 100 |
| 123K | 0,855 | 100 |
| 32K | 0,855 | 100 |
| 125D | 0,821 | 74 |
| 145R | 0,801 | 90 |
| 109D | 0,778 | 82 |
| 77T | 0,775 | 56 |
| 127E | 0,760 | 100 |
| 36Q | 0,749 | 95 |
| 131 E | 0,725 | 100 |
| 152L | 0,718 | 97 |
| 6E | 0,712 | 100 |
| 96E | 0,696 | 100 |
| 156D | 0,693 | 97 |
| 63P | 0,692 | 97 |
| 76H | 0,683 | 90 |
| 8 E | 0,638 | 97 |
| 134K | 0,630 | 100 |
| 45E | 0,623 | 100 |
| 10T | 0,613 | 97 |
| 12V | 0,592 | 100 |
| 20K | 0,584 | 100 |
| 62L | 0,575 | 5 |
| 155S | 0,568 | 97 |
| 126H | 0,551 | 95 |
| 50P | 0,541 | 100 |
| 78N | 0,538 | 100 |
| 119K | 0,529 | 100 |
| 2V | 0,528 | 100 |
| 24L | 0,528 | 100 |
| 42E | 0,519 | 100 |
| 4N | 0,517 | 95 |
| 153A | 0,513 | 100 |
| 44I | 0,508 | 97 |
| 138E | 0,496 | 100 |
| 61G | 0,488 | 100 |
| 130A | 0,479 | 97 |
| 70R | 0,474 | 100 |
| 28N | 0,469 | 90 |
| 35P | 0,467 | 100 |
| 149S | 0,455 | 92 |
| 103K | 0,447 | 100 |
| 150Y | 0,438 | 100 |
| 154H | 0,436 | 100 |
| 43N | 0,412 | 100 |
| 106A | 0,411 | 95 |
| 115K | 0,411 | 100 |
| 14P | 0,410 | 97 |
| 5Y | 0,410 | 100 |
| 137K | 0,396 | 100 |
| 141 E | 0,387 | 95 |
| 87E | 0,385 | 100 |
| 73E | 0,384 | 100 |
| 16A | 0,367 | 100 |
| 79F | 0,362 | 100 |
| 3F | 0,355 | 100 |
| 158Y | 0,346 | 100 |
| 105V | 0,336 | 100 |
| 101 E | 0,326 | 100 |
| 64F | 0,325 | 100 |
| 86I | 0,322 | 100 |
| 39S | 0,314 | 100 |
| 124G | 0,310 | 100 |
| 72D | 0,308 | 97 |
| 142T | 0,293 | 67 |
| 66Y | 0,289 | 100 |
| 55K | 0,288 | 100 |
| 7T | 0,279 | 67 |
| 40S | 0,274 | 95 |
| 25D | 0,271 | 87 |
| 135A | 0,267 | 92 |
| 68K | 0,262 | 100 |
| 97K | 0,247 | 100 |
| 46G | 0,235 | 100 |
| 27D | 0,232 | 97 |
| 1 G | 0,227 | 100 |
| 113I | 0,225 | 77 |
| 51 G | 0,220 | 100 |
| 92G | 0,218 | 100 |
| 80K | 0,212 | 100 |
| 110G | 0,211 | 100 |
| 107T | 0,203 | 85 |
| 94T | 0,202 | 92 |
| 41 V | 0,201 | 97 |
| 48G | 0,198 | 100 |
| 91I | 0,192 | 18 |
| 31 P | 0,188 | 100 |
| 75D | 0,188 | 97 |
| 33V | 0,183 | 100 |
| 49G | 0,176 | 100 |
| 17R | 0,172 | 100 |
| 99S | 0,158 | 64 |
| 89G | 0,154 | 100 |
| 53I | 0,154 | 100 |
| 121 H | 0,153 | 100 |
| 9T | 0,150 | 72 |
| 74V | 0,148 | 97 |
| 132Q | 0,146 | 72 |
| 57S | 0,137 | 49 |
| 148E | 0,135 | 100 |
| 82N | 0,133 | 41 |
| 128V | 0,125 | 64 |
| 117S | 0,124 | 87 |
| 90P | 0,117 | 67 |
| 116I | 0,112 | 100 |
| 122T | 0,107 | 100 |
| 139M | 0,104 | 62 |
| 95L | 0,104 | 97 |
| 54K | 0,096 | 100 |
| 146A | 0,095 | 100 |
| 59P | 0,088 | 97 |
| 157A | 0,088 | 100 |
| 133V | 0,077 | 44 |
| 88G | 0,068 | 100 |
| 140G | 0,053 | 85 |
| 37A | 0,042 | 95 |
| 81Y | 0,041 | 100 |
| 23I | 0,036 | 95 |
| 104I | 0,036 | 92 |
| 15A | 0,036 | 97 |
| 58F | 0,029 | 100 |
| 29L | 0,028 | 100 |
| 19F | 0,027 | 100 |
| 100N | 0,022 | 97 |
| 22F | 0,021 | 97 |
| 71 V | 0,014 | 100 |
| 111 G | 0,014 | 100 |
| 13I | 0,014 | 100 |
| 18L | 0,014 | 97 |
| 114L | 0,014 | 100 |
| 11 S | 0,007 | 100 |
| 151 L | 0,007 | 97 |
| 144L | 0,007 | 90 |
| 52T | 0,007 | 100 |
| 84S | 0,007 | 97 |
| 118N | 0,007 | 97 |
| 102I | 0,007 | 100 |
| 21A | 0,000 | 97 |
| 26G | 0,000 | 97 |
| 30F | 0,000 | 44 |
| 34A | 0,000 | 100 |
| 38I | 0,000 | 87 |
| 56I | 0,000 | 100 |
| 67V | 0,000 | 97 |
| 69D | 0,000 | 62 |
| 83Y | 0,000 | 95 |
| 85V | 0,000 | 72 |
| 98I | 0,000 | 95 |
| 112S | 0,000 | 77 |
| 120Y | 0,000 | 95 |
| 136S | 0,000 | 67 |
| 143L | 0,000 | 100 |
| 147V | 0,000 | 100 |

### Der p 2

55 amino acids highly solvent exposed:
R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, D-114, L-17, G-60, P-95, E-53, V-81, K-51, N-103, Q-2, N-46, E-42, T-91, D-87, N-10, M-111, C-8, H-124, I-68, P-79, K-109, K-15.

54 amino acids highly solvent exposed and conserved (>70%):
R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109, K-15.

6 mutations performed:
K6A, K15E, H30N, E62S, H74N, K82N

Table 2 shows a listing in descending order of solvent exposure of Der p 2 amino acids. Column 1 lists the amino acid number starting from the amino-terminal, column 2 lists the amino acid in one letter abbreviation, column 3 lists the normalised solvent exposure index, column 4 lists the percent of known sequences having the concerned amino acid in this position.

**Table 2: Der p 2**

| NO AA | Solv_exp | Cons % |
|---|---|---|
| 128R | 1,000 | 100 |
| 129D | 0,965 | 100 |
| 11H | 0,793 | 100 |
| 30H | 0,712 | 100 |
| 1 S | 0,700 | 100 |
| 77K | 0,694 | 100 |
| 75Y | 0,681 | 100 |
| 31 R | 0,677 | 100 |
| 82K | 0,658 | 100 |
| 6K | 0,645 | 100 |
| 96K | 0,643 | 100 |
| 48K | 0,642 | 100 |
| 55K | 0,641 | 100 |
| 89K | 0,627 | 100 |
| 85 Q | 0,624 | 100 |
| 92W | 0,610 | 100 |
| 97I | 0,581 | 100 |
| 22H | 0,568 | 100 |
| 65V | 0,559 | 100 |
| 24S | 0,557 | 100 |
| 74H | 0,542 | 100 |
| 126K | 0,542 | 100 |
| 61 L | 0,539 | 100 |
| 26P | 0,516 | 100 |
| 93N | 0,513 | 100 |
| 64D | 0,509 | 100 |
| 28I | 0,504 | 100 |
| 14K | 0,493 | 100 |
| 100K | 0,489 | 100 |
| 62E | 0,454 | 100 |
| 127I | 0,439 | 100 |
| 102E | 0,428 | 100 |
| 25E | 0,428 | 100 |
| 66P | 0,427 | 100 |
| 114D | 0,418 | 57 |
| 17L | 0,412 | 100 |
| 60G | 0,390 | 100 |
| 95P | 0,388 | 100 |
| 53E | 0,377 | 100 |
| 81 V | 0,377 | 100 |
| 51K | 0,370 | 100 |
| 103N | 0,369 | 100 |
| 2Q | 0,366 | 100 |
| 46N | 0,360 | 100 |
| 42E | 0,357 | 100 |
| 91 T | 0,340 | 100 |
| 87D | 0,334 | 100 |
| 10N | 0,333 | 100 |
| 111M | 0,325 | 71 |
| 8C | 0,323 | 100 |
| 124H | 0,315 | 100 |
| 68I | 0,313 | 100 |
| 79P | 0,307 | 100 |
| 109K | 0,307 | 100 |
| 15K | 0,302 | 100 |
| 49T | 0,292 | 100 |
| 44N | 0,291 | 100 |
| 113D | 0,290 | 100 |
| 63 V | 0,286 | 100 |
| 105V | 0,280 | 100 |
| 19P | 0,270 | 100 |
| 84Q | 0,264 | 100 |
| 76M | 0,262 | 86 |
| 7D | 0,251 | 100 |
| 116V | 0,244 | 100 |
| 78C | 0,238 | 100 |
| 36Q | 0,235 | 100 |
| 45Q | 0,233 | 100 |
| 40V | 0,223 | 57 |
| 57S | 0,212 | 100 |
| 38E | 0,205 | 100 |
| 69D | 0,203 | 100 |
| 9A | 0,196 | 100 |
| 71N | 0,190 | 100 |
| 98A | 0,188 | 100 |
| 115G | 0,180 | 100 |
| 13I | 0,179 | 100 |
| 123T | 0,179 | 100 |
| 34P | 0,178 | 100 |
| 4D | 0,157 | 100 |
| 20G | 0,150 | 100 |
| 107T | 0,143 | 100 |
| 12E | 0,137 | 100 |
| 94V | 0,137 | 100 |
| 121I | 0,136 | 100 |
| 83G | 0,128 | 100 |
| 70P | 0,128 | 100 |
| 73C | 0,120 | 100 |
| 3V | 0,116 | 100 |
| 35F | 0,111 | 100 |
| 59D | 0,099 | 100 |
| 29I | 0,098 | 100 |
| 23G | 0,085 | 100 |
| 54I | 0,075 | 100 |
| 5V | 0,075 | 100 |
| 101S | 0,074 | 100 |
| 72A | 0,069 | 100 |
| 27C | 0,060 | 100 |
| 32G | 0,059 | 100 |
| 99P | 0,058 | 100 |
| 86Y | 0,056 | 100 |
| 16V | 0,052 | 100 |
| 50A | 0.040 | 100 |
| 90Y | 0,039 | 100 |
| 18V | 0,035 | 100 |
| 33K | 0,033 | 100 |
| 52I | 0,029 | 100 |
| 58I | 0,029 | 100 |
| 104V | 0,024 | 100 |
| 112G | 0,023 | 100 |
| 21C | 0,023 | 100 |
| 88I | 0,023 | 100 |
| 117L | 0,016 | 100 |
| 56A | 0,011 | 100 |
| 41 F | 0,011 | 100 |
| 120A | 0,006 | 100 |
| 119C | 0,006 | 100 |
| 67G | 0,005 | 100 |
| 122A | 0,005 | 100 |
| 37L | 0,000 | 100 |
| 39A | 0,000 | 100 |
| 43A | 0,000 | 100 |
| 47T | 0,000 | 29 |
| 80L | 0,000 | 100 |
| 106V | 0,000 | 100 |
| 108V | 0,000 | 100 |
| 110V | 0,000 | 100 |
| 118A | 0,000 | 100 |
| 125A | 0,000 | 100 |

### Ves v 5

89 amino acids highly solvent exposed:
K-16, K-185, K-11, K-44, K-210, R-63, K-13, F-6, K-149, K-128, E-184, K-112, K-202, F-157, E-3, K-29, N-203, N-34, K-78, K-151, L-15, L-158, Y-102, W-186, K-134, D-87, K-52, T-67, T-125, K-150, Y-40, Q-48, L-65, K-81, Q-101, Q-208, K-144, N-8, N-70, H-104, Q-45, K-137, K-159, E-205, N-82, A-111, D-131, K-24, V-36, N-7, M-138, T-209, V-84, K-172, V-19, D-56, P-73, G-33, T-106, N-170, L-28,-T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85, I-182. 88 amino acids highly solvent exposed and conserved (>70%):
K-16, K-185, K-11, K-44, K-210, R-63, K-13, F-6, K-149, K-128, E-184, K-112, F-157, E-3, K-29, N-203, N-34, K-78., K-151, L-15, L-158, Y-102, W-186, K-134, D-87, K-52, T-67, T-125, K-150, Y-40, Q-48, L-65, K-81, Q-101, Q-208, K-144, N-8, N-70, H-104, Q-45, K-137, K-159, E-205, N-82, A-111, D-131, K-24, V-36, N-7, M-138, T-209, V-84, K-172, V-19, D-56, P-73, G-33, T-106, N-170, L-28, T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85, I-182.

9 mutations performed:
K29A, T67A, K78A, V84S, Y102A, K112S, K144A, K202M, N203G

Table 3 shows a listing in descending order of solvent exposure of Ves v 5 amino acids. Column 1 lists the amino acid number starting from the amino-terminal, column 2 lists the amino acid in one letter abbreviation, column 3 lists the normalised solvent exposure index, column 4 lists the percent of known sequences having the concerned amino acid in this position.

**Table 3: Ves v 5**

| NO AA | Solv_exp | |
|---|---|---|
| 16K | 1,000 | 100 |
| 185K | 0,989 | 100 |
| 11K | 0,978 | 100 |
| 44K | 0,978 | 100 |
| 210K | 0,962 | 100 |
| 63R | 0,956 | 100 |
| 13K | 0,951 | 100 |
| 6F | 0,868 | 100 |
| 149K | 0,868 | 100 |
| 128K | 0,857 | 100 |
| 184E | 0,841 | 100 |
| 112K | 0,824 | 100 |
| 202K | 0,824 | 50 |
| 157F | 0,819 | 100 |
| 3E | 0,802 | 100 |
| 29K | 0,797 | 100 |
| 203N | 0,797 | 100 |
| 34N | 0,775 | 100 |
| 78K | 0,775 | 100 |
| 151K | 0,753 | 100 |
| 15L | 0,714 | 100 |
| 158L | 0,714 | 100 |
| 102Y | 0,687 | 100 |
| 186W | 0,665 | 100 |
| 134K | 0,654 | 100 |
| 87D | 0,621 | 100 |
| 52K | 0,615 | 100 |
| 67T | 0,610 | 100 |
| 125T | 0,610 | 100 |
| 150K | 0,604 | 100 |
| 40Y | 0,593 | 100 |
| 48Q | 0,593 | 100 |
| 65L | 0,593 | 100 |
| 81 K | 0,588 | 100 |
| 101Q | 0,577 | 100 |
| 208Q | 0,566 | 100 |
| 144K | 0,560 | 100 |
| 8N | 0,555 | 100 |
| 70N | 0,549 | 100 |
| 104H | 0,549 | 100 |
| 45Q | 0,538 | 100 |
| 137K | 0,538 | 100 |
| 159K | 0,533 | 100 |
| 205E | 0,511 | 100 |
| 82N | 0,500 | 100 |
| 111A | 0,500 | 100 |
| 131 D | 0,495 | 100 |
| 24K | 0,489 | 100 |
| 36V | 0,489 | 100 |
| 7N | 0,484 | 100 |
| 138M | 0,473 | 100 |
| 209T | 0,473 | 100 |
| 84V | 0,462 | 100 |
| 172K | 0,451 | 100 |
| 19V | 0,445 | 100 |
| 56D | 0,445 | 100 |
| 73P | 0,440 | 100 |
| 33G | 0,429 | 100 |
| 106T | 0,429 | 100 |
| 170N | 0,429 | 100 |
| 28L | 0,423 | 100 |
| 43T | 0,423 | 100 |
| 114Q | 0,423 | 100 |
| 10C | 0,412 | 100 |
| 60K | 0,407 | 100 |
| 31 N | 0,396 | 100 |
| 47 K | 0,396 | 100 |
| 5E | 0,390 | 100 |
| 145D | 0,390 | 100 |
| 38V | 0,379 | 100 |
| 127A | 0,379 | 100 |
| 156D | 0,379 | 100 |
| 204 E | 0,374 | 100 |
| 71 P | 0,363 | 100 |
| 26G | 0,352 | 100 |
| 129Y | 0,352 | 100 |
| 141 D | 0,341 | 100 |
| 201 F | 0,341 | 100 |
| 68 R | 0,335 | 100 |
| 200N | 0,308 | 100 |
| 49 D | 0,302 | 100 |
| 153S | 0,302 | 100 |
| 35K | 0,297 | 100 |
| 39 S | 0,291 | 100 |
| 25Y | 0,280 | 100 |
| 37V | 0,280 | 100 |
| 18G | 0,275 | 100 |
| 85W | 0,275 | 100 |
| 182I | 0,275 | 100 |
| 46E | 0,264 | 100 |
| 126A | 0,253 | 100 |
| 88E | 0,247 | 100 |
| 76P | 0,236 | 100 |
| 79N | 0,236 | 100 |
| 124S | 0,236 | 100 |
| 30P | 0,231 | 100 |
| 123G | 0,231 | 100 |
| 162H | 0,231 | 100 |
| 183Q | 0,231 | 100 |
| 12I | 0,225 | 100 |
| 197P | 0,225 | 100 |
| 130D | 0,220 | 100 |
| 148P | 0,214 | 100 |
| 180K | 0,214 | 100 |
| 23C | 0,209 | 100 |
| 75P | 0,209 | 100 |
| 113Y | 0,209 | 100 |
| 108 R | 0,203 | 100 |
| 188 K | 0,203 | 100 |
| 51 L | 0,198 | 100 |
| 59Q | 0,198 | 100 |
| 121 L | 0,198 | 100 |
| 122T | 0,198 | 100 |
| 154G | 0,192 | 100 |
| 53E | 0,170 | 100 |
| 72G | 0,170 | 100 |
| 41 G | 0,165 | 100 |
| 86N | 0,165 | 100 |
| 147N | 0,165 | 100 |
| 173E | 0,165 | 100 |
| 27S | 0,159 | 100 |
| 94Q | 0,159 | 100 |
| 187H | 0,159 | 100 |
| 142E | 0,154 | 100 |
| 64G | 0,148 | 100 |
| 17G | 0,143 | 100 |
| 133V | 0,137 | 100 |
| 42L | 0,121 | 100 |
| 155N | 0,121 | 100 |
| 55N | 0,115 | 100 |
| 91Y | 0,115 | 100 |
| 69G | 0,110 | 100 |
| 103G | 0,110 | 100 |
| 198S | 0,110 | 100 |
| 109D | 0,093 | 100 |
| 207Y | 0,082 | 100 |
| 96W | 0,077 | 100 |
| 161 G | 0,077 | 100 |
| 140E | 0,071 | 100 |
| 152F | 0,071 | 100 |
| 80M | 0,066 | 100 |
| 117Q | 0,066 | 100 |
| 4A | 0,060 | 100 |
| 32C | 0,055 | 100 |
| 90A | 0,055 | 100 |
| 206L | 0,055 | 100 |
| 22A | 0,049 | 100 |
| 110V | 0,044 | 100 |
| 146Y | 0,044 | 100 |
| 14C | 0,038 | 100 |
| 9Y | 0,033 | 100 |
| 62A | 0,033 | 100 |
| 132P | 0,033 | 100 |
| 57F | 0,027 | 100 |
| 99Q | 0,027 | 100 |
| 100C | 0,027 | 100 |
| 199G | 0,027 | 100 |
| 77A | 0,022 | 100 |
| 105D | 0,022 | 100 |
| 119V | 0,022 | 100 |
| 20H | 0,016 | 100 |
| 83L | 0,016 | 100 |
| 120A | 0,016 | 100 |
| 139W | 0,016 | 100 |
| 176C | 0,016 | 100 |
| 178S | 0,016 | 100 |
| 181Y | 0,016 | 100 |
| 95V | 0,011 | 100 |
| 115V | 0,011 | 100 |
| 116G | 0,011 | 100 |
| 165Q | 0,011 | 100 |
| 169A | 0,011 | 100 |
| 189H | 0,011 | 100 |
| 66E | 0,005 | 100 |
| 74Q | 0,005 | 100 |
| 89L | 0,005 | 100 |
| 92V | 0,005 | 100 |
| 98N | 0,005 | 100 |
| 118N | 0,005 | 100 |
| 168W | 0,005 | 100 |
| 21T | 0,000 | 100 |
| 50I | 0,000 | 100 |
| 54H | 0,000 | 100 |
| 58 R | 0,000 | 100 |
| 61I | 0,000 | 100 |
| 93A | 0,000 | 100 |
| 97A | 0,000 | 100 |
| 107C | 0,000 | 100 |
| 135L | 0,000 | 100 |
| 136V | 0,000 | 100 |
| 143V | 0,000 | 100 |
| 160T | 0,000 | 100 |
| 163Y | 0,000 | 100 |
| 164T | 0,000 | 100 |
| 166M | 0,000 | 100 |
| 167V | 0,000 | 100 |
| 171T | 0,000 | 100 |
| 174V | 0,000 | 100 |
| 175G | 0,000 | 100 |
| 177G | 0,000 | 100 |
| 179I | 0,000 | 100 |
| 190Y | 0,000 | 100 |
| 191 L | 0,000 | 100 |
| 192V | 0,000 | 100 |
| 193C | 0,000 | 100 |
| 194N | 0,000 | 100 |
| 195Y | 0,000 | 100 |
| 196G | 0,000 | 100 |

### EXAMPLE 4

This Example describes preparation and characterisation of recombinant mutant Bet v 1 allergens according to the invention, i.e. allergens with diminished IgE-binding affinity comprising at least four primary mutations.

### Selection of amino acid residues for site-directed mutagenesis of Bet v 1

Solvent accessibility of amino acid residues of Bet v 1 is shown in Example 3, table 1. The rate of amino acid conservation is based on sequence alignment performed at the ExPaSy Molecular Biology Server (http://www.expasy.ch/) using the ClustalW algorithm on a BLAST search where the Bet V 1.2801 wild type amino acid sequence is used as input sequence. The alignment includes 67 allergen sequences (39 Bet v 1 sequences, 11 Car b 1 sequences, 6 Cor a 1 sequences, and 13 Aln g 1 sequences) from species within the order *Fagales* (Bet v 1: *Betula verrrucosa*; Car b 1: *Carpinus betulus*; Cor a 1: *Corylus avellana*; Aln g 1: *alnus glutinosa*). In respect to the mutated recombinant Bet v 1 allergens shown in the examples, target residues for substitution was based on ≥95% amino acid identity.

As described in Example 1, amino acid residues with a high degree of solvent-exposure and a high degree of conservation between pollen allergens from related species, were selected for site-directed mutagenesis. Residues having a low degree of solvent exposure (<20%) were not regarded relevant for mutagenesis due to the possible disruption of the tertiary structure or lack of antibody interaction.

The introduced residues were all present in corresponding positions in isoforms of a group of plant proteins called pathogenesis related (PR-10) proteins. Molecular modelling suggests that the tertiary structures of *Fagales* allergens and PR-10 proteins are close to being identical. Bet v 1 shares significant sequence identity (20-40%) with PR-10 proteins. However, there are no reports of allergic cross-reactivity towards these PR-10 proteins. Thus, exchange of a highly conserved and solvent exposed amino acid from Bet v 1 with an amino acid in the corresponding position in a PR-10 protein, results in a mutated Bet v 1 protein with an unaltered α-carbon backbone tertiary structure but with diminished IgE-binding affinity.

### In vitro mutagenesis

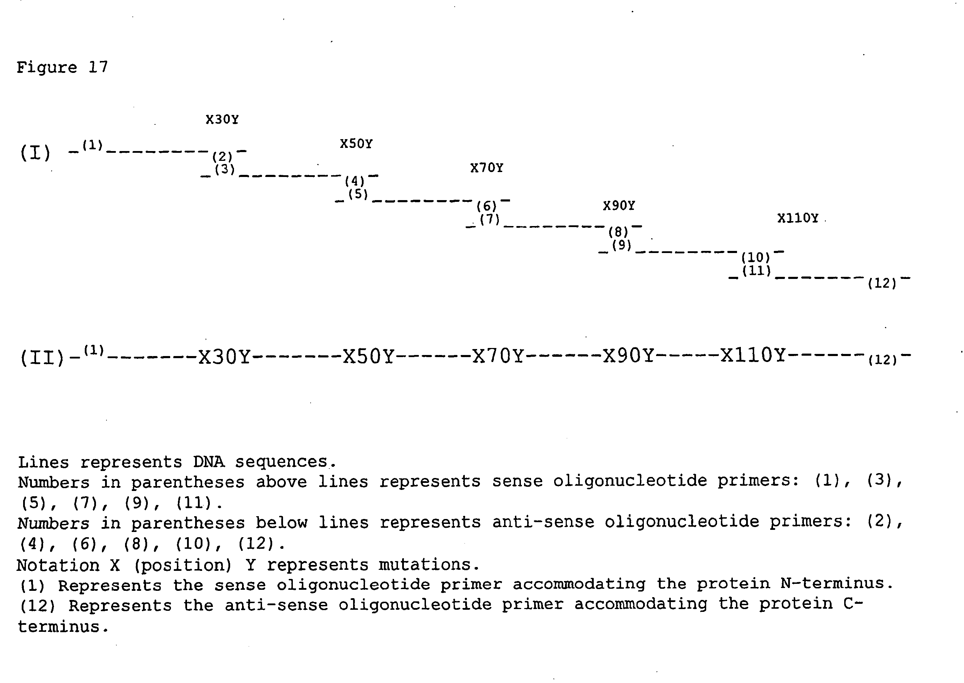

*In vitro* mutagenesis was performed by PCR using recombinant pMAL-c with *Bet v* 1 inserted as template. Preparation of recombinant mutant allergens comprising five to nine primary mutations included two PCR steps; step I and II. First, each single mutation (or several mutations if located closely together in the DNA sequence) was introduced into sequential DNA sequences of *Bet v 1.2801* or *Bet v 1.2801* derivatives using sense and anti-sense mutation-specific oligonucleotide primers accommodating each mutation(s) along with sense and anti-sense oligonucleotide primers accommodating either upstream or downstream neighbour mutations or the N-terminus/C-terminus of Bet v 1, respectively as schematically illustrated in Figure 17(I). Secondly, PCR products from PCR reaction I were purified, mixed and used as templates for an additional PCR reaction (II) with oligonucleotide primers accommodating the N-terminus and C-terminus of Bet v 1 as schematically illustrated in Figure 17 (II). The PCR products were purified by agarose gel electrophoresis and PCR gel purification (Life Techhnologies) followed by ethanol precipitation, cut with restriction enzymes *(SacI*/*EcoRI)* or *(SacI*/ *XbaI),* and ligated directionally into pMAL-c restricted with the same enzymes.

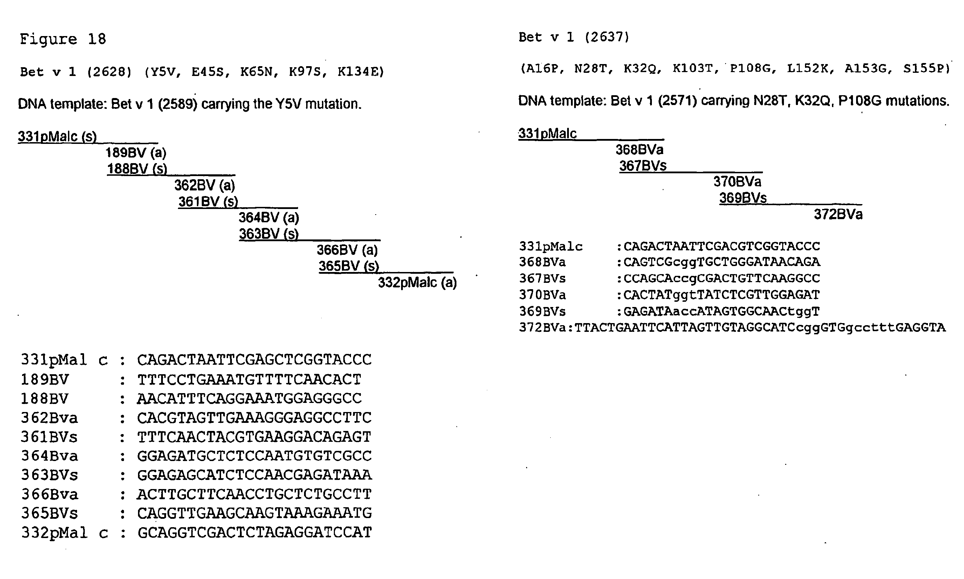

Figure 18 shows synthesised oligonucleotide primers and schematically illustrations for the construction of Bet v 1 mutants with five to nine primary mutations. The mutated amino acids were preferably selected from the group consisting of amino acids that are characterised by being highly solvent exposed and conserved as described in Example 3. The Bet v 1 mutants are the following primary and secondary mutations stated in parenthesis:
Mutant Bet v 1 (2628): Tyr5Val, Glu45Ser, Lys65Asn, Lys97Ser, Lys134Glu.
Mutant Bet v 1 (2637): Ala16Pro, (Asn28Thr, Lys32Gln), Lys103Thr, Pro108Gly, (Leu152Lys, Ala153Gly, Ser155Pro).
Mutant Bet v 1 (2733): (Tyr5Val, Lys134Glu), (Asn28Thr, Lys32Gln), Glu45Ser, Lys65Asn, (Asn78Lys, Lys103Val), Lys97Ser, Pro108Gly, Arg145Glu, (Asp156His, +160Asn)
Mutant Bet v 1 (2744): (Tyr5Val, Lys134Glu), (Glu42Ser, Glu45Ser), (Asn78Lys, Lys103Val), Lys123Ile, (Asp156His, +160Asn).
Mutant Bet v 1 (2753): (Asn28Thr, Lys32Gln), Lys65Asn, (Glu96Leu, Lys97Ser), (Pro108Gly, Asp109Asn), (Asp125Tyr, Glu127Ser), Arg145Glu.

### Nucleotide sequencing

Determination of the nucleotide sequence of the Bet v 1 encoding gene was performed before and after subcloning, and following in vitro mutagenesis, respectively.

Plasmid DNA's from 10 ml of bacterial culture grown to saturation overnight in LB medium supplemented with 0.1 g/l ampicillin were purified on Qiagen-tip 20 columns and sequenced using the Ready reaction dye terminator cycle sequencing kit and a Fluorescence Sequencer AB PRISM 377, both from (Perkin Elmer), following the recommendations of the suppliers.

### Expression and purification of recombinant Bet v 1 and mutants

Recombinant Bet v 1 (Bet v 1.2801 and mutants) were over-expressed in *Escherichia coli* DH 5α fused to maltose-binding protein and purified as described in ref. 15. Briefly, recombinant *E. coli* cells were grown at 37°C to an optical density of 0.8 at 600 nm, whereupon expression of Bet v 1 fusion protein was induced by addition of IPTG. Cells were harvested by centrifugation 3 hours post-induction, re-suspended in lysis buffer and broken by sonication. After sonication and additional centrifugation, recombinant fusion protein was isolated by amylose affinity chromatography and subsequently cleaved by incubation with Factor Xa (ref. 15). After F Xa cleavage, recombinant Bet v 1 was isolated by gelfiltration and subjected to another round of amylose affinity chromatography in order to remove trace amounts of maltose-binding protein.

Purified recombinant Bet v 1 was concentrated by ultrafiltration to about 5 mg/ml and stored at 4 °C. The final yields of the purified recombinant Bet v 1 preparations were between 2-5 mg per litre *E*. *coli* cell culture.

The purified recombinant Bet v 1 preparations appeared as single bands after silver-stained SDS-polyacrylamide electrophoresis with an apparent molecular weight of 17.5 kDa.

We have previously shown (ref. 15) that recombinant *Bet v* 1 No. 2801 is immunochemically indistinguishable from naturally occurring Bet v 1.

### Bet v 1 (2628) and Bet v 1 (2637) mutants

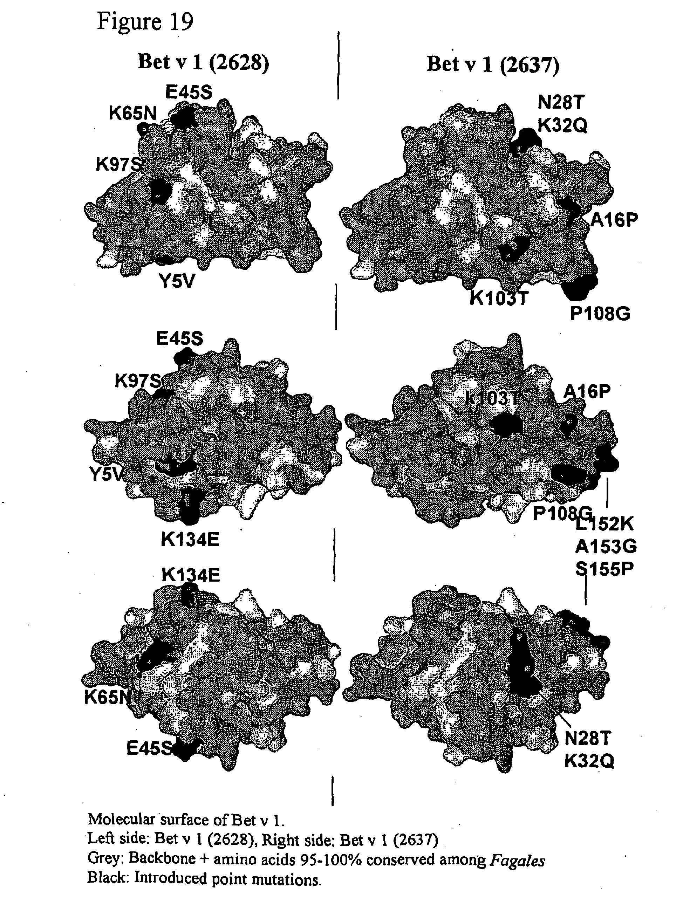

Figure 19 shows introduced point mutations at the molecular surface of Bet v 1 (2628) and Bet v 1 (2637). In mutant Bet v 1 (2628) five primary mutations were introduced in one half of Bet v 1 leaving the other half unaltered. In mutant Bet v 1(2637) five primary and three secondary mutations were introduced in the other half leaving the first half unaltered. In this way, mutations in mutant Bet v 1 (2628) and mutant Bet v 1(2637) affects different halves of the Bet v 1 surface, respectively.

### Crystallisation and structural determination of recombinant Bet v 1(2628) mutant protein.

Crystals of recombinant Bet v 1 (2628) were grown by vapour diffusion at 25°C, essentially as described in (Spangfort *et al* 1996b, ref. 21). Bet v 1 (2628), at a concentration of 5 mg/ml, was mixed with an equal volume of 2.2 M ammonium sulphate, 0.1 M sodium citrate, 1% (v/v) dioxane, pH 6.3 and equilibrated against 100x volume of 2.2 M ammonium sulfate, 0.1 M sodium citrate, 1% (v/v) dioxane, pH 6.3. After 24 hours of equilibration, crystal growth was induced by applying the seeding technique described in ref. 21, using crystals of recombinant wild-type *Bet v* 1 as a source of seeds.

After about 4 months, crystals were harvested and analysed using X-rays generated from a Rigaku rotating anode as described in ref. 21 and the structure was solved using molecular replacement.

### Structure of Bet v 1 (2628) mutant

The structural effect of the mutations was addressed by growing three-dimensional *Bet v* 1 (2628) protein crystals diffracting to 2.0 Å resolution when analysed by X-rays generated from a rotating anode. The substitutions Tyr5Val, Glu45Ser, Lys65Asn, Lys97Ser, Lys134Glu were verified by the Bet v 1 (2628) structure electron density map which also showed that the overall α-carbon backbone tertiary structure is preserved.

### Structural analysis of Bet v 1 (2637) mutant

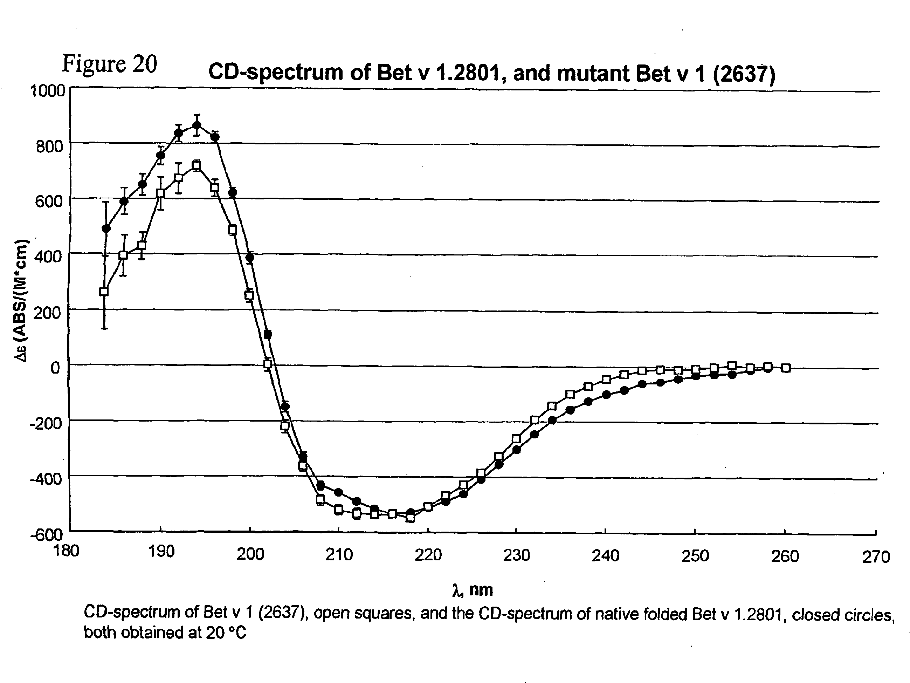

The structural integrity of the purified Bet v 1 (2637) mutant was analysed by circular dichroism (CD) spectroscopy. Figure 20 shows the CD spectra of recombinant Bet v 1.2801 (wildtype) and Bet v 1 (2637) mutant, recorded at close to equal concentrations. The overlap in peak amplitudes and positions in the CD spectra from the two recombinant proteins shows that the two preparations contain equal amounts of secondary structures strongly suggesting that the α-carbon backbone tertiary structure is not affected by the introduced amino acid substitutions.

### IgE-binding properties of Bet v 1 (2628) and Bet v 1 (2637) mutants.

The IgE-binding properties of Bet v 1 (2628) and Bet v 1 (2637) as well as a 1:1 mix of Bet v 1 (2628) and Bet v 1 (2637) was compared with recombinant wild type Bet v 1.2801 in a fluid-phase IgE-inhibition assay using a pool of serum IgE derived from birch allergic patients.

As described in Example 1, recombinant Bet v 1.2801 was biotinylated at a molar ratio of 1:5 (Bet v 1 no. 2801:biotic). The inhibition assay was performed as follows: a serum sample (25 µl) was incubated with solid phase anti IgE, washed, re-suspended and further incubated with a mixture of biotinylated Bet v 1.2801 and a given mutant or 1:1 mix of the two mutants. The amount of biotinylated Bet v 1.2801 bound to the solid phase was estimated from the measured RLU after incubation with acridinium ester labelled streptavidin. The degree of inhibition was calculated as the ratio between the RLU's obtained using buffer and mutant as inhibitor.

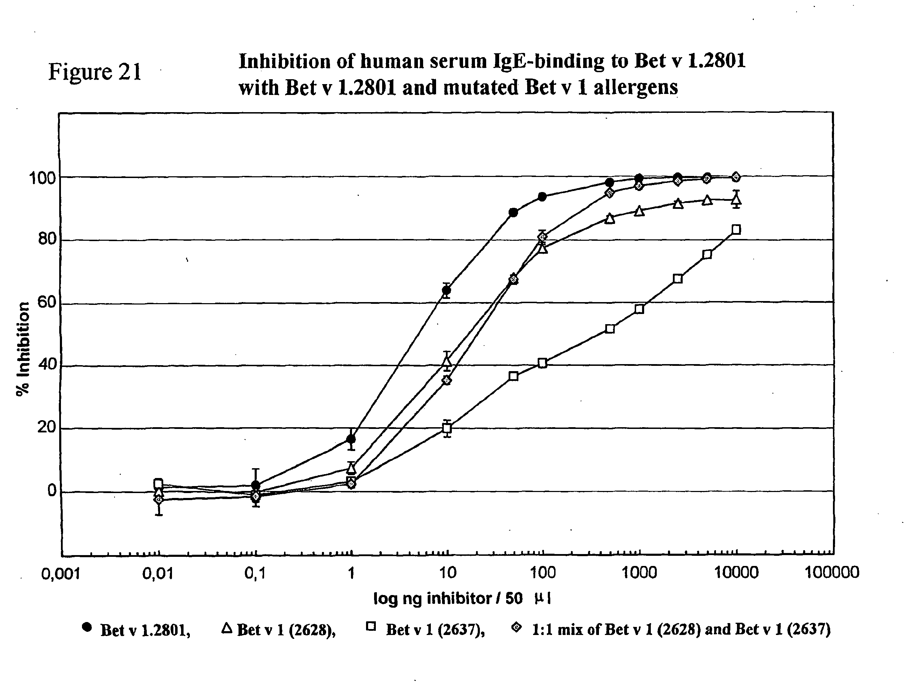

Figure 21 shows the inhibition of the binding of biotinylated recombinant Bet v 1.2801 to serum IgE from a pool of allergic patients by non-biotinylated Bet v 1.2801 and by Bet v 1 (2628), Bet v 1 (2637) and a 1:1 mix of Bet v 1 (2628) and Bet v 1 (2637).

There is a clear difference in the amount of respective recombinant proteins necessary to reach 50% inhibition of the binding to serum IgE present in the serum pool. Recombinant Bet v 1.2801 reaches 50% inhibition at about 5 ng whereas the corresponding concentration for Bet v 1 (2628) mutant is about 15-20 ng. This show that the point mutation introduced in the Bet v 1 (2628) mutant lowers the affinity for specific serum IgE by a factor of about 3-4.

The maximum level of inhibition reached by the Bet v 1 (2628) mutant protein is clearly lower compared to recombinant Bet v 1.2801. This may indicate that some of the specific IgE present in the serum pool are unable to recognise the Bet v 1 (2628) mutant protein due to the introduced point mutations.

Bet v 1 (2637) reaches 50% inhibition at about 400-500 ng showing that the point mutation introduced in the Bet v 1 (2637) mutant lowers the affinity for specific serum IgE by 80 to 100-fold compared to Bet v 1.2801. The large difference in IgE-binding is further supported by a clear difference in inclination of the inhibition curve obtained with Bet v 1(2637) mutant protein compared to the inhibition curve for Bet v 1.2801. The different inclination provide evidence that the reduction in IgE-binding is due to a distinctly different epitope pattern of the mutant compared to Bet v 1.2801.

In addition to the inhibition assays with single modified allergens a 1:1 mix of Bet 1 (2628) and Bet v 1 (2637) having same molar concentration of Bet v 1 as each of the samples with Bet 1 (2628) or Bet v 1 (2637), respectively was tested and showed full (100%) capacity to inhibit IgE-binding to rBet v 1.2801. The capacity to fully inhibit IgE-binding is a clear indication that all reactive epitopes present on Bet v 1.2801 were present in the 1:1 allergen mix. Further support comes from the comparable inclination of the two inhibition curves for Bet v 1.2801 and the allergen mix. Reduced IgE-reactivity of the mixed allergen sample is demonstrated by the need of a four-fold higher concentration of the allergen mix, when compared to Bet v 1.2801, for obtaining 50% inhibition of IgE-binding.

### T cell proliferation assay using mutated recombinant Bet v 1 allergens.

The analysis was carried out as described in ref. 15. Bet v 1 (2628) and Bet v 1(2637) mutant protein were both able to induce proliferation in T cell lines from birch pollen allergic patients with stimulation indices similar to recombinant and naturally occurring. This suggests that both of Bet v 1 (2628) and Bet v 1 (2637) mutant protein can each initiate the cellular immune response necessary for antibody production.

### Histamine release assays with human basophil.

Histamine release from basophil leucocytes was performed as follows. Heparinized blood (20 ml) was drawn from each birch pollen patient, stored at room temperature, and used within 24 hours. Twenty-five microlitres of heparinized whole blood was applied to glass fibre coated microtitre wells (Reference Laboratory, Copenhagen, Denmark) and incubated with 25 microlitres of allergen or anti-IgE for 1 hour at 37°C. Thereafter the plates were rinsed and interfering substances were removed. Finally, histamine bound to the microfibres was measured spectrophotofluometrically.

### Histamine release properties of Bet v 1 (2628) and Bet v 1 (2637) mutant protein.

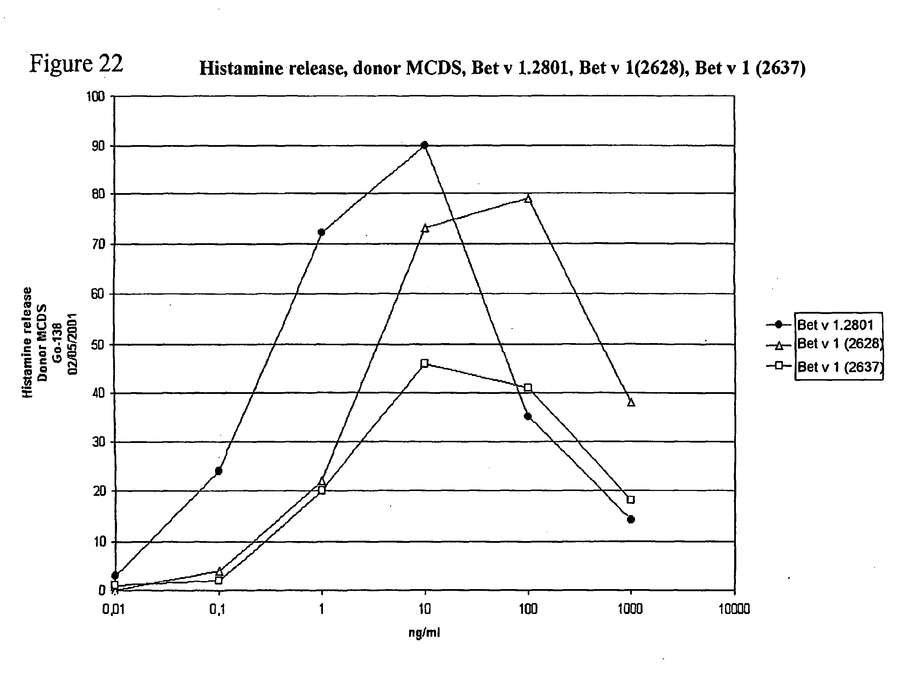

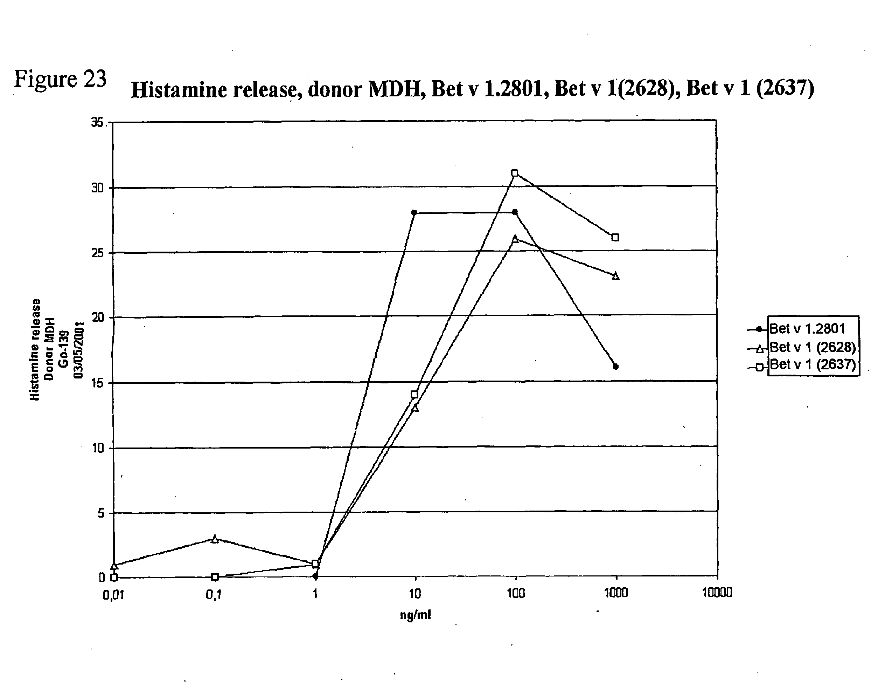

Histamine release data is shown in Figure 22 and Figure 23. The potency of Bet v 1 (2628) and Bet v 1 (2637) mutant protein to induce histamine release in human basophil from two birch pollen allergic patients has been tested. In both cases the release curve of the mutated allergens to induce histamine release is clearly shifted to the right compared to the release curve of Bet v 1.2801. The shift indicate that the potency of Bet v 1 (2628) and Bet v 1 (2637) is reduced 3 to 10-fold.

### Mutant Bet v 1 (2744) and mutant Bet v 1 (2753)

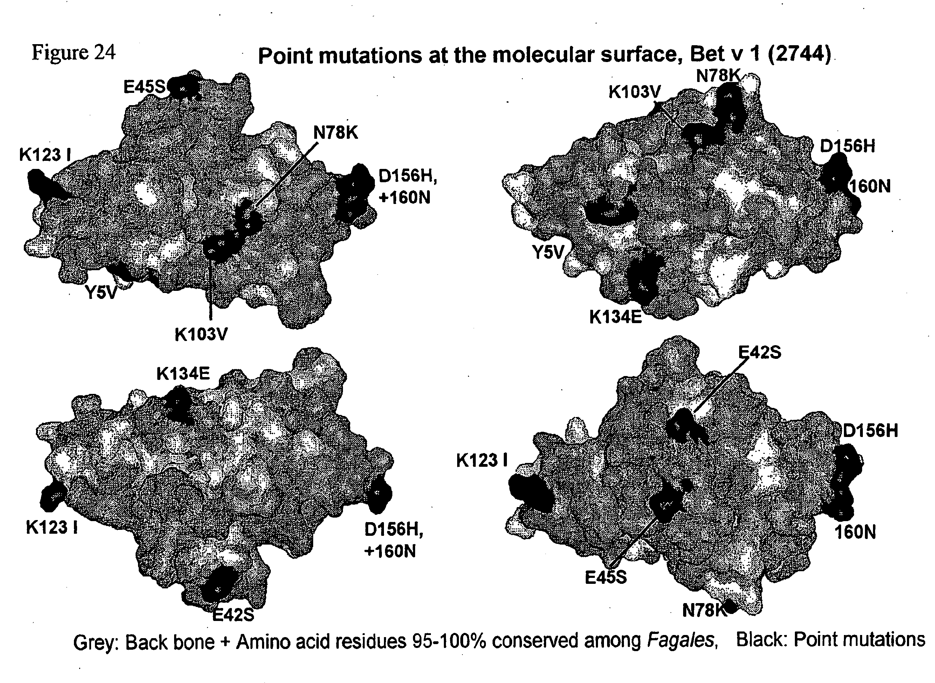

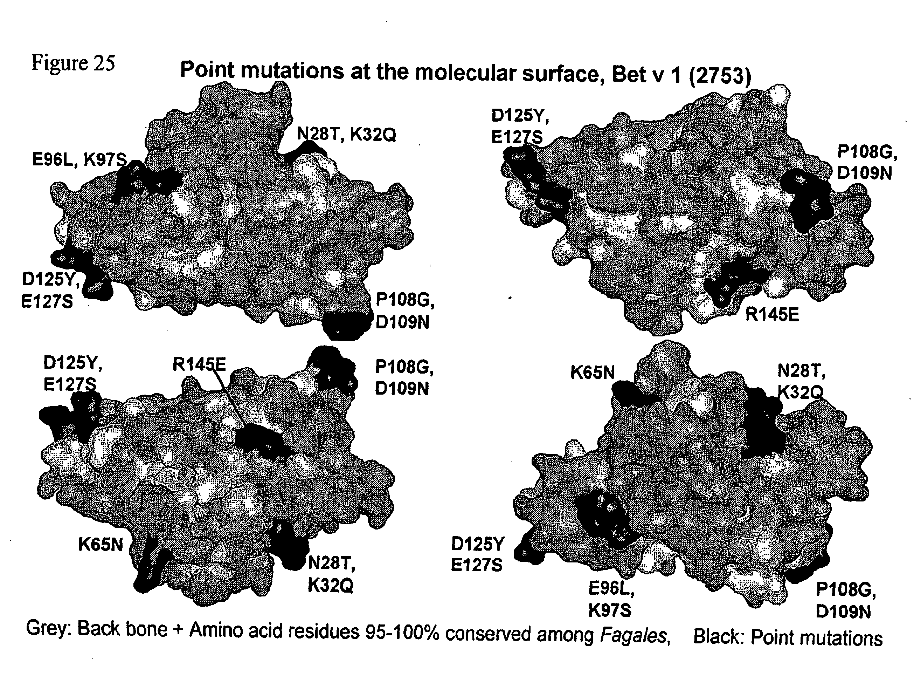

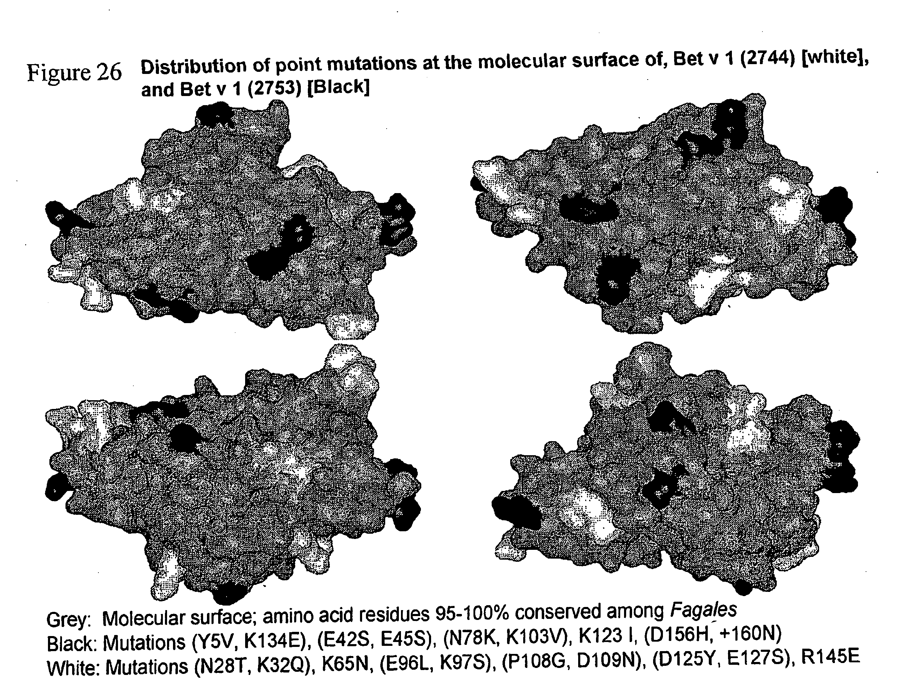

Bet v 1 (2744) and Bet v 1 (2753) was likewise constructed for use as a mixed allergen vaccine. In these mutated allergens point mutations were distributed in an all surface arranged fashion as shown in Figure 24 and Figure 25 and was again designed to affect different surface areas in the two molecules, respectively, as shown in Figure 26. However these modified allergens might individually be used as single allergen vaccines as well.

### Structural analysis of Bet v 1 (2744) mutant protein

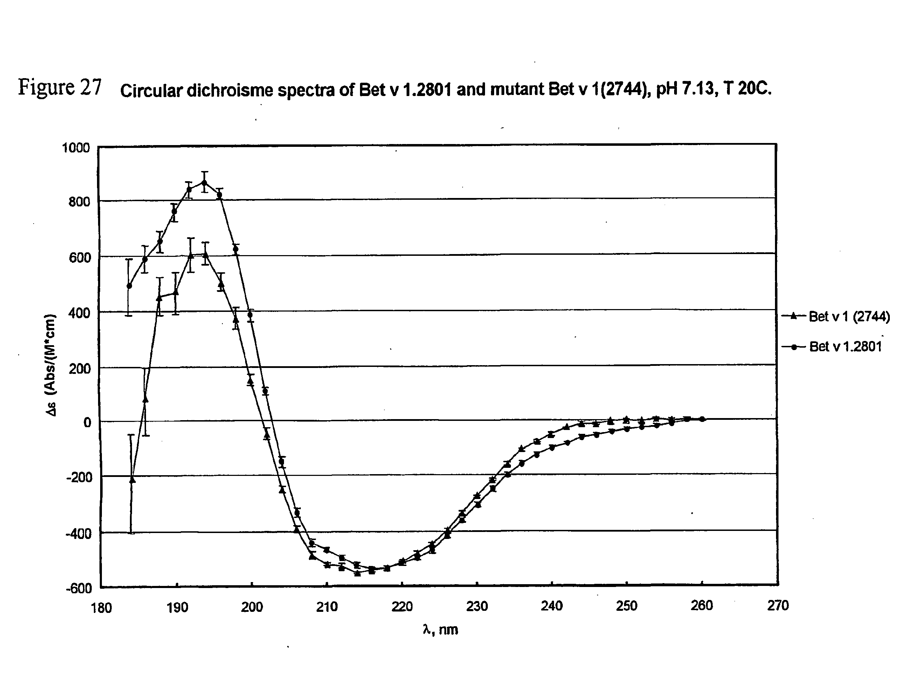

The structural integrity of the purified Bet v 1 (2744) mutant was analysed by circular dichroism (CD) spectroscopy. Figure 27 shows the CD spectra of recombinant Bet v 1.2801 (wildtype) and Bet v 1 (2744) mutant, recorded at close to equal concentrations. The overlap in peak amplitudes and positions in the CD spectra from the two recombinant proteins shows that the two preparations contain equal amounts of secondary structures strongly suggesting that the α-carbon backbone tertiary structure is not affected by the introduced amino acid substitutions.

### Histamine release properties of Bet v 1 (2744)

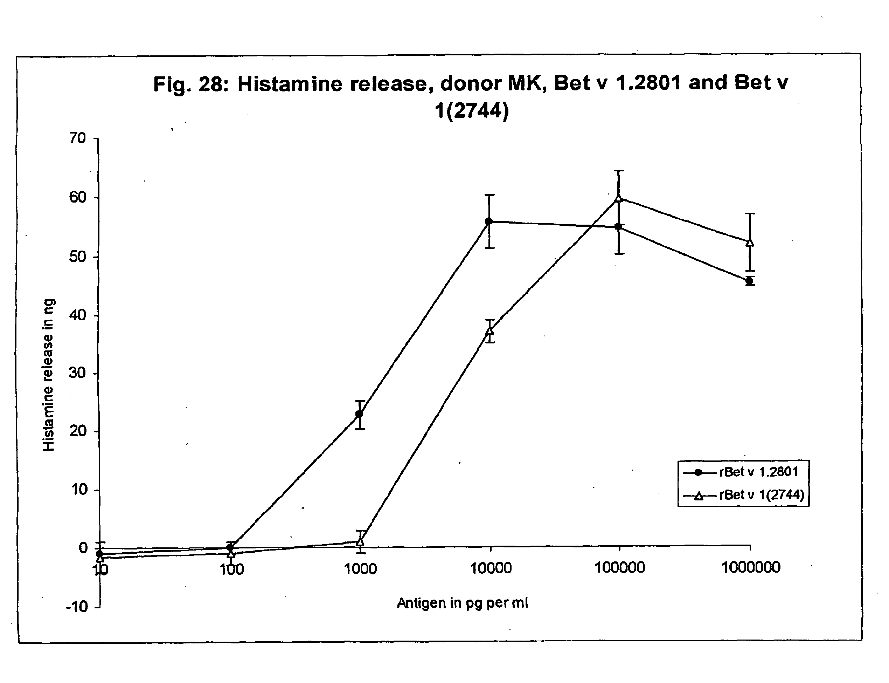

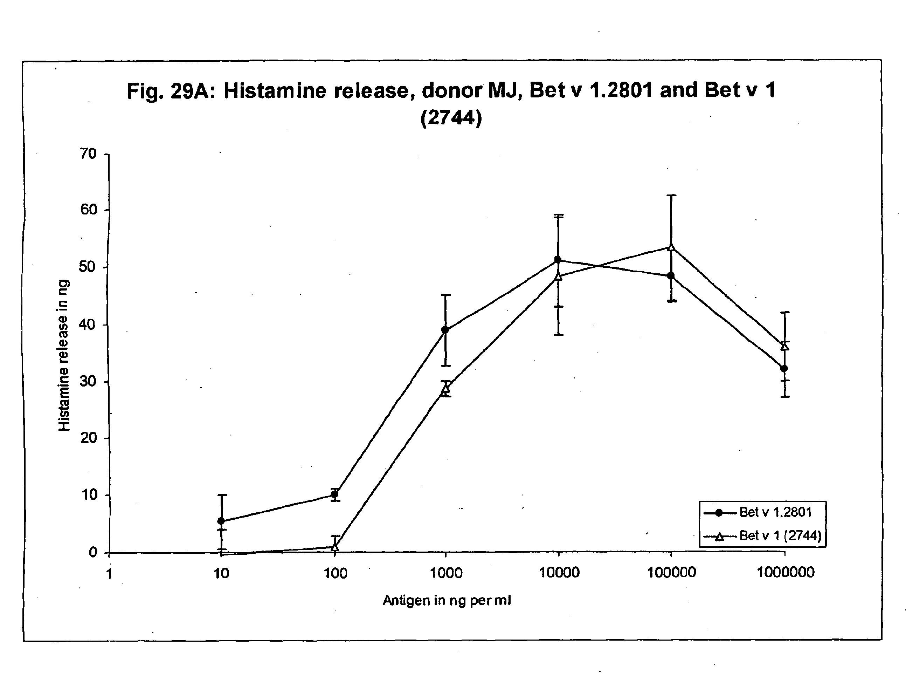

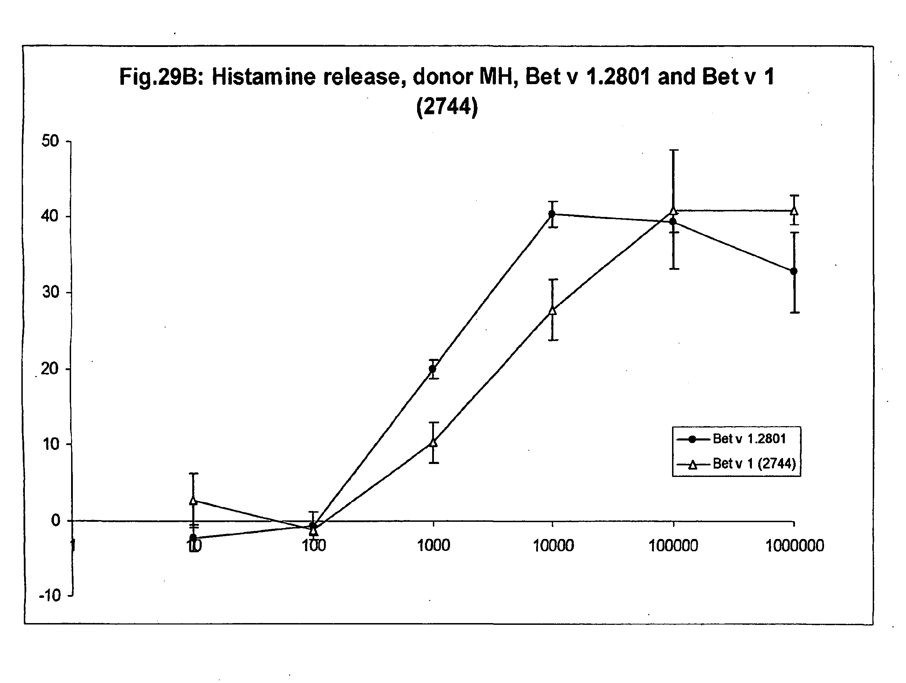

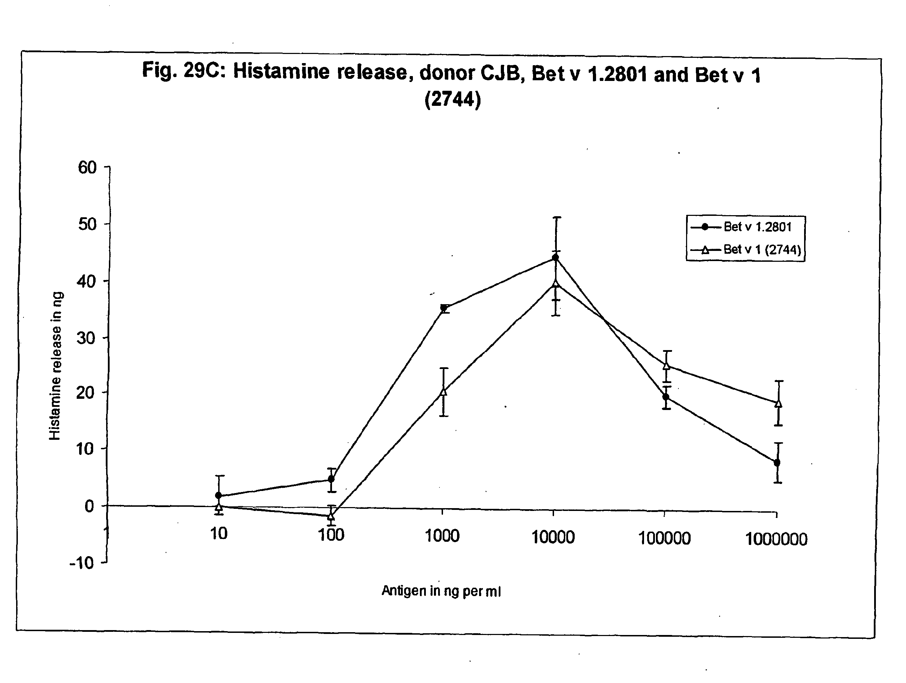

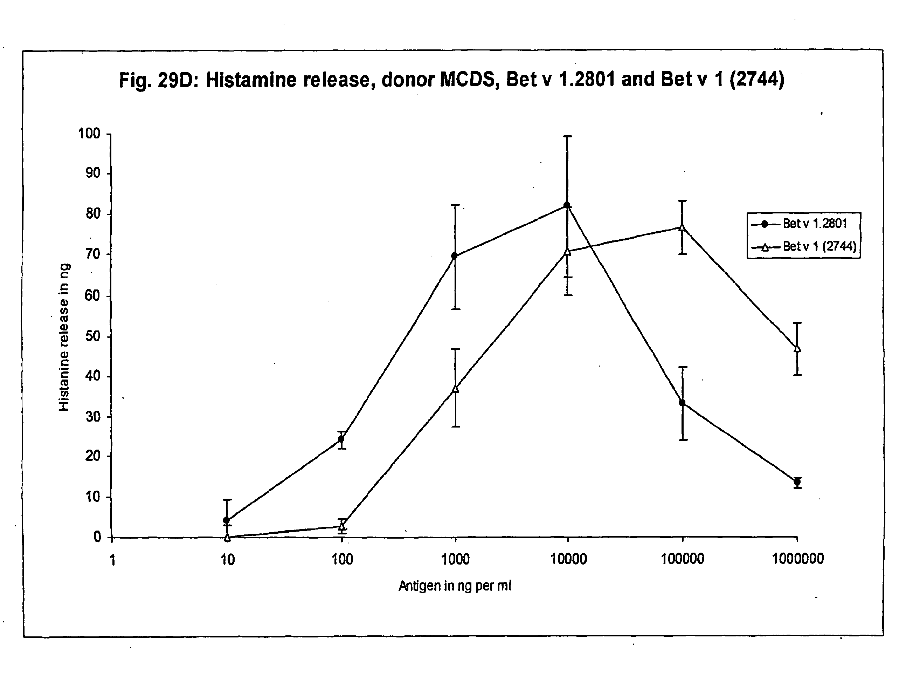

Histamine release data from five experiments with basophil leucocytes from five different birch pollen allergic patients is shown in Figure 28 and Figure 29A-D. The potency of Bet v 1 (2744) mutant protein to induce histamine release in human basophil has been tested. The release curves of the mutated allergens are clearly shifted to the right compared to the release curve of Bet v 1.2801 indicating that the potency of Bet v 1 (2744) to release histamine is reduced 3 to 5-fold.

### Mutant Bet v 1 (2733)

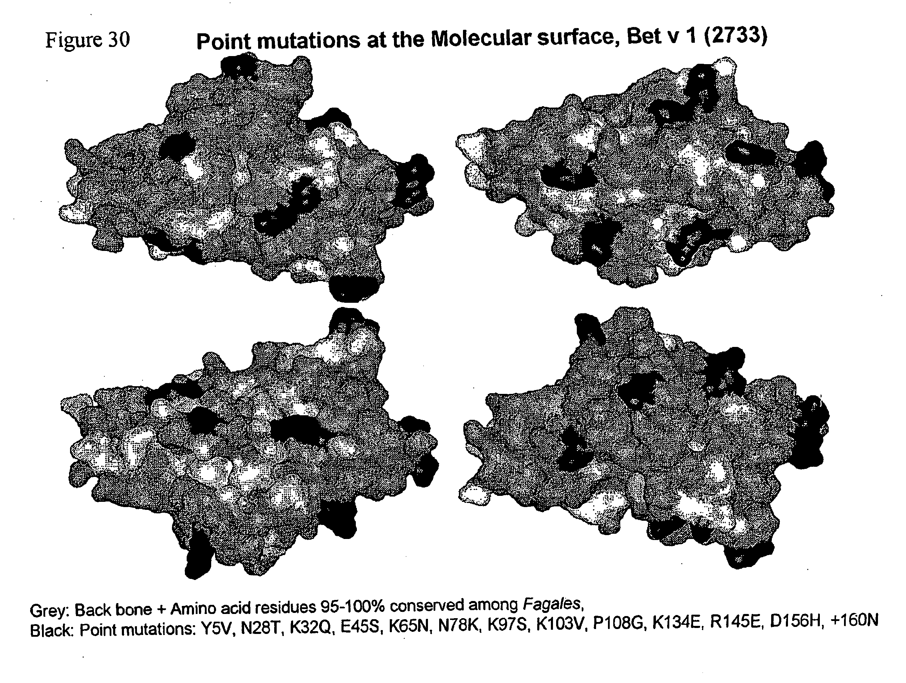

A Mutant Bet v 1 (2733) with nine primary mutations has been constructed and recombinantly expressed. The distribution of point mutations in Bet v 1 (2733) leave several surface areas constituting >400Å² unaltered. Figure 30 show introduced point mutations at the molecular surface of Bet v 1 (2733).

### EXAMPLE 5

This Example describes cloning of the gene encoding Der p 2 from *Dermatophagoides pteronyssinus* and construction of mutants with reduced IgE-binding affinity.

PCR amplified products from first strand cDNA synthesis of *Dermatophagoides pteronyssinus* total RNA was obtained from Dr. Wendy-Anne Smith and Dr. Wayne Thomas (TVW Telethon Institute for Child Health Research, 100 Roberts Rd, Subiaco, Western Australia 6008). During the amplification of the first strand cDNA library, Der p 2 had been selectively amplified using Der p 2 specific primers. PCR fragments were subsequently cloned into the Bam HI site of pUC19 (New England BioLabs). DNA sequencing of Der p 2 was performed using vector specific sense (5'-GGCGATTAAGTTGGGTAACGCCAGGG-3') and anti-sense (5'-GGAAACAGCTATGACCATGATTACGCC-3') primers.

A total of seven unique Der p 2 isoforms designated ALK-101, ALK-102, ALK-103, ALK-104, ALK-113, ALK-114, and ALK120 were identified. The clone entitled ALK-114 was chosen as starting point for generation of low-affinity IgE-mutants because of its high sequence identity with the Der p 2 NMR structure with the data base accession number 1A9V. Compared to ALK-114, the 6 other naturally occurring isoforms comprise the following substitutions:
ALK-101: M76V.
ALK-102: V40L, T47S.
ALK-103: M111L, D114N.
ALK-104: T47S, M111I, D114N.
ALK-113: T47S.
ALK-120: V40L, T47S, D114N.

### Insertion of Der p 2 into pGAPZα-A

The gene encoding Der p 2 (ALK-114) was subsequently inserted into the pGAPZα-A vector (Invitrogen) for secreted expression of Der p 2 in the yeast, *Pichia pastoris.* The gene was amplified using sense primer OB27 (5'- GGAATTCCTCGAGAAAAGAGATCAAGTCGATGTCAAAGATTGTGCC-3') and anti-sense primer OB28 (5'-CGTTCTAGACTATTAATCGCGGATTTTAGCATGAGTTGC-3') corresponding to the amino- and the carboxytermi of the Der p 2 polypeptide, respectively. The primers were extended in the 5'-end to accomodate the restriction sites Xho I and Xba I, respectively. The Xho I restriction site fuses the first codon of Der p2 in frame with the nucleic acid sequence encoding the KEX2 cleavage site (LYS-ARG) of pGAPZα-A. A single round of PCR amplification was performed in a 100 microliter (µl) volume: 0.1 mg of template ALK-114 DNA, 1 X Expand polymerase buffer (available from Boehringer Mannheim), 0.2 millimolar (mM) each of the four dNTPs, 0.3 micromolar (µM) each of the sense and anti-sense primers and 2.5 Units of Expand polymerase (available from Boehringer Mannheim). The DNA was amplified following 25 cycles of: 95°C for 15 seconds, 45°C for 30 seconds, 72°C for 1 minute, followed by 1 cycle of 72°C for 7 minutes. The resulting 475 base pair ALK-114 PCR fragment was purified using a QIAquick spin purification procedure (available from Qiagen). The purified DNA fragment was then digested with Xho I and Xba I, gel purified and ligated into similarly digested pGAPZα-A. The ligation reaction was trasformed into E.coli strain DH5α, resulting in plasmid, pCBo06.

The nucleotide sequence of Der p 2 was confirmed by DNA sequencing before and after cloning and following *in vitro* mutagenesis (see below).

### Der p 2 sequences

SEQ ID NO 1 corresponds to the nucleic acid sequence of Der p 2 (ALK-114):

| | |
|---|---|
| 1 | gatcaagtcgatgtcaaagattgtgccaatcatgaaatcaaaaaagttttggtaccagga |
| 61 | tgccatggttcagaaccatgtatcattcatcgtggtaaaccattccaattggaagccgtt |
| 121 | ttcgaagccaaccaaaacacaaaaaccgctaaaattgaaatcaaagcctcaatcgatggt |
| 181 | ttagaagttgatgttcccggtatcgatccaaatgcatgccattacatgaaatgcccattg |
| 241 | gttaaaggacaacaatatgatattaaatatacatggaatgttccgaaaattgcaccaaaa |
| 301 | tctgaaaatgttgtcgtcactgttaaagttatgggtgatgatggtgttttggcctgtgct |
| 361 | attgctactcatgctaaaatccgcgattaa |

SEQ ID NO. 2 corresponds to the deduced amino acid sequence of Der p 2 (ALK-114):

| | |
|---|---|
| 1 | dqvdvkdcanheikkvlvpgchgsepciihrgkpfqleavfeanqntktakieikasidg |
| 61 | levdvpgidpnachymkcplvkgqqydikytwnvpkiapksenvvvtvkvmgddgvlaca |
| 121 | iathakird |

### Insertion of pGAPZα-A-Der p 2 into P. pastoris

The vector, pCBo06 was linearized using Avr II restriction enzyme and transformed into competent *P. pastoris* strain, X-33, as described in the Invitrogen manual. Recombinant cells resistant to 100 micrograms per milliliter (µg/ml) of Zeocin were selected, and colony purifed on fresh YPD plates containing 100 µg/ml Zeocin.

### Expression and purification of recombinant Der p 2

A 250 ml of YPD medium (1% yeast extract, 2% peptone, 2% glucose) containing 100 µg/ml Zeocin was inoculated with an overnight culture of recombinant yeast cells expressing Der p 2. The culture was grown at 30°C for 72 hours to obtain optimal Der p 2 expression. Cells were harvested by centrifugation and the resulting culture supernatant was saturated with 50% ammonium sulfate. Following centrifugation at 3000x g for 30 minutes, the supernatant was saturated with 80% ammonium sulfate. Following centrifugation, the pellet was resuspended in 150 millimolar (mM) NH₄HCO₃ and fractionated on a Superdex 75 gel filtration column, equilibrated with the same buffer. Der p 2 was eluted as a major peak corresponding to its expected molecular weight. The elution of Der p 2 was monitered both by SDS page electrophoresis, followed by silver staining and by immunoblot analysis using Der p 2 specific polyclonal antibodies.

### Selection of amino acid residues for site-directed mutagenesis

Selection of amino acid residues for mutagenesis was based on identification of residues that are highly solvent exposed and highly conserved among allergens from House Dust Mites (Der p 2/f 2 and Eur m 2) and storage mites (Tyr p 2, Lep d 2, Gly d 2). Highly solvent exposed amino acid residues were identified visually by analysis of the molecular surface of the Der p 2 NMR structure (#1.9, 1A9V.pdb). Twelve amino acid residues were selected for mutagenesis: K6A, N10S, K15E, S24N, H30N, K48A, E62S, H74N, K77N, K82N, K100N and R128Q.

### Site-directed mutagenesis

Construction of recombinant mutant allergens with single primary mutations and multiple combinations thereof, are described in the following.

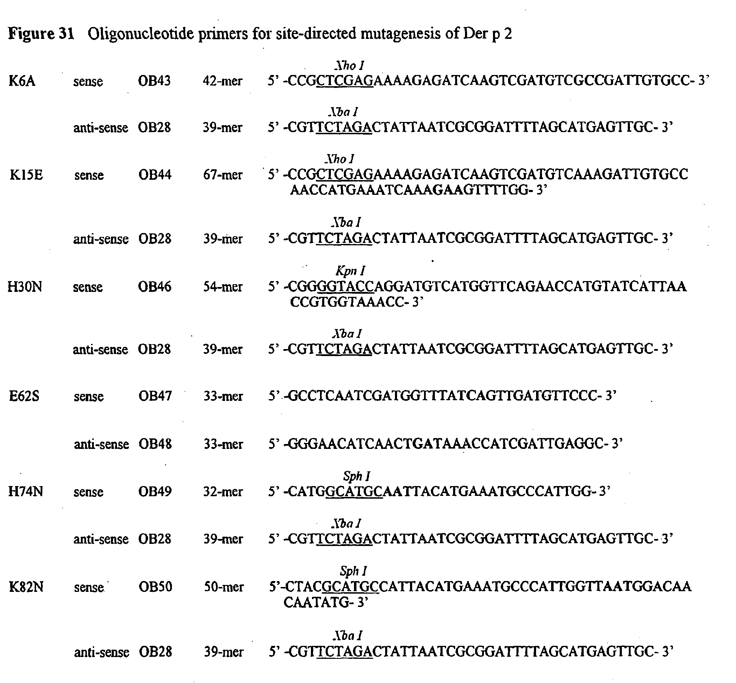

Expression plasmids encoding Der p 2 mutants were produced using pCBo06 as DNA template. PCR reactions were performed using sense and anti-sense primers incorporating the specified mutations. Primer pairs used in the PCR reactions to generate the specified mutations are listed in Figure 31. The mutations are designated in bold and the restriction sites used in the subsequent cloning step are underlined in the figure. For the construction of mutants K6A, K15E, H30N, H74N and K82N, PCR reactions were performed essentially as described in the section "Cloning of Der p 2 into pGAPZα-A". The purified PCR fragments were digested with the designated restriction enzyme sites (see Figure 31), gel purified, ligated into similarly digested pCBo06 and transformed into *E.coli* DH5α.

The mutation E62S was generated using an alternative PCR mutagenesis methodology described for the generation of Bet v 1 mutants in Example 1. Two mutation specific oligonucleotide primers were synthesized covering the specified mutations (OB47 and OB48, listed in Figure 31). Two additional primers used for the secondary amplification step were OB27 and OB28 as described in the section: "Insertion of Der p 2 into pGAPZα-A".

The mutant allergens produced are characterised using the same methods as described in example 4, e.g. circular dichroism (CD) spectroscopy, crystallisation, measurements of IgE binding properties, histamin-release, T-cell proliferation stimulation capacity, etc.

### EXAMPLE 6

### Mutated recombinant mite allergens (Der p 2) with improved safety for specific allergy vaccination

In this example the application of the concept of the current invention on house dust mite allergens is exemplified by one allergen, Der p 2. Manipulation of other house dust mite allergens may be performed by equivalent procedures.

### Design of mutated recombinant Der p 2 molecules.

SEQ ID NO. 3 shows the nucleotide and deduced amino acid sequence of Der p 2-ALK-G clone, which is a wild type isoform.

SEQ ID NO. 3: Nucleotide and deduced amino acid sequence of Der p 2-ALK-G.

GAT CAA GTC GAT GTC AAA GAT TGT GCC AAT CAT GAA ATC AAA AAA 45
D Q V D V K D C A N H E I K K 15

GTT TTG GTA CCA GGA TGC CAT GGT TCA GAA CCA TGT ATC ATT CAT 90
V L V P G C H G S E P C I I H 30

CGT GGT AAA CCA TTC CAA TTG GAA GCT TTA TTC GAA GCC AAT CAA 135
R G K P F Q L E A L F E A N Q 45

AAC TCA AAA ACA GCT AAA ATT GAA ATC AAA GCT TCA ATC GAT GGT 180
N S K T A K I E I K A S I D G 60

TTA GAA GTT GAT GTT CCC GGT ATC GAT CCA AAT GCA TGC CAT TAT 225
L E V D V P G I D P N A C H Y 75

ATG AAA TGT CCA TTG GTT AAA GGA CAA CAA TAT GAT ATT AAA TAT 270
M K C P L V K G Q Q Y D I K Y 90

ACA TGG AAT GTT CCA AAA ATT GCA CCA AAA TCT GAA AAT GTT GTC 315
T W N V P K I A P K S E N V V 105

GTC ACT GTT AAA GTT TTG GGT GAT AAT GGT GTT TTG GCC TGT GCT 360
V T V K V L G D N G V L A C A 120

ATT GCT ACT CAT GCT AAA ATC CGC GAT 387
I A T H A K I R D 129

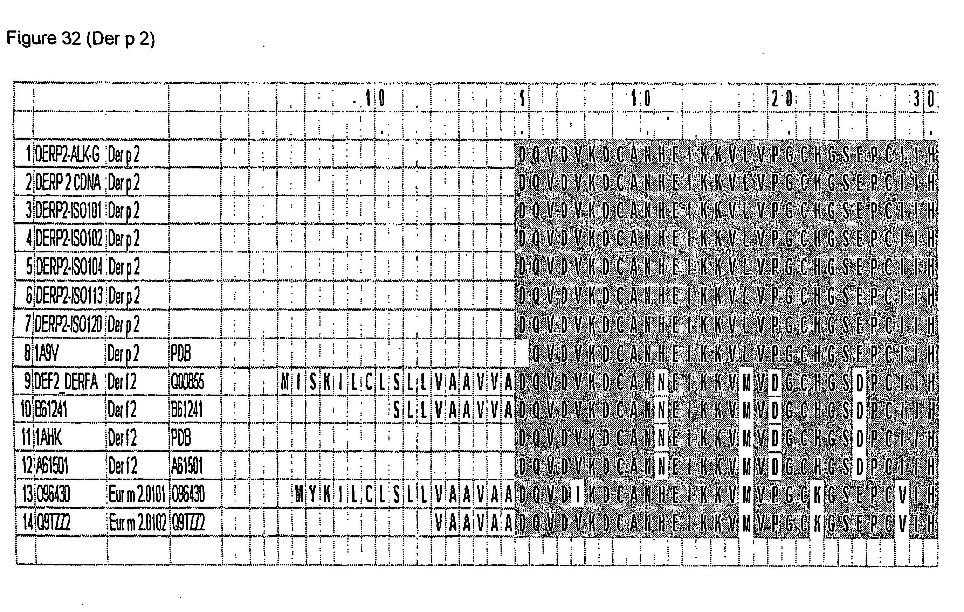

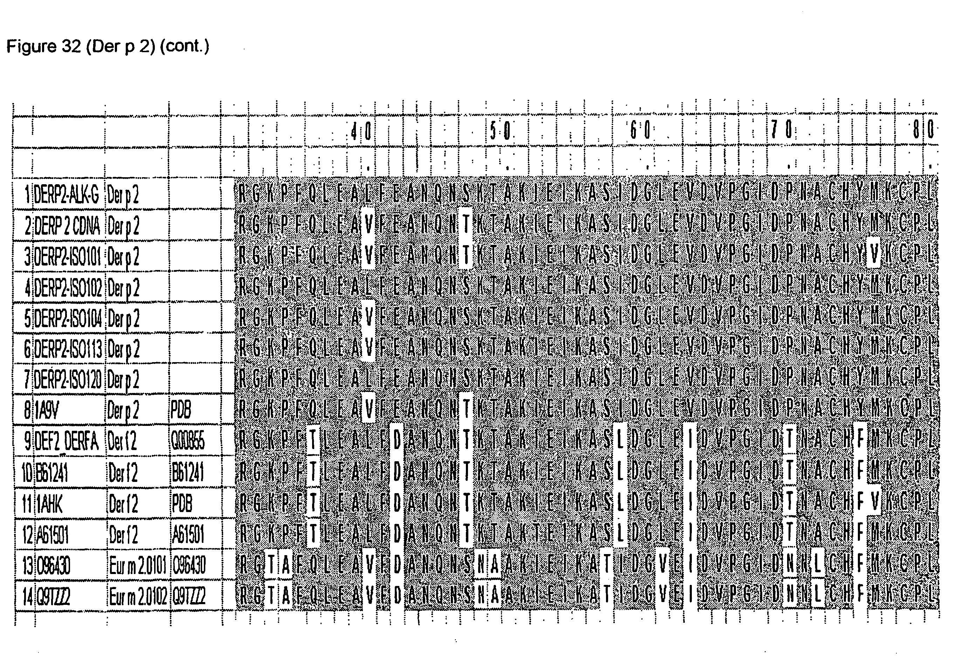

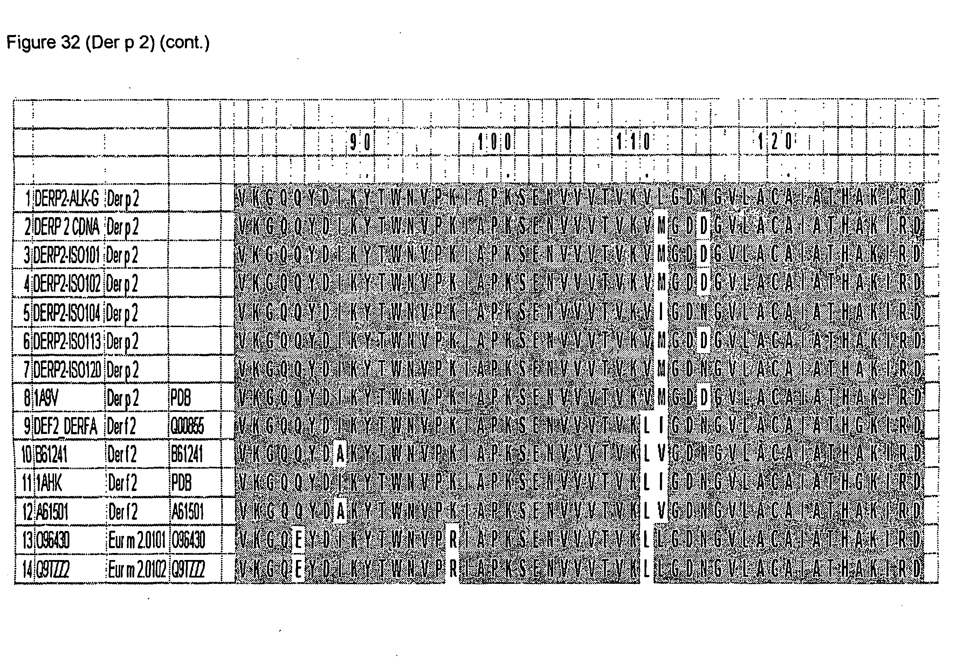

Fig. 32 shows a sequence alignment performed at the ExPaSy Molecular Biology Server (http://www.expasy.ch/) using the ClustalW algorithm on a BLAST search using the Der p 2-ALK-G amino acid sequence shown in SEQ ID NO. 3 as input sequence. The alignment includes sequences from house dust mite species, i.e. Der p 2, Der f 2 and Eur m 2. In Fig. 32 amino acid residues identical to amino acids in the same position in the Der p 2-ALK-G protein sequence are highlighted using black letters on grey background. Non-identical amino acids are printed in black on a white background.

### Surface structure images

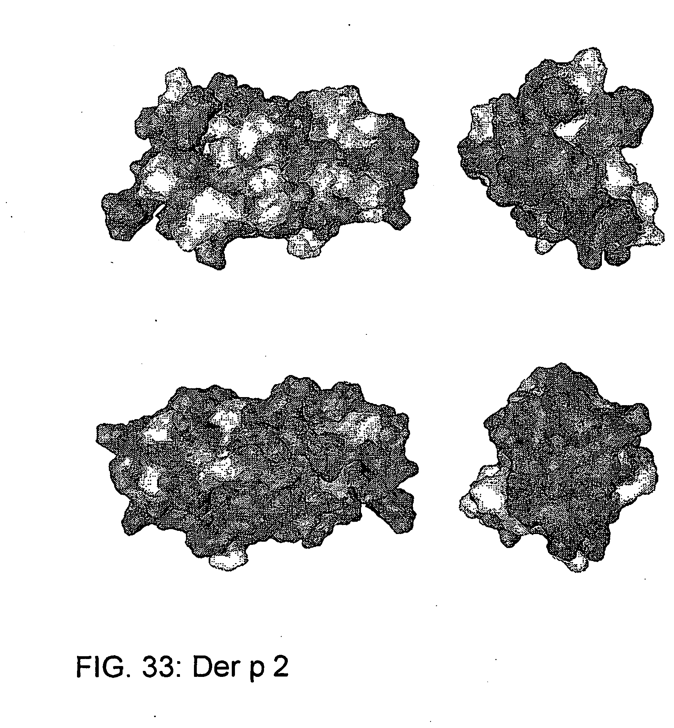

Amino acid sequences representing the house dust mite group 2 allergens have a similarity greater than 85 % and some of the molecular surface is conserved (grey-coloured zones), see Fig. 33.

Fig. 33 shows surface contours viewed from 4 different angles when superimposing the Der p 2-ALK-G protein sequence on to the published PDB:1A9V NMR structure, structure number 1 of 10 contained in the PDB file.

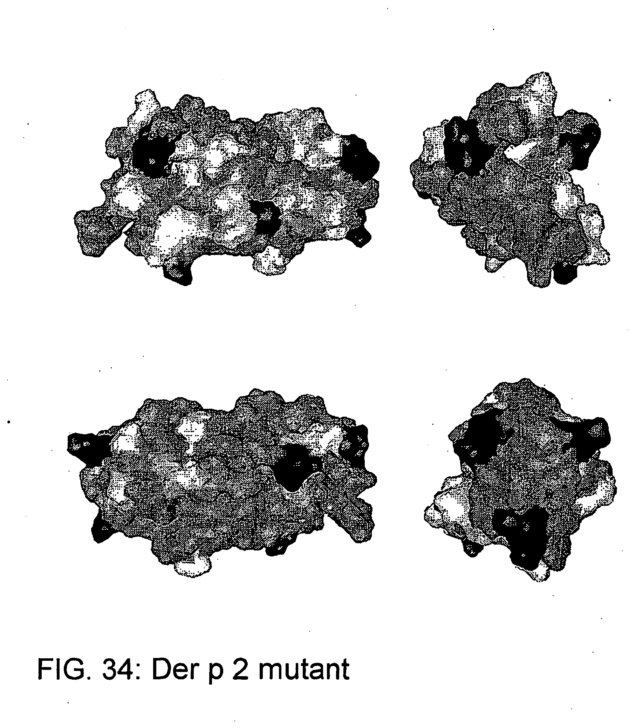

Conserved and highly solvent exposed amino acid spatially distributed over the entire surface within distances in the range of 25-30 Å are selected for mutation. In the sections below the following information is given: A list of amino acids considered to be appropriate for mutation (A), a list of the mutants designed (B) and the DNA sequences representing the mutants designed (C). Fig. 34 shows surface contours of mutant number 1 as an example. Grey colour indicates conserved amino acid residues. Black colour indicates amino acid residues selected for mutation.
A. List of amino acids selected for mutation
   K15, S24, H30, R31, K48, E62, H74, K77, K82, K100, R128
B. List of mutants designed
   Mutant 1:
      K15E, S24N, H30G, K48A, E62S, K77N, K82N, K100N
   Mutant 2:
      K15E, S24N, H30G, K48A, E62S, K77N, K82N, R128Q
   Mutant 3:
      K15E, S24N, H30G, K48A, K77N, K82N, K100N, R128Q
   Mutant 4:
      K15E, S24N, H30G, E62S, K77N, K82N, K100N, R128Q
   Mutant 5:
      K15E, H30G, K48A, E62S, K77N, K82N, K100N, R128Q
   Mutant 6:
      S24N, H30G, K48A, E62S, K77N, K82N, K100N, R128Q
   Mutant 7:
      K15E, S24N, R31S, K48A, E62S, H74N, K82N, K100N
   Mutant 8:
      K15E, S24N, R31S, K48A, E62S, H74N, K82N, R128Q
   Mutant 9:
      K15E, S24N, R31S, K48A, H74N, K82N, K100N, R128Q
   Mutant 10:
      K15E, S24N, R31S, E62S, H74N, K82N, K100N, R128Q
   Mutant 11:
      K15E, R31S, K48A, E62S, H74N, K82N, K100N, R128Q
   Mutant 12:
      S24N, R31S, K48A, E62S, H74N, K82N, K100N, R128Q
C. Nucleotide sequence of mutants
   Mutant 1:
      K15E, S24N, H30G, K48A, E62S, K77N, K82N, K100N 
GatcaagtcgatgtcaaagattgtgccaatcatgaaatcaaaGAAgttttggtacca
ggatgccatggtAACgaaccatgtatcattGGCcgtggtaaaccattccaattggaa
gctttattcgaagccaatcaaaactcaGCGacagctaaaattgaaatcaaagcttca
atcgatggtttaAGCgttgatgttcccggtatcgatccaaatgcatgccattatatg
AACtgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaatccgcgat
   Mutant 2:
      K15E, S24N, H30G, K48A, E62S, K77N, K82N, R128Q 
GatcaagtcgatgtcaaagattgtgccaatcatgaaatcaaaGAAgttttggtacca
ggatgccatggtAACgaaccatgtatcattGGCcgtggtaaaccattccaattggaa
gctttattcgaagccaatcaaaactcaGCGacagctaaaattgaaatcaaagcttca
atcgatggtttaAGCgttgatgttcccggtatcgatccaaatgcatgccattatatg
AACtgtccattggttAACggacaacaatatgatattaaatatacatggaatgttcca
aaaattgcaccaaaatctgaaaatgttgtcgtcactgttaaagttttgggtgataat
ggtgttttggcctgtgctattgctactcatgctaaaatcCAGgat
   Mutant 3:
      K15E, S24N, H30G, K48A, K77N, K82N, K100N, R128Q 
GatcaagtcgatgtcaaagattgtgccaatcatgaaatcaaaGAAgttttggtacca
ggatgccatggtAACgaaccatgtatcattGGCcgtggtaaaccattccaattggaa
gctttattcgaagccaatcaaaactcaGCGacagctaaaattgaaatcaaagcttca
atcgatggtttagaagttgatgttcccggtatcgatccaaatgcatgccattatatg
AACtgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaaatcCAGgat
   Mutant 4:
      K15E, S24N, H30G, E62S, K77N, K82N, K100N, R128Q 
GatcaagtcgatgtcaaagattgtgccaatcatgaaatcaaaGAAgttttggtacca
ggatgccatggtAACgaaccatgtatcattGGCcgtggtaaaccattccaattggaa
gctttattcgaagccaatcaaaactcaaaaacagctaaaattgaaatcaaagcttca
atcgatggtttaAGCgttgatgttcccggtatcgatccaaatgcatgccattatatg
AACtgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaaatcCAGgat
   Mutant 5:
      K15E, H30G, K48A, E62S, K77N, K82N, K100N, R128Q 
GatcaagtcgatgtcaaagattgtgccaatcatgaaatcaaaGAAgttttggtacca
ggatgccatggttcagaaccatgtatcattGGCcgtggtaaaccattccaattggaa
gctttattcgaagccaatcaaaactcaGCGacagctaaaattgaaatcaaagcttca
atcgatggtttaAGCgttgatgttcccggtatcgatccaaatgcatgccattatatg
AACtgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaatcCAGgat
   Mutant 6:
      S24N, H30G, K48A, E62S, K77N, K82N, K100N, R128Q 
Gatcaagtcgatgtcaaagattgtgccaatcatgaatcaaaaagttttggtacca
ggatgccatggtAACgaaccatgtatcattGGCcgtggtaaaccattccaattggaa
gctttattcgaagccaatcaaaactcaGCGacagctaaaattgaaatcaaagcttca
atcgatggtttaAGCgttgatgttcccggtatcgatccaaatgcatgccattatatg
AACtgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaaatcCAGgat
   Mutant 7:
      K15E, S24N, R31S, K48A, E62S, H74N, K82N, K100N 
GatcaagtcgatgtcaaagattgtgccaatcatgaaatcaaaGAAgttttggtacca
ggatgccatggtAACgaaccatgtatcattcatAGCggtaaaccattccaattggaa
gctttattcgaagccaatcaaaactcaGCGacagctaaaattgaaatcaaagcttca
atcgatggtttaAGCgttgatgttcccggtatcgatccaaatgcatgcAACtatatg
aaatgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaatccgcgat
   Mutant 8:
      K15E, S24N, R31S, K48A, E62S, H74N, K82N, R128Q 
GatcaagtcgatgtcaaagattgtgccaatcatgaaatcaaaGAAgttttggtacca
ggatgccatggtAACgaaccatgtatcattcatAGCggtaaaccattccaattggaa
gctttattcgaagccaatcaaaactcaGCGacagctaaaattgaaatcaaagcttca
atcgatggtttaAGCgttgatgttcccggtatcgatccaaatgcatgcAACtatatg
aaatgtccattggttAACggacaacaatatgatattaaatatacatggaatgttcca
aaaattgcaccaaaatctgaaaatgttgtcgtcactgttaaagttttgggtgataat
ggtgttttggcctgtgctattgctactcatgctaaatcCAGgat
   Mutant 9:
      K15E, S24N, R31S, K48A, H74N, K82N, K100N, R128Q 
GatcaagtcgatgtcaaagattgtgccaatcatgaaatcaaaGAAgttttggtacca
ggatgccatggtAACgaaccatgtatcattcatAGCggtaaaccattccaattggaa
gctttattcgaagccaatcaaaactcaGCGacagctaaaattgaaatcaaagcttca
atcgatggtttagaagttgatgttcccggtatcgatccaaatgcatgcAACtatatg
aaatgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaaatcCAGgat
   Mutant 10:
      K15E, S24N, R31S, E62S, H74N, K82N, K100N, R128Q 
GatcaagtcgatgtcaaagattgtgccaatcatgaaatcaaaGAAgttttggtacca
ggatgccatggtAACgaaccatgtatcattcatAGCggtaaaccattccaattggaa
gctttattcgaagccaatcaaaactcaGCGacagctaaaattgaaatcaaagcttca
atcgatggtttagaagttgatgttcccggtatcgatccaaatgcatgcAACtatatg
aaatgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaaatcCAGgat
   Mutant 11:
      K15E, R31S, K48A, E62S, H74N, K82N, K100N, R128Q 
GatcaagtcgatgtcaaagattgtgccaatcatgaaatcaaaGAAgttttggtacca
ggatgccatggttcagaaccatgtatcattcatAGCggtaaaccattccaattggaa
gctttattcgaagccaatcaaaactcaGCGacagctaaaattgaaatcaaagcttca
atcgatggtttaAGCgttgatgttcccggtatcgatccaaatgcatgcAACtatatg
aaatgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaaatcCAGgat
   Mutant 12:
      S24N, R31S, K48A, E62S, H74N, K82N, K100N, R128Q

Gatcaagtcgatgtcaaagattgtgccaatcatgaaatcaaaaagttttggtacca
ggatgccatggtAACgaaccatgtatcattcatAGCggtaaaccattccaattggaa
gctttattcgaagccaatcaaaactcaGCGacagctaaaattgaaatcaaagcttca
atcgatggtttaAGCgttgatgttcccggtatcgatccaaatgcatgcAACtatatg
aaatgtccattggttAACggacaacaatatgatattaaatatacatggaatgttcca
aaaattgcaccaAACtctgaaaatgttgtcgtcactgttaaagttttgggtgataat
ggtgttttggcctgtgctattgctactcatgctaaaatcCAGgat

### EXAMPLE 7

### Mutated recombinant mite allergens (Der p 1) with improved safety for specific allergy vaccination

In this example the application of the concept of the current invention on house dust mite allergens is exemplified by one allergen, Der p 1. Manipulation of other house dust mite allergens may be performed by equivalent procedures.

### Design of mutated recombinant Der p 1 molecules.

SEQ ID NO. 4 shows the nucleotide and deduced amino acid sequence of Der p 1-ALK clone, which is a wild-type isoform.

SEQ ID NO. 4: Nucleotide and deduced amino acid sequence of Der p 1-ALK

ACT AAC GCC TGC AGT ATC AAT GGA AAT GCT CCA GCT GAA ATC GAT 45
T N A C S I N G N A P A E I D 15

TTG CGA CAA ATG CGA ACT GTC ACT CCC ATT CGT ATG CAA GGA GGC 90
L R Q M R T V T P I R M Q G G 30

TGT GGT TCA TGT TGG GCT TTC TCT GGT GTT GCC GCA ACT GAA TCA 135
C G S C W A F S G V A A T E S 45

GCT TAT TTG GCT TAC CGT AAT CAA TCA TTG GAT CTT GCT GAA CAA 180
A Y L A Y R N Q S L D L A E Q 60

GAA TTA GTC GAT TGT GCT TCC CAA CAC GGT TGT CAT GGT GAT ACC 225
E L V D C A S Q H G C H G D T 75

ATT CCA CGT GGT ATT GAA TAC ATC CAA CAT AAT GGT GTC GTC CAA 270
I P R G I E Y I Q H N G V V Q 90

GAA AGC TAC TAT CGA TAC GTT GCA CGA GAA CAA TCA TGC CGA CGA 315
E S Y Y R Y V A R E Q S C R R 105

CCA AAT GCA CAA CGT TTC GGT ATC TCA AAC TAT TGC CAA ATT TAC 360
P N A Q R F G I S N Y C Q I Y 120

CCA CCA AAT GTA AAC AAA ATT CGT GAA GCT TTG GCT CAA ACC CAC 405
P P N V N K I R E A L A Q T H 135

AGC GCT ATT GCC GTC ATT ATT GGC ATC AAA GAT TTA GAC GCA TTC 450
S A I A V I I G I K D L D A F 150

CGT CAT TAT GAT GGC CGA ACA ATC ATT CAA CGC GAT AAT GGT TAC 495
R H Y D G R T I I Q R D N G Y 165

CAA CCA AAC TAT CAC GCT GTC AAC ATT GTT GGT TAC AGT AAC GCA 540

Q P N Y H A V N I V G Y S N A 180

CAA GGT GTC GAT TAT TGG ATC GTA CGA AAC AGT TGG GAT ACC AAT 585
Q G V D Y W I V R N S W D T N 195

TGG GGT GAT AAT GGT TAC GGT TAT TTT GCT GCC AAC ATC GAT TTG 630
W G D N G Y G Y F A A N I D L 210

ATG ATG ATT GAA GAA TAT CCA TAT GTT GTC ATT CTC 666
M M I E E Y P Y V V I L 222

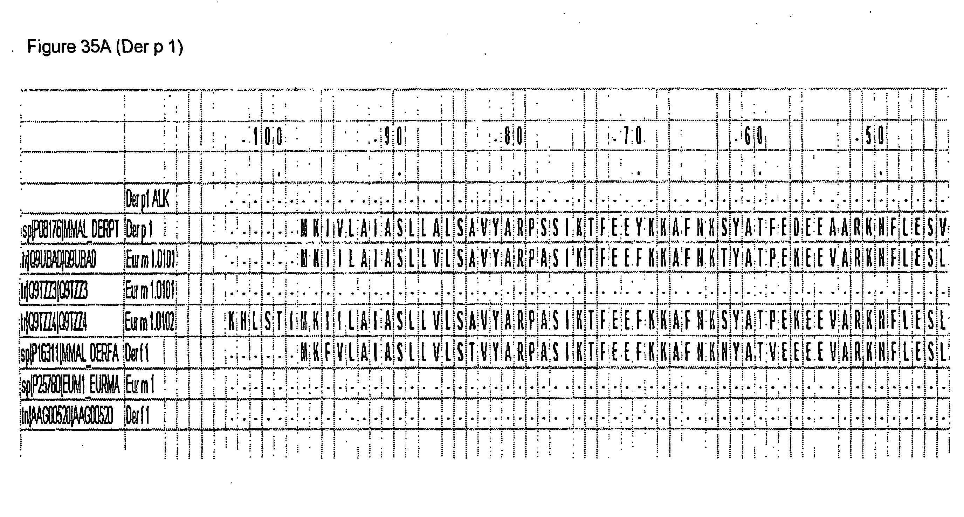

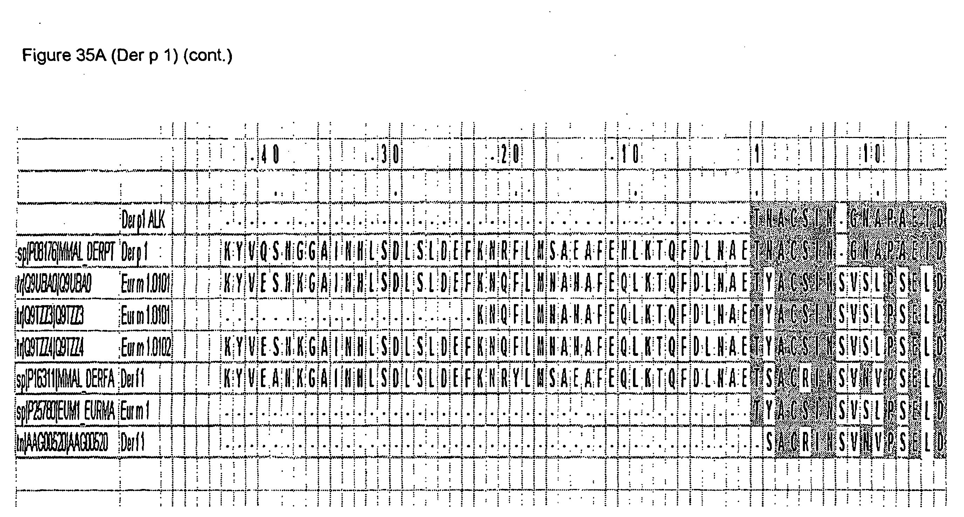

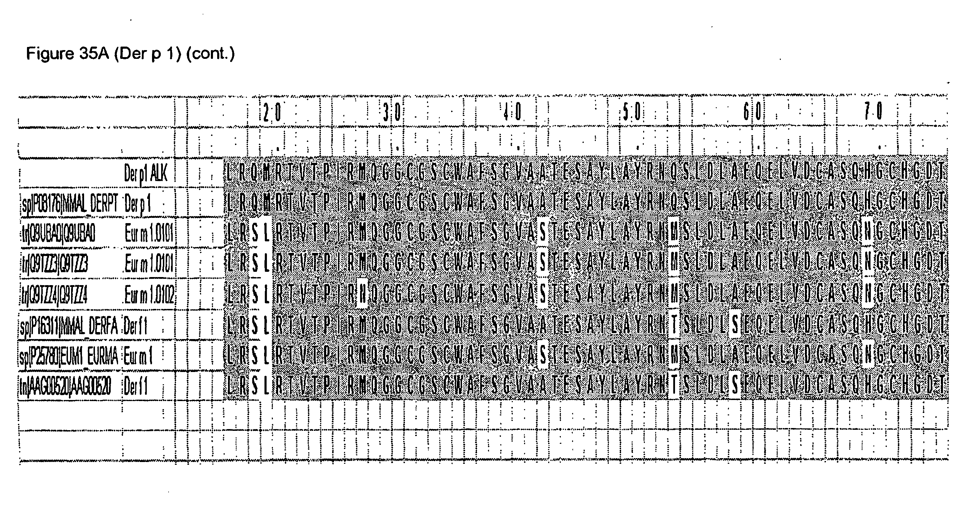

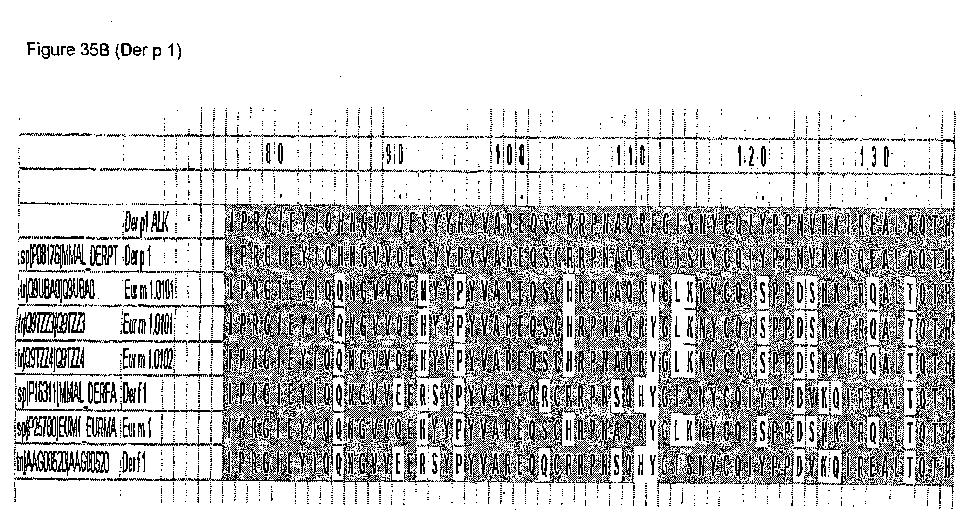

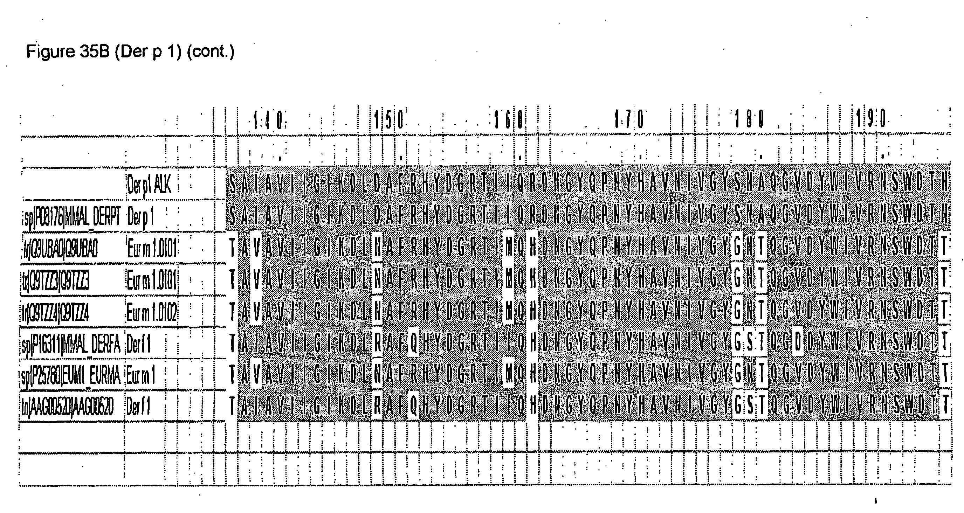

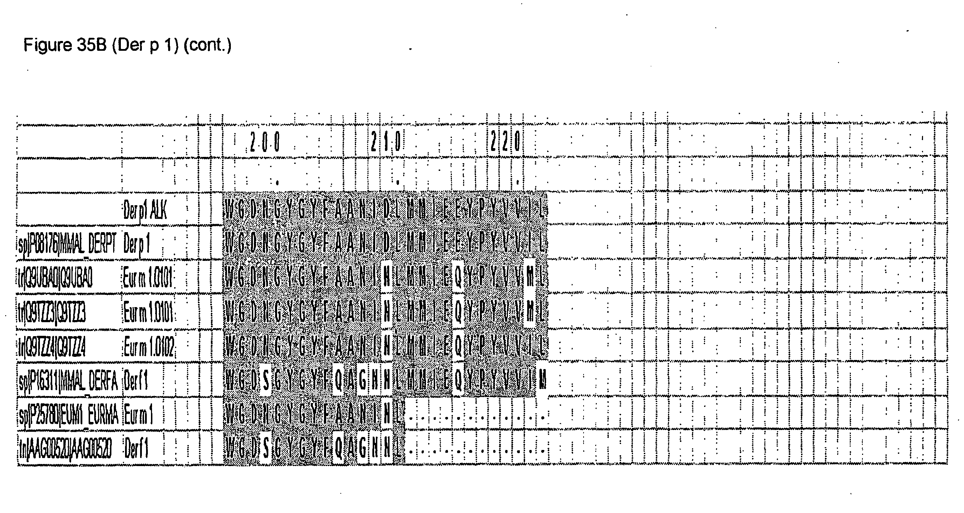

Fig. 35 shows a sequence alignment performed at the ExPaSy Molecular Biology Server (http://www.expasy.ch/) using the ClustalW algorithm on a BLAST search using the Der p 1-ALK amino acid sequence shown in SEQ ID NO. 4 as input sequence. The alignment includes sequences from house dust mite species, i.e. Der p 1, Der f 1 and Eur m 1. In Fig. 35 amino acid residues identical to amino acids in the same position in the Der p 1-ALK protein sequence are highlighted using black letters on grey background. Non-identical amino acids are printed in black on a white background.

### Surface structure images

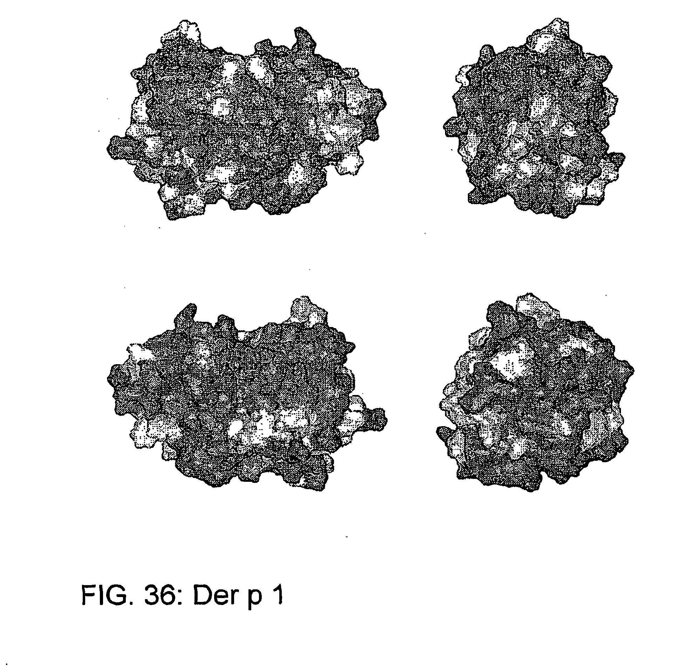

Amino acid sequences representing the house dust mite group 1 allergens are similar to a certain degree and some of the molecular surface is conserved (grey-coloured zones), see Fig. 36. Fig. 36 shows surface contours viewed from 4 different angles when superimposing the Der p 1-ALK protein sequence on to a Der p 1 molecular structure model.

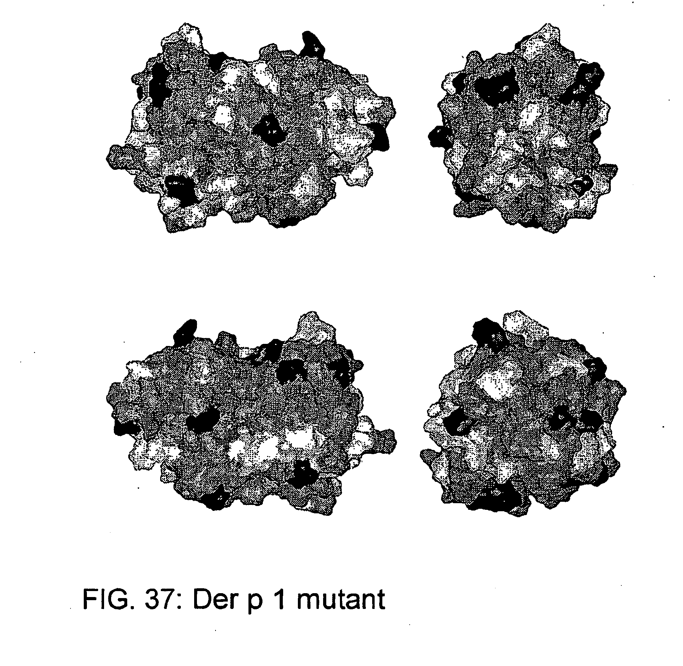

Conserved and highly solvent exposed amino acid spatially distributed over the entire surface within distances in the range of 25-30 Å are selected for mutation. In the sections below the following information is given: A list of amino acids considered to be appropriate for mutation (A), a list of the mutants designed (B) and the DNA sequences representing the mutants designed (C). Fig. 37 shows surface contours of mutant number 11 as an example.

Grey colour indicates conserved amino acid residues. Black colour indicates amino acid residues selected for mutation.
A. List of amino acids selected for mutation
   E13, P24, R20, Y50, S67, R78, R99, Q109, R128, R156, R161, P167, W192
B. List of mutants designed
   Mutant 1:
      P24T, Y50V, R78E, R99Q, R156Q, R161E, P167T
   Mutant 2:
      P24T, Y50V, R78Q, R99E, R156E, R161Q, P167T
   Mutant 3:
      R20E, Y50V, R78Q, R99Q, R156E, R161E, P167T
   Mutant 4:
      R20Q, Y50V, R78E, R99E, R156Q, R161Q, P167T
   Mutant 5:
      P24T, Y50V, S67N, R99E, R156Q, R161Q, P167T
   Mutant 6:
      R20E, Y50V, S67N, R99E, R156Q, R161E, P167T
   Mutant 7:
      R20Q, Y50V, S67N, R99Q, R156E, R161E, P167T
   Mutant 8:
      E13S, P24T, Y50V, R78E, R99Q, Q109D, R128E, R156Q, R161E, P167T
   Mutant 9:
      E13S, P24T, Y50V, R78Q, R99E, Q109D, R128Q, R156E, R161Q, P167T
   Mutant 10:
      E13S, P24T, Y50V, R78E, R99Q, Q109D, R128E, R156Q, R161E, P167T, W192F
   Mutant 11:
      E13S, P24T, Y50V, R78Q, R99E, Q109D, R128Q, R156E, R161Q, P167T, W192F
C. Nucleotide sequences of mutants
   Mutant 1:
      P24T, Y50V, R78E, R99Q, R156Q, R161E, P167T 
ACT AAC GCC TGC AGT ATC AAT GGA AAT GCT CCA GCT GAA ATC GAT TTG CGA CAA ATG CGA 60
ACT GTC ACT ACC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
GAA TTA GTC GAT TGT GCT TCC CAA CAC GGT TGT CAT GGT GAT ACC ATT CCA GAA GGT ATT 240
GAA TAC ATC CAA CAT AAT GGT GTC GTC CAA GAA AGC TAC TAT CGA TAC GTT GCA CAG GAA 300
CAA TCA TGC CGA CGA CCA AAT GCA CAA CGT TTC GGT ATC TCA AAC TAT TGC CAA ATT TAC 360
CCA CCA AAT GTA AAC AAA ATT CGT GAA GCT TTG GCT CAA ACC CAC AGC GCT ATT GCC GTC 420
ATT ATT GGC ATC AAA GAT TTA GAC GCA TTC CGT CAT TAT GAT GGC CAG ACA ATC ATT CAA 480
GAA GAT AAT GGT TAC CAA ACC AAC TAT CAC GCT GTC AAC ATT GTT GGT TAC AGT AAC GCA 540
CAA GGT GTC GAT TAT TGG ATC GTA CGA AAC AGT TGG GAT ACC AAT TGG GGT GAT AAT GGT 600
TAC GGT TAT TTT GCT GCC AAC ATC GAT TTG ATG ATG ATT GAA GAA TAT CCA TAT GTT GTC 660
ATT CTC 666
   Mutant 2:
      P24T, Y50V, R78Q, R99E, R156E, R161Q, P167T ACT AAC GCC TGC AGT ATC AAT GGA AAT GCT CCA GCT GAA ATC GAT TTG CGA CAA ATG CGA 60 
ACT GTC ACT ACC ATT CGT ATG CAA GGA GGC TGT GGT TCA TGT TGG GCT TTC TCT GGT GTT 120

GCC GCA ACT GAA TCA GCT TAT TTG GCT GTG CGT AAT CAA TCA TTG GAT CTT GCT GAA CAA 180

GAA TTA GTC GAT TGT GCT TCC CAA CAC GGT TGT CAT GGT GAT ACC ATT CCA CAG GGT ATT 240

GAA TAC ATC CAA CAT AAT GGT GTC GTC CAA GAA AGC TAC TAT CGA TAC GTT GCA GAA GAA 300

CAA TCA TGC CGA CGA CCA AAT GCA CAA CGT TTC GGT ATC TCA AAC TAT TGC CAA ATT TAC 360

CCA CCA AAT GTA AAC AAA ATT CGT GAA GCT TTG GCT CAA ACC CAC AGC GCT ATT GCC GTC 420

ATT ATT GGC ATC AAA GAT TTA GAC GCA TTC CGT CAT TAT GAT GGC GAA ACA ATC ATT CAA 480

CAG GAT AAT GGT TAC CAA ACC AAC TAT CAC GCT GTC AAC ATT GTT GGT TAC AGT AAC GCA 540

CAA GGT GTC GAT TAT TGG ATC GTA CGA AAC AGT TGG GAT ACC AAT TGG GGT GAT AAT GGT 600

TAC GGT TAT TTT GCT GCC AAC ATC GAT TTG ATG ATG ATT GAA GAA TAT CCA TAT GTT GTC 660

ATT CTC 666
   Mutant 3:
      R20E, Y50V, R78Q, R99Q, R156E, R161E, P167T 
ACT AAC GCC TGC AGT ATC AAT GGA AAT GCT CCA GCT GAA ATC GAT TTG CGA CAA ATG GAA 60
ACT GTC ACT CCC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
GAA TTA GTC GAT TGT GCT TCC CAA CAC GGT TGT CAT GGT GAT ACC ATT CCA CAG GGT ATT 240
GAA TAC ATC CAA CAT AAT GGT GTC GTC CAA GAA AGC TAC TAT CGA TAC GTT GCA CAG GAA 300
CAA TCA TGC CGA CGA CCA AAT GCA CAA CGT TTC GGT ATC TCA AAC TAT TGC CAA ATT TAC 360
CCA CCA AAT GTA AAC AAA ATT CGT GAA GCT TTG GCT CAA ACC CAC AGC GCT ATT GCC GTC 420
ATT ATT GGC ATC AAA GAT TTA GAC GCA TTC CGT CAT TAT GAT GGC GAA ACA ATC ATT CAA 480
CAG GAT AAT GGT TAC CAA ACC AAC TAT CAC GCT GTC AAC ATT GTT GGT TAC AGT AAC GCA 540
CAA GGT GTC GAT TAT TGG ATC GTA CGA AAC AGT TGG GAT ACC AAT TGG GGT GAT AAT GGT 600
TAC GGT TAT TTT GCT GCC AAC ATC GAT TTG ATG ATG ATT GAA GAA TAT CCA TAT GTT GTC 660
ATT CTC 666
   Mutant 4:
      R20Q, Y50V, R78E, R99E, R156Q, R161Q, P167T 
ACT AAC GCC TGC AGT ATC AAT GGA AAT GCT CCA GCT GAA ATC GAT TTG CGA CAA ATG CAG 60
ACT GTC ACT CCC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
 
GAA TTA GTC GAT TGT GCT TCC CAA CAC GGT TGT CAT GGT GAT ACC ATT CCA GAA GGT ATT 240
GAA TAC ATC CAA CAT AAT GGT GTC GTC CAA GAA AGC TAC TAT CGA TAC GTT GCA GAA GAA 300
CAA TCA TGC CGA CGA CCA AAT GCA CAA CGT TTC GGT ATC TCA AAC TAT TGC CAA ATT TAC 360
CCA CCA AAT GTA AAC AAA ATT CGT GAA GCT TTG GCT CAA ACC CAC AGC GCT ATT GCC GTC 420
ATT ATT GGC ATC AAA GAT TTA GAC GCA TTC CGT CAT TAT GAT GGC CAG ACA ATC ATT CAA 480
CAG GAT AAT GGT TAC CAA ACC AAC TAT CAC GCT GTC AAC ATT GTT GGT TAC AGT AAC GCA 540
CAA GGT GTC GAT TAT TGG ATC GTA CGA AAC AGT TGG GAT ACC AAT TGG GGT GAT AAT GGT 600
TAC GGT TAT TTT GCT GCC AAC ATC GAT TTG ATG ATG ATT GAA GAA TAT CCA TAT GTT GTC 660
ATT CTC 666
   Mutant 5:
      P24T, Y50V, S67N, R99E, R156Q, R161Q, P167T 
ACT AAC GCC TGC AGT ATC AAT GGA AAT GCT CCA GCT GAA ATC GAT TTG CGA CAA ATG CGA 60
ACT GTC ACT ACC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
GAA TTA GTC GAT TGT GCT AAC CAA CAC GGT TGT CAT GGT GAT ACC ATT CCA CGT GGT ATT 240
GAA TAC ATC CAA CAT AAT GGT GTC GTC CAA GAA AGC TAC TAT CGA TAC GTT GCA GAA GAA 300
CAA TCA TGC CGA CGA CCA AAT GCA CAA CGT TTC GGT ATC TCA AAC TAT TGC CAA ATT TAC 360
CCA CCA AAT GTA AAC AAA ATT CGT GAA GCT TTG GCT CAA ACC CAC AGC GCT ATT GCC GTC 420
ATT ATT GGC ATC AAA GAT TTA GAC GCA TTC CGT CAT TAT GAT GGC CAG ACA ATC ATT CAA 480
CAG GAT AAT GGT TAC CAA ACC AAC TAT CAC GCT GTC AAC ATT GTT GGT TAC AGT AAC GCA 540
CAA GGT GTC GAT TAT TGG ATC GTA CGA AAC AGT TGG GAT ACC AAT TGG GGT GAT AAT GGT 600
TAC GGT TAT TTT GCT GCC AAC ATC GAT TTG ATG ATG ATT GAA GAA TAT CCA TAT GTT GTC 660
ATT CTC 666
   Mutant 6:
      R20E, Y50V, S67N, R99E, R156Q, R161E, P167T 
ACT AAC GCC TGC AGT ATC AAT GGA AAT GCT CCA GCT GAA ATC GAT TTG CGA CAA ATG GAA 60
ACT GTC ACT CCC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
GAA TTA GTC GAT TGT GCT AAC CAA CAC GGT TGT CAT GGT GAT ACC ATT CCA CGT GGT ATT 240
GAA TAC ATC CAA CAT AAT GGT GTC GTC CAA GAA AGC TAC TAT CGA TAC GTT GCA GAA GAA 300
 
CAA TCA TGC CGA CGA CCA AAT GCA CAA CGT TTC GGT ATC TCA AAC TAT TGC CAA ATT TAC 360
CCA CCA AAT GTA AAC AAA ATT CGT GAA GCT TTG GCT CAA ACC CAC AGC GCT ATT GCC GTC 420
ATT ATT GGC ATC AAA GAT TTA GAC GCA TTC CGT CAT TAT GAT GGC CAG ACA ATC ATT CAA 480
GAA GAT AAT GGT TAC CAA ACC AAC TAT CAC GCT GTC AAC ATT GTT GGT TAC AGT AAC GCA 540
CAA GGT GTC GAT TAT TGG ATC GTA CGA AAC AGT TGG GAT ACC AAT TGG GGT GAT AAT GGT 600
TAC GGT TAT TTT GCT GCC AAC ATC GAT TTG ATG ATG ATT GAA GAA TAT CCA TAT GTT GTC 660
ATT CTC 666
   Mutant 7:
      R20Q, Y50V, S67N, R99Q, R156E, R161E, P167T 
ACT AAC GCC TGC AGT ATC AAT GGA AAT GCT CCA GCT GAA ATC GAT TTG CGA CAA ATG CAG 60
ACT GTC ACT CCC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
GAA TTA GTC GAT TGT GCT AAC CAA CAC GGT TGT CAT GGT GAT ACC ATT CCA CGT GGT ATT 240
GAA TAC ATC CAA CAT AAT GGT GTC GTC CAA GAA AGC TAC TAT CGA TAC GTT GCA CAG GAA 300
CAA TCA TGC CGA CGA CCA AAT GCA CAA CGT TTC GGT ATC TCA AAC TAT TGC CAA ATT TAC 360
CCA CCA AAT GTA AAC AAA ATT CGT GAA GCT TTG GCT CAA ACC CAC AGC GCT ATT GCC GTC 420
ATT ATT GGC ATC AAA GAT TTA GAC GCA TTC CGT CAT TAT GAT GGC GAA ACA ATC ATT CAA 480
GAA GAT AAT GGT TAC CAA ACC AAC TAT CAC GCT GTC AAC ATT GTT GGT TAC AGT AAC GCA 540
CAA GGT GTC GAT TAT TGG ATC GTA CGA AAC AGT TGG GAT ACC AAT TGG GGT GAT AAT GGT 600
TAC GGT TAT TTT GCT GCC AAC ATC GAT TTG ATG ATG ATT GAA GAA TAT CCA TAT GTT GTC 660
ATT CTC 666
   Mutant 8:
      E13S, P24T, Y50V, R78E, R99Q, Q109D, R128E, R156Q, R161E, P167T 
ACT AAC GCC TGC AGT ATC AAT GGA AAT GCT CCA GCT AGC ATC GAT TTG CGA CAA ATG CGA 60
ACT GTC ACT ACC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
GAA TTA GTC GAT TGT GCT TCC CAA CAC GGT TGT CAT GGT GAT ACC ATT CCA GAA GGT ATT 240
GAA TAC ATC CAA CAT AAT GGT GTC GTC CAA GAA AGC TAC TAT CGA TAC GTT GCA CAG GAA 300
CAA TCA TGC CGA CGA CCA AAT GCA GAT CGT TTC GGT ATC TCA AAC TAT TGC CAA ATT TAC 360
 
CCA CCA AAT GTA AAC AAA ATT GAA GAA GCT TTG GCT CAA ACC CAC AGC GCT ATT GCC GTC 420
ATT ATT GGC ATC AAA GAT TTA GAC GCA TTC CGT CAT TAT GAT GGC CAG ACA ATC ATT CAA 480
GAA GAT AAT GGT TAC CAA ACC AAC TAT CAC GCT GTC AAC ATT GTT GGT TAC AGT AAC GCA 540
CAA GGT GTC GAT TAT TGG ATC GTA CGA AAC AGT TGG GAT ACC AAT TGG GGT GAT AAT GGT 600
TAC GGT TAT TTT GCT GCC AAC ATC GAT TTG ATG ATG ATT GAA GAA TAT CCA TAT GTT GTC 660
ATT CTC 666
   Mutant 9:
      E13S, P24T, Y50V, R78Q, R99E, Q109D, R128Q, R156E, R161Q, P167T 
ACT AAC GCC TGC AGT ATC AAT GGA AAT GCT CCA GCT AGC ATC GAT TTG CGA CAA ATG CGA 60
ACT GTC ACT ACC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
GAA TTA GTC GAT TGT GCT TCC CAA CAC GGT TGT CAT GGT GAT ACC ATT CCA CAG GGT ATT 240
GAA TAC ATC CAA CAT AAT GGT GTC GTC CAA GAA AGC TAC TAT CGA TAC GTT GCA GAA GAA 300
CAA TCA TGC CGA CGA CCA AAT GCA GAT CGT TTC GGT ATC TCA AAC TAT TGC CAA ATT TAC 360
CCA CCA AAT GTA AAC AAA ATT CAG GAA GCT TTG GCT CAA ACC CAC AGC GCT ATT GCC GTC 420
ATT ATT GGC ATC AAA GAT TTA GAC GCA TTC CGT CAT TAT GAT GGC GAA ACA ATC ATT CAA 480
CAG GAT AAT GGT TAC CAA ACC AAC TAT CAC GCT GTC AAC ATT GTT GGT TAC AGT AAC GCA 540
CAA GGT GTC GAT TAT TGG ATC GTA CGA AAC AGT TGG GAT ACC AAT TGG GGT GAT AAT GGT 600
TAC GGT TAT TTT GCT GCC AAC ATC GAT TTG ATG ATG ATT GAA GAA TAT CCA TAT GTT GTC 660
ATT CTC 666
   Mutant 10:
      E13S, P24T, Y50V, R78E, R99Q, Q109D, R128E, R156Q, R161E, P167T, W192F 
ACT AAC GCC TGC AGT ATC AAT GGA AAT GCT CCA GCT AGC ATC GAT TTG CGA CAA ATG CGA 60
ACT GTC ACT ACC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
GAA TTA GTC GAT TGT GCT TCC CAA CAC GGT TGT CAT GGT GAT ACC ATT CCA GAA GGT ATT 240
GAA TAC ATC CAA CAT AAT GGT GTC GTC CAA GAA AGC TAC TAT CGA TAC GTT GCA CAG GAA 300
CAA TCA TGC CGA CGA CCA AAT GCA GAT CGT TTC GGT ATC TCA AAC TAT TGC CAA ATT TAC 360
 
CCA CCA AAT GTA AAC AAA ATT GAA GAA GCT TTG GCT CAA ACC CAC AGC GCT ATT GCC GTC 420

ATT ATT GGC ATC AAA GAT TTA GAC GCA TTC CGT CAT TAT GAT GGC CAG ACA ATC ATT CAA 480

GAA GAT AAT GGT TAC CAA ACC AAC TAT CAC GCT GTC AAC ATT GTT GGT TAC AGT AAC GCA 540

CAA GGT GTC GAT TAT TGG ATC GTA CGA AAC AGT TTT GAT ACC AAT TGG GGT GAT AAT GGT 600

TAC GGT TAT TTT GCT GCC AAC ATC GAT TTG ATG ATG ATT GAA GAA TAT CCA TAT GTT GTC 660

ATT CTC 666
   Mutant 11:
      E13S, P24T, Y50V, R78Q, R99E, Q109D, R128Q, R156E, R161Q, P167T, W192F

ACT AAC GCC TGC AGT ATC AAT GGA AAT GCT CCA GCT AGC ATC GAT TTG CGA CAA ATG CGA 60
ACT GTC ACT ACC ATT CGT ATG CAA GGA GGC TGT GGT TCA TGT TGG GCT TTC TCT GGT GTT 120
GCC GCA ACT GAA TCA GCT TAT TTG GCT GTG CGT AAT CAA TCA TTG GAT CTT GCT GAA CAA 180
GAA TTA GTC GAT TGT GCT TCC CAA CAC GGT TGT CAT GGT GAT ACC ATT CCA CAG GGT ATT 240
GAA TAC ATC CAA CAT AAT GGT GTC GTC CAA GAA AGC TAC TAT CGA TAC GTT GCA GAA GAA 300
CAA TCA TGC CGA CGA CCA AAT GCA GAT CGT TTC GGT ATC TCA AAC TAT TGC CAA ATT TAC 360
CCA CCA AAT GTA AAC AAA ATT CAG GAA GCT TTG GCT CAA ACC CAC AGC GCT ATT GCC GTC 420
ATT ATT GGC ATC AAA GAT TTA GAC GCA TTC CGT CAT TAT GAT GGC GAA ACA ATC ATT CAA 480
CAG GAT AAT GGT TAC CAA ACC AAC TAT CAC GCT GTC AAC ATT GTT GGT TAC AGT AAC GCA 540
CAA GGT GTC GAT TAT TGG ATC GTA CGA AAC AGT TTT GAT ACC AAT TGG GGT GAT AAT GGT 600
TAC GGT TAT TTT GCT GCC AAC ATC GAT TTG ATG ATG ATT GAA GAA TAT CCA TAT GTT GTC 660
ATT CTC 666

### EXAMPLE 8

### Mutated recombinant grass allergens (Phl p 5) with improved safety for specific allergy vaccination

In this example the application of the concept of the current invention on grass pollen allergens is exemplified by one allergen, Phl p 5. Manipulation of other grass pollen allergens may be performed by equivalent procedures.

### Design of mutated recombinant Phl p 5 molecules

SEQ ID NO. 5 shows the nucleotide and deduced amino acid sequence of the Phl p 5.0103 clone, which is a wild-type isoform.

SEQ ID NO. 5: Nucleotide and deduced amino acid sequence of Phl p 5.0103.

gccgatctcggttacggccccgccaccccagctgccccggccgccggctacacccccgcc 60
A D L G Y G P A T P A A P A A G Y T P A 20
acccccgccgccccggccggagcggagccagcaggtaaggcgacgaccgaggagcagaag 120
T P A A P A G A E P A G K A T T E E Q K 40
ctgatcgagaagatcaacgccggcttcaaggcggccttggccgctgccgccggcgtcccg 180
L I E K I N A G F K A A L A A A A G V P 60
ccagcggacaagtacaggacgttcgtcgcaaccttcggcgcggcctccaacaaggccttc 240
P A D K Y R T F V A T F G A A S N K A F 80
gcggagggcctctcgggcgagcccaagggcgccgccgaatccagctccaaggccgcgctc 300
A E G L S G E P K G A A E S S S K A A L 100
acctccaagctcgacgccgcctacaagctcgcctacaagacagccgagggcgcgacgcct 360
T S K L D A A Y K L A Y K T A E G A T P 120
gaggccaagtacgacgcctacgtcgccaccgtaagcgaggcgctccgcatcatcgccggc 420
E A K Y D A Y V A T V S E A L R I I A G 140
accctcgaggtccacgccgtcaagcccgcggccgaggaggtcaaggtcatccccgccggc 480
T L E V H A V K P A A E E V K V I P A G 160
gagctgcaggtcatcgagaaggtcgacgccgccttcaaggtcgctgccaccgccgccaac 540
E L Q V I E K V D A A F K V A A T A A N 180
gccgcccccgccaacgacaagttcaccgtcttcgaggccgccttcaacgacgccatcaag 600
A A P A N D K F T V F E A A F N D A I K 200
gcgagcacgggcggcgcctacgagagctacaagttcatccccgccctggaggccgccgtc 660
A S T G G A Y E S Y K F I P A L E A A V 220
aagcaggcctacgccgccaccgtcgccaccgcgccggaggtcaagtacactgtctttgag 720
K Q A Y A A T V A T A P E V K Y T V F E 240
accgcactgaaaaaggccatcaccgccatgtccgaagcacagaaggctgccaagcccgcc 780
T A L K K A I T A M S E A Q K A A K P A 260
gccgctgccaccgccaccgcaaccgccgccgttggcgcggccaccggcgccgccaccgcc 840
A A A T A T A T A A V G A A T G A A T A 280
gctactggtggctacaaagtc 861

A T G G Y K V

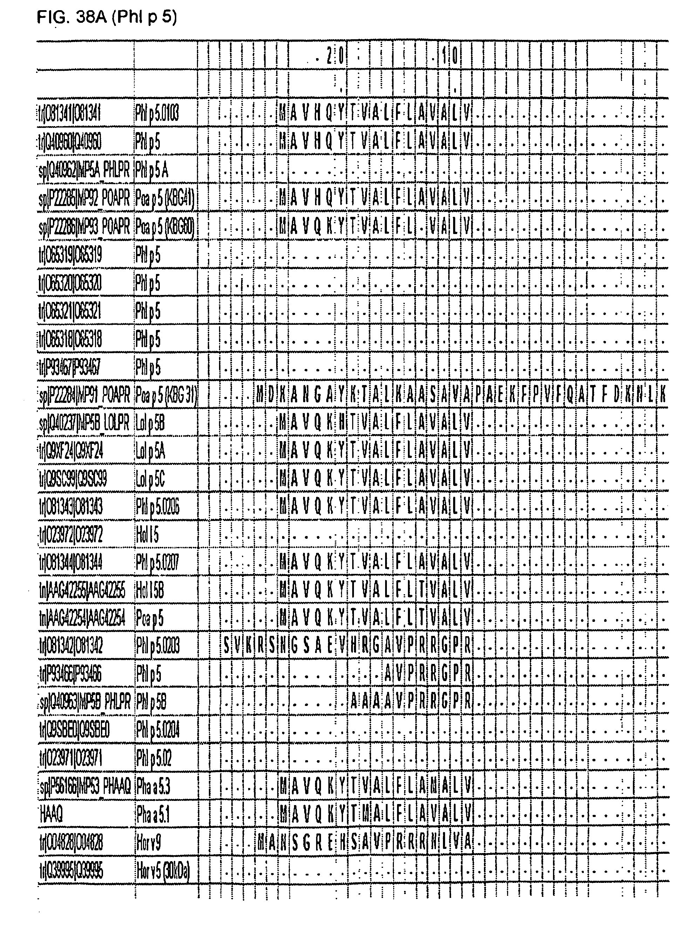

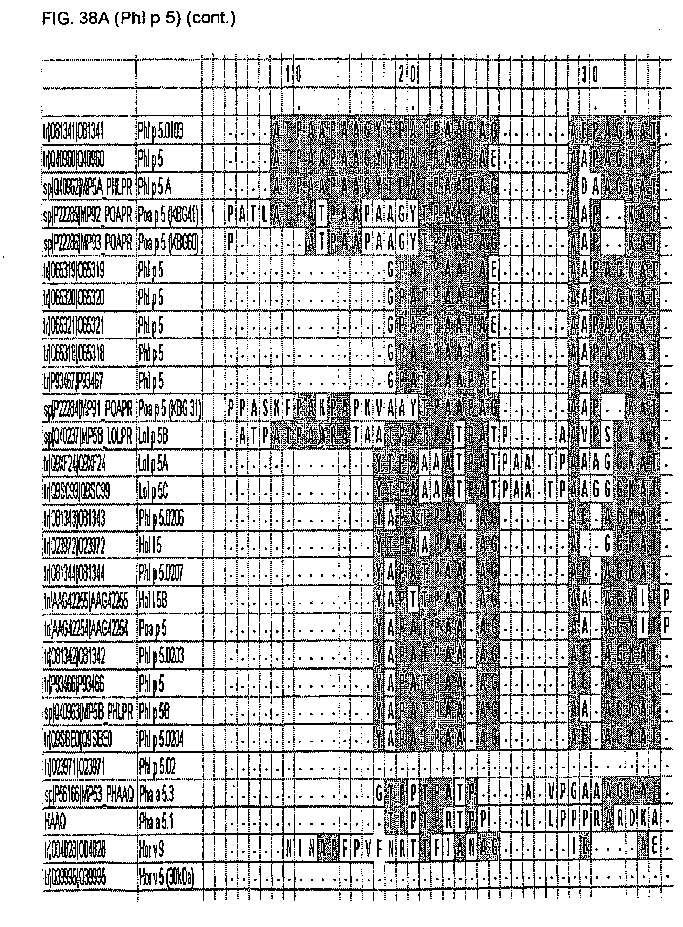

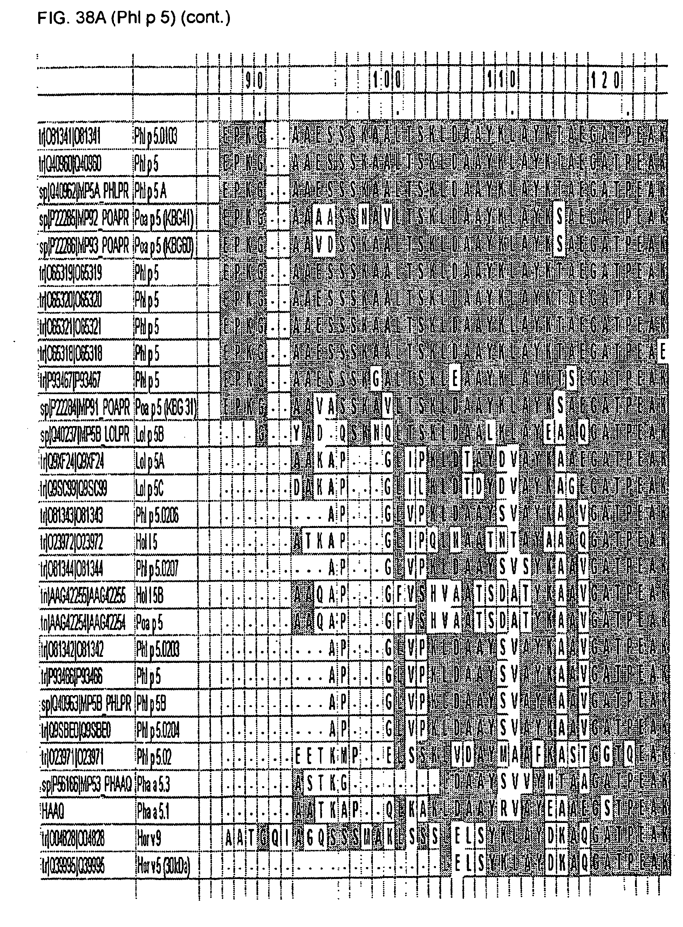

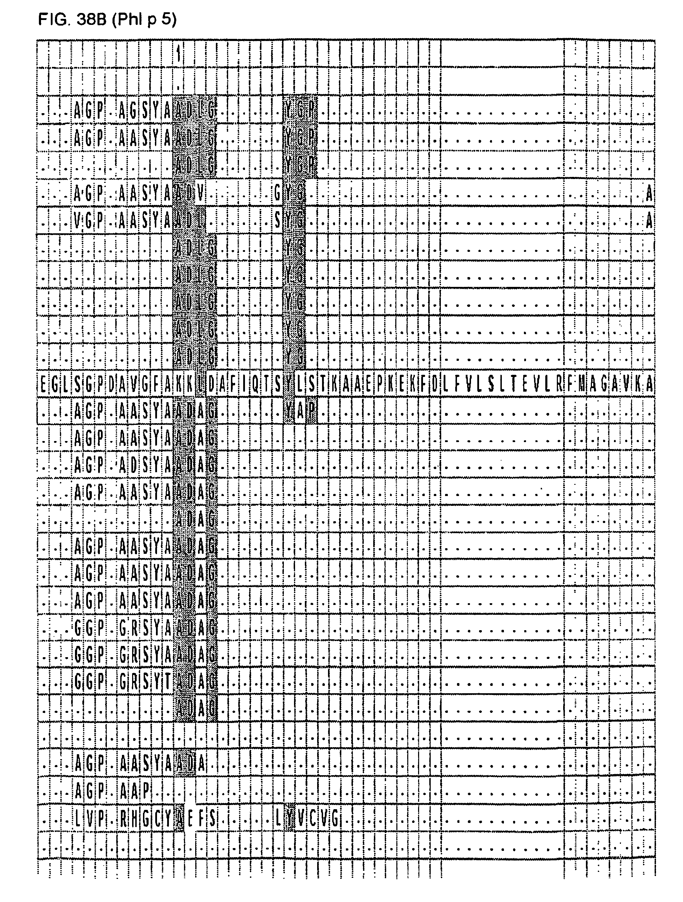

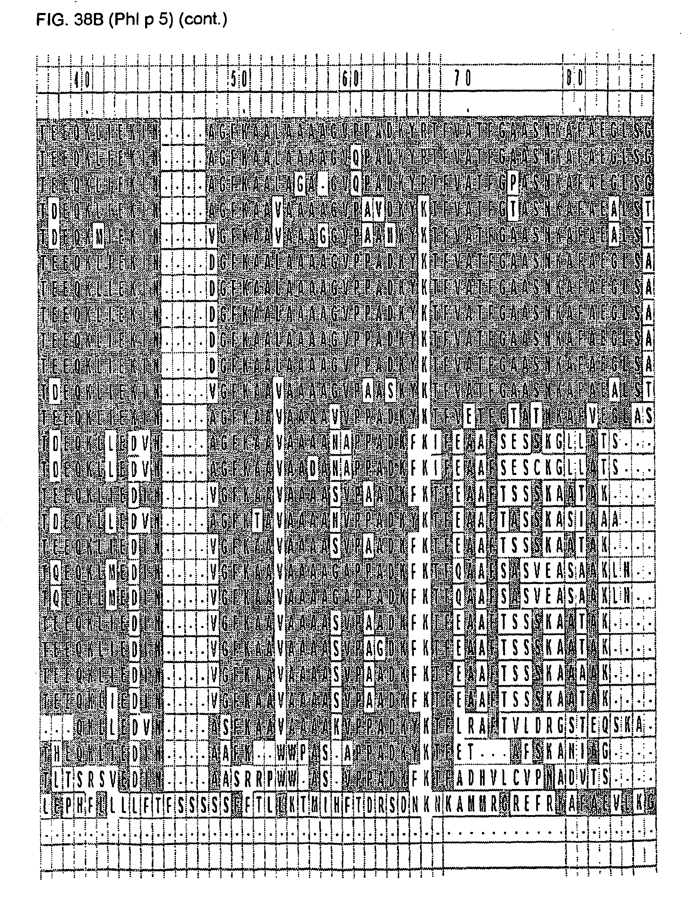

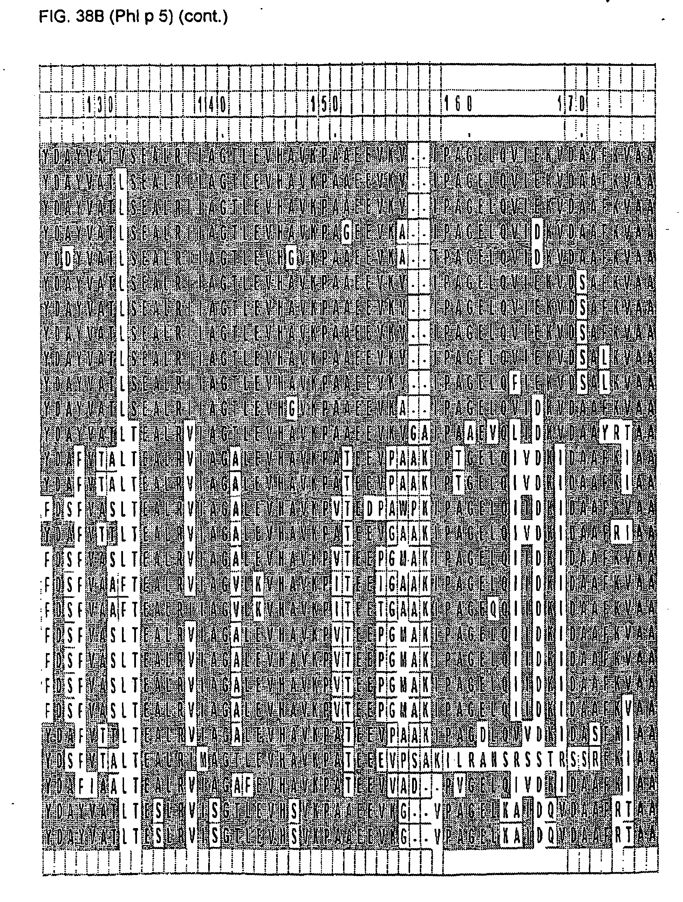

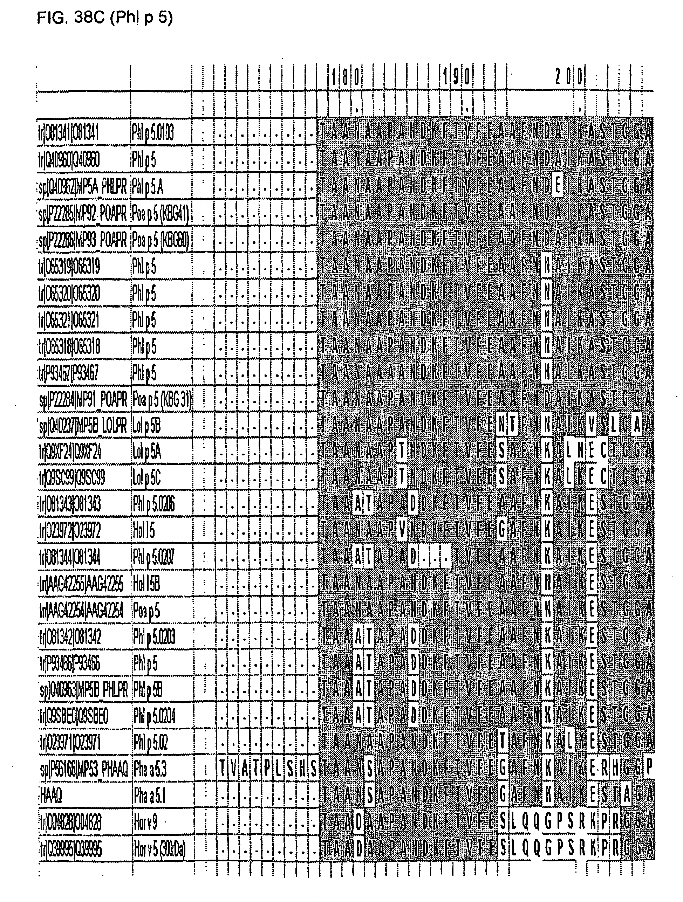

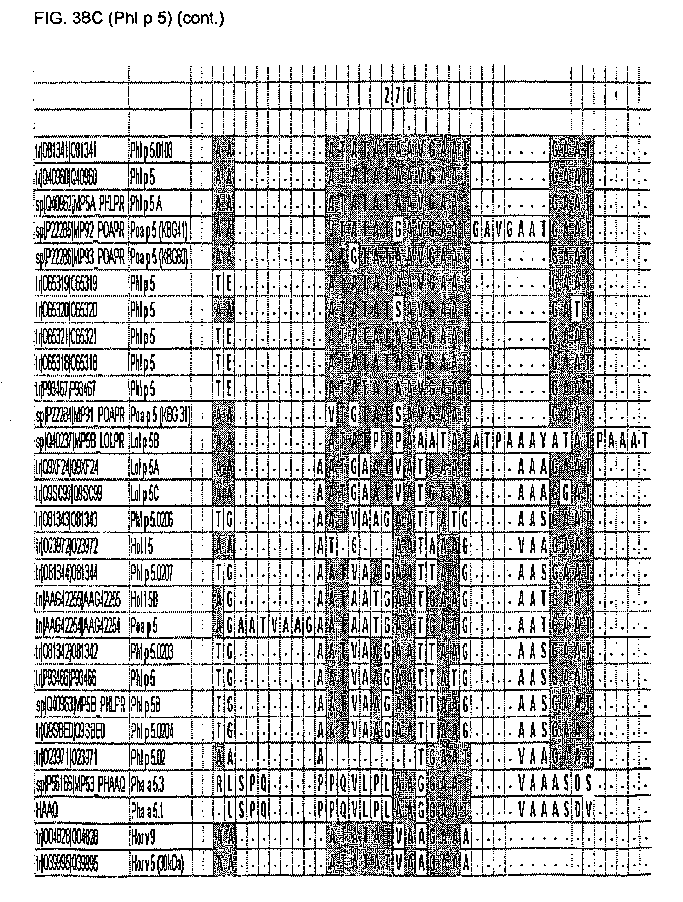

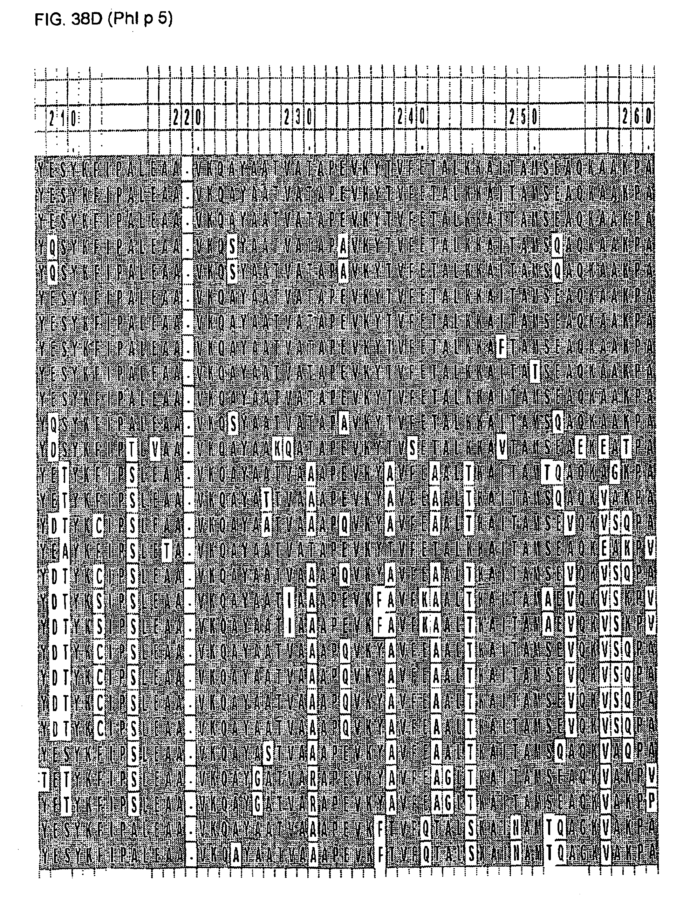

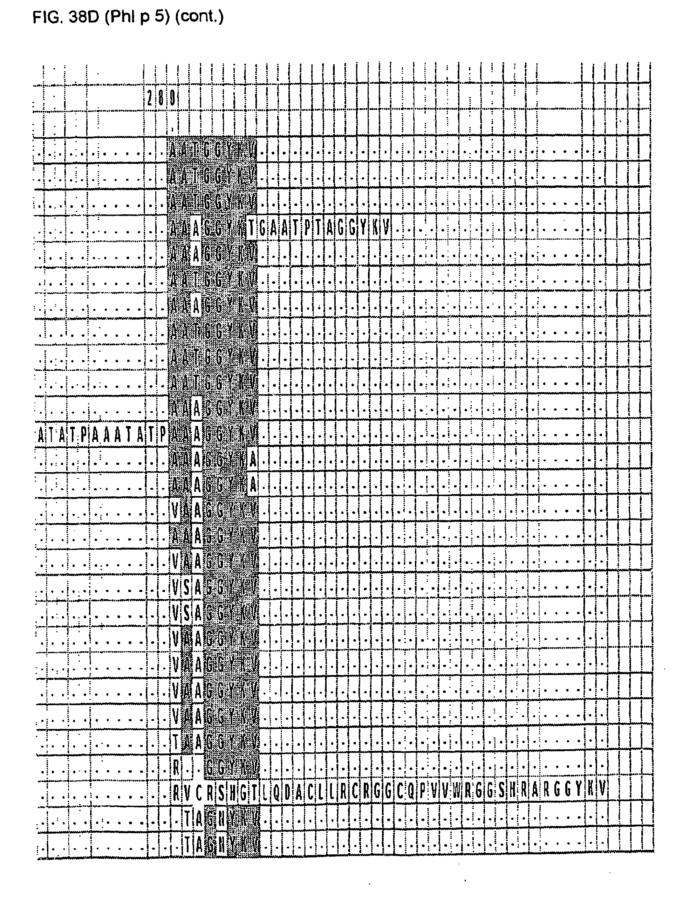

Fig. 38 shows a sequence alignment performed at the ExPaSy Molecular Biology Server (http://www.expasy.ch/) using the ClustalW algorithm on a BLAST search using the Phl p 5.0103 amino acid sequence shown in SEQ ID NO. 5 as input sequence. The alignment includes group 5 allergen sequences from grass species, i.e. Phl p 5, Poa p 5, Lol p 5, Hol 1 5, Pha a 5, Hor v 9 and Hor v 5. In Fig. 38 amino acid residues identical to amino acids in the same position in the Phl p 5.0103 protein sequence are highlighted using black letters on grey background. Non-identical amino acids are printed in black on a white background.

### Surface structure images

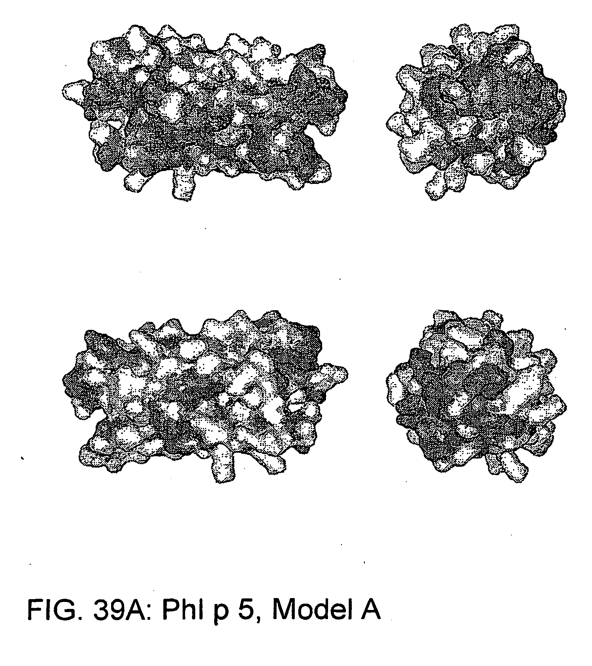

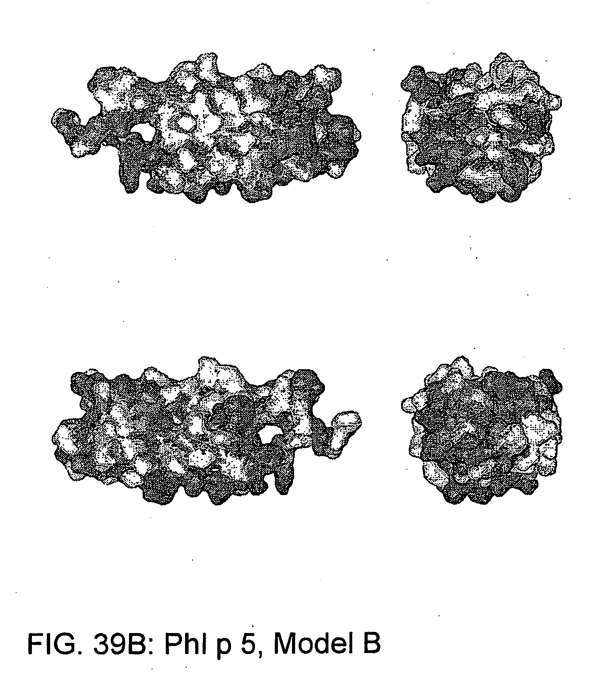

Amino acid sequences representing the grass pollen group 5 allergens are similar to a certain degree and some of the molecular surface is conserved (grey-coloured zones), see Fig. 39. Fig. 39 shows surface contours viewed from 4 different angles when superimposing the Phl p 5.0103 protein sequence on to a Phl p 5 molecular structure model. The structure model encompass the molecule in two halves, Model A (amino acid 34-142) shown in Fig. 39A, and Model B (amino acid 149-259) shown in Fig. 39B.

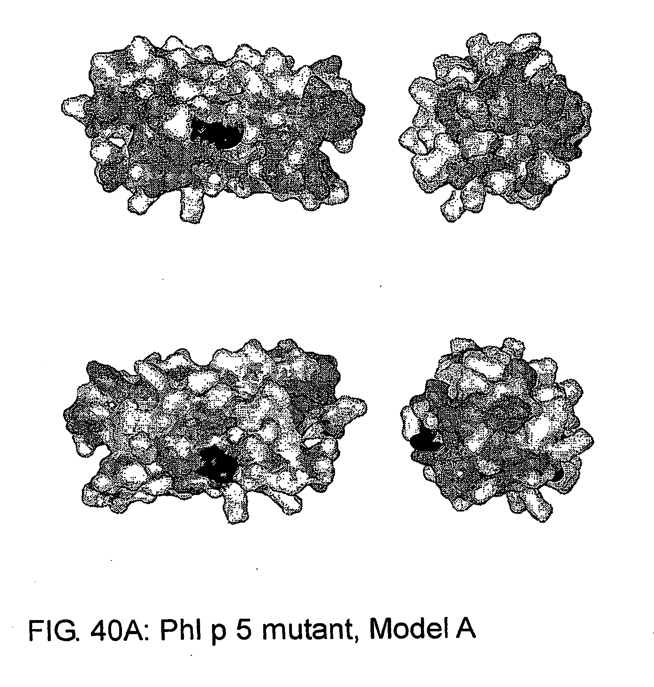

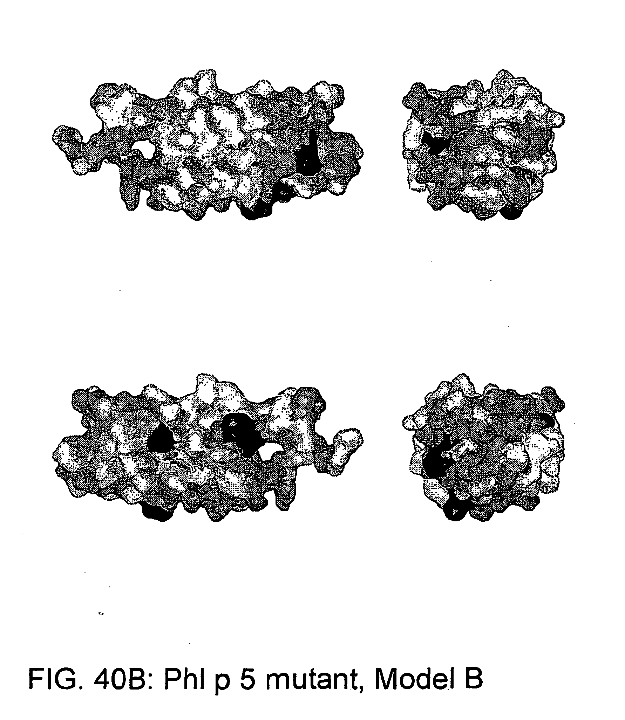

Highly solvent exposed amino acid spatially distributed over the entire surface within distances in the range of 25-30 Å are selected for mutation. In the sections below, the following information is given: A list of amino acids considered to be appropriate for mutation (A), a list of the mutants designed (B) and the DNA sequences representing the mutants designed (C). Fig. 40 A and B shows surface contours of mutant number 1 Model A and Model B, respectively, as an example. Grey colour indicates conserved amino acid residues. Black colour indicates amino acid residues selected for mutation.
A: List of amino acids selected for mutation
   145, R66, E133, R136, 1137, D186, F188, K211, P214, Q222, P232, L243, Q254
B. List of mutants designed
   Mutant 1:
      I45K, E133S, F188I, Q222K, L243E, Q254K
   Mutant 2:
      R66N, E133S, F188I, Q222K, L243E, Q254K
   Mutant 3:
      I45K, R136S, F188I, Q222K, L243E, Q254K
   Mutant 4:
      I45K, I137K, F188I, Q222K, L243E, Q254K
   Mutant 5:
      I45K, E133S, D186H, Q222K, L243E, Q254K
   Mutant 6:
      I45K, E133S, Q222K, P232G, L243E, Q254K
   Mutant 7:
      I45K, E133S, F188I, P214G, L243E, Q254K
   Mutant 8:
      I45K, E133S, F188I, K211N, L243E, Q254K
   Mutant 9:
      R66N, R136S, F188I, Q222K, L243E, Q254K
   Mutant 10:
      R66N, I137K, F188I, Q222K, L243E, Q254K
   Mutant 11:
      I45K, E133S, D186H, P214G, L243E, Q254K
   Mutant 12:
      I45K, E133S, D186H, K211N, L243E, Q254K
   Mutant 13:
      I45K, E133S, P214G, P232G, L243E, Q254K
   Mutant 14:
      I45K, E133S, K211N, P232G, L243E, Q254K
C. Nucleotide sequence of mutants
   Mutant 1:
      I45K, E133S, F188I, Q222K, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagATTaccgtcttcgaggccgccttcaacgacgccatcaag 600
gcgagcacgggcggcgcctacgagagctacaagttcatccccgccctggaggccgccgtc 660
aagAAAgcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 2:
      R66N, E133S, F188I, Q222K, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagatcaacgccggcttcaaggcggccttggccgctgccgccggcgtcccg 180
ccagcggacaagtacAACacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
 
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagATTaccgtcttcgaggccgccttcaacgacgccatcaag 600
gcgagcacgggcggcgcctacgagagctacaagttcatccccgccctggaggccgccgtc 660
aagAAAgcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 3:
      I45K, R136S, F188I, Q222K, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcggcgagcccaaggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcgaggcgctcAGCatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagATTaccgtcttcgaggccgccttcaacgacgccatcaag 600
gcgagcacgggcggcgcctacgagagctacaagttcatccccgccctggaggccgccgtc 660
aagAAAgcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 4:
      I45K, I137K, F188I, Q222K, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgaggcgcgacgcct 360
gaggccaagtacgacgcctacgtcgccaccgtaagcgaggcgctccgcAAAatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagATTaccgtcttcgaggccgccttcaacgacgccatcaag 600
gcgagcacgggcggcgcctacgagagctacaagttcatccccgccctggaggccgccgtc 660
aagAAAgcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 5:
      I45K, E133S, D186H, Q222K, L243E, Q254K: gccgatctcggttacggccccgccaccccagctgccccggccgccggctacacccccgcc 60 
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacCATaagttcaccgtcttcgaggccgccttcaacgacgccatcaag 600
gcgagcacgggcggcgcctacgagagctacaagttcatccccgccctggaggccgccgtc 660
aagAAAgcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 6:
      I45K, E133S, Q222K, P232G, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagttcaccgtcttcgaggccgccttcaacgacgccatcaag 600
gcgagcacgggcggcgcctacgagagctacaagttcatccccgccctggaggccgccgtc 660
aagAAAgcctacgccgccaccgtcgccaccgcgGGCgaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 7:
      I45K, E133S, F188I, P214G, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagATTaccgtcttcgaggccgccttcaacgacgccatcaag 600
gcgagcacgggcggcgcctacgagagctacaagttcatcGGCgccctggaggccgccgtc 660
aagcaggcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 8:
      I45K, E133S, F188I, K211N, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagATTaccgtcttcgaggccgccttcaacgacgccatcaag 600
gcgagcacgggcggcgcctacgagagctacAACttcatccccgccctggaggccgccgtc 660
aagcaggcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 9:
      R66N, R136S, F188I, Q222K, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagatcaacgccggcttcaaggcggccttggccgctgccgccggcgtcccg 180
ccagcggacaagtacAACacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcgaggcgctcAGCatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagATTaccgtcttcgaggccgccttcaacgacgccatcaag 600
gcgagcacgggcggcgcctacgagagctacaagttcatccccgccctggaggccgccgtc 660
aagAAAgcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 10:
      R66N, I137K, F188I, Q222K, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagatcaacgccggcttcaaggcggccttggccgctgccgccggcgtcccg 180
ccagcggacaagtacAACacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcgaggcgctccgcAAAatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagATTaccgtcttcgaggccgccttcaacgacgccatcaag 600
gcgagcacgggcggcgcctacgagagctacaagttcatccccgccctggaggccgccgtc 660
aagAAAgcctacgccgccaccgtcgccaccgcgccggaggtcaagtacactgtctttgag 720
accgcaGAAaaaaaggccatcaccgccatgtccgaagcaAAAaaggctgccaagcccgcc 780
 
gccgctgccaccgc[?]accgcaaccgccgccgttggcgcggccaccggcgccgccaccgcc 840
gctactggtggctacaaagtc 861
   Mutant 11:
      I45K, E133S, D186H, P214G, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacCATaagttcaccgtcttcgaggccgccttcaacgacgccatcaag 600
gcgagcacgggcggcgcctacgagagctacaagttcatcGGCgccctggaggccgccgtc 660
aagcaggcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 12:
      I45K, E133S, D186H, K211N, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcgggcgagcccaaggcgccgccgaatccagctccaaggccgcgctc 300
acctccaagctcgacgccgcctacaagctcgcctacaagacagccgaggcgcgacgcct 360
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacCATaagttcaccgtcttcgaggccgccttcaacgacgccatcaag 600
gcgagcacgggcggcgcctacgagagctacAACttcatccccgccctggaggccgccgtc 660
aagcaggcctacgccgccaccgtcgccaccgcgccggaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861
   Mutant 13:
      I45K, E133S, P214G, P232G, L243E, Q254K: 
gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
 
gagctgcaggtcatcgagaaggtcgacgccgccttcaaggtcgctgccaccgccgccaac 540
gccgcccccgccaacgacaagttcaccgtcttcgaggccgccttcaacgacgccatcaag 600
gcgagcacgggcggcgcctacgagagctacaagttcatcGGCgccctggaggccgccgtc 660
aagcaggcctacgccgccaccgtcgccaccgcgGGCgaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
   Mutant 14:
      I45K, E133S, K211N, P232G, L243E, Q254K:

gccgatctcggttacggccccgccaccccagctgccccggccgccggctacacccccgcc 60
acccccgccgccccggccggagcggagccagcaggtaaggcgacgaccgaggagcagaag 120
ctgatcgagaagAAAaacgccggcttcaaggcggccttggccgctgccgccggcgtcccg 180
ccagcggacaagtacaggacgttcgtcgcaaccttcggcgcggcctccaacaaggccttc 240
gcggagggcctctcgggcgagcccaagggcgccgccgaatccagctccaaggccgcgctc 300
acctccaagctcgacgccgcctacaagctcgcctacaagacagccgagggcgcgacgcct 360
gaggccaagtacgacgcctacgtcgccaccgtaagcAGCgcgctccgcatcatcgccggc 420
accctcgaggtccacgccgtcaagcccgcggccgaggaggtcaaggtcatccccgccggc 480
gagctgcaggtcatcgagaaggtcgacgccgccttcaaggtcgctgccaccgccgccaac 540
gccgcccccgccaacgacaagttcaccgtcttcgaggccgccttcaacgacgccatcaag 600
gcgagcacgggcggcgcctacgagagctacAACttcatccccgccctggaggccgccgtc 660
aagcaggcctacgccgccaccgtcgccaccgcgGGCgaggtcaagtacactgtctttgag 720
accgcaGAAaaaaaggccatcaccgccatgtccgaagcaAAAaaggctgccaagcccgcc 780
gccgctgccaccgccaccgcaaccgccgccgttggcgcggccaccggcgccgccaccgcc 840
gctactggtggctacaaagtc 861

### EXAMPLE 9

### T-cell reactivity of recombinant and mutant Bet v 1:

### Purpose:

To investigate an *in vitro* T-cell response to the mutated allergens in terms of proliferation and' cytokine production.

### Methods:

PBL (Peripheral blood lymphocytes) from allergic patients were used in the following investigation.

Eight bet v 1 specific T-cell lines were established from the PBL with naturally purified bet v 1 in order to sustain the variety of bet v 1 isoforms the T-cells are presented to, as described in a previously published protocol (26).

Ten PBL and eight T-cell lines were stimulated with birch extract (Bet v), naturally purified bet v 1 (nBet v 1), recombinant Bet v 1 (rBet v 1 or wt; 27) and four different mutated forms of rBet v 1 (described elsewhere): 2595, 2628, 2637, 2744, 2773. The 2637 mutant was later found to be partly unfolded and will not be discussed.

In brief: In a round-bottomed 96 well plate PBL were added in 2 x 10⁵ per well. The different birch samples were added in three different concentrations in quadroplicates and allowed to grow for 6 days. At day 6 cell half of volume (100 µl) from each well with the highest concentration of birch were harvested for cytokine production. Radioactive labelled thymidine was added to the wells. Next day (day 7) the cells were harvested on a filter. Scintilation fluid was added to the filter and the radioactivity was measured in a scintilation counter.

Likewise in a 96 well round-bottomed 96 well plate T-cells were added in 3x10⁴ T-cells per well and stimulated with irradiated autologous PBL (1x10⁵ cells/well) and 3 different concentrations of the different birch samples. After 1 day cells from each well with the highest concentration birch were harvested for cytokine production. Radioactive labelled thymidine were added to the wells. At day 2 the cells were harvested onto a filter and counted as described for PBL.

Supernatant from the quadroplicates were pooled and cytokines were measured using a CBA (cytokine bead array) kit from Becton Dickinson.

### Results:

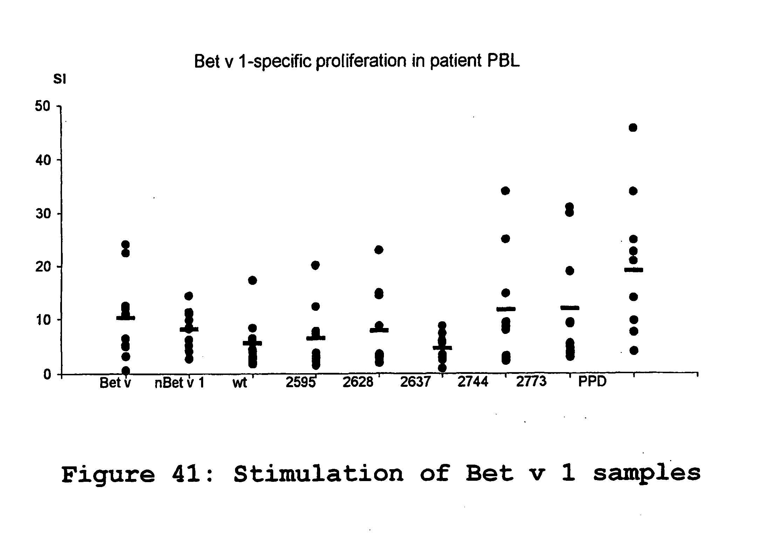

Ten PBL cultures showed specific stimulation to birch. In general proliferation of the PBL to the different birch samples were similar, although variations could be seen. In 3 PBL, nBet v 1 stimulated proliferation better than rBet v 1 and the mutants. The mutant birch samples stimulated PBL almost identical to rBet v 1 (Fig. 41). Fig. 41 shows the Stimulation Index for the above-mentioned Bet v 1 preparations. The Stimulation Index (SI) is calculated as proliferation (cpm: count per minute) of the stimulated sample (highest concentration) divided with the proliferation (cpm) of the medium control. PPD designates purified protein derivative from mucobacterium tuberculosis, which serves as a positive control.

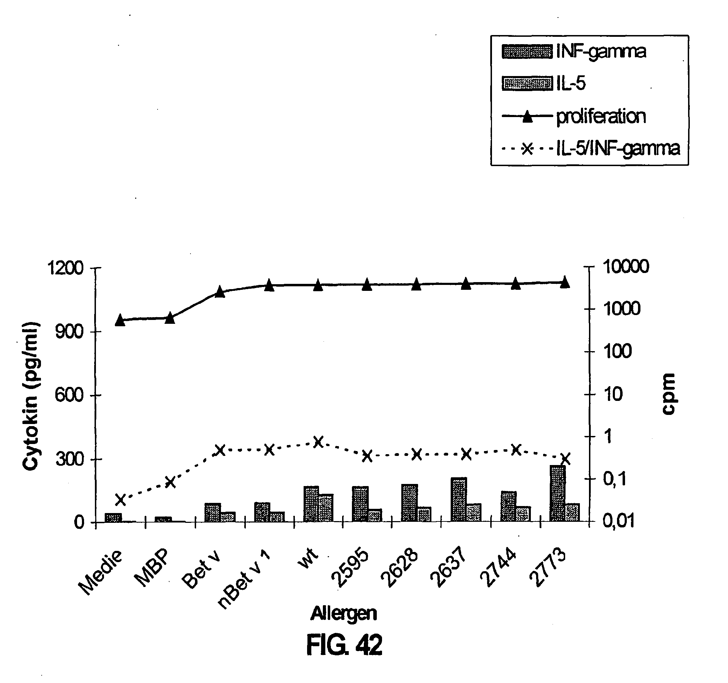

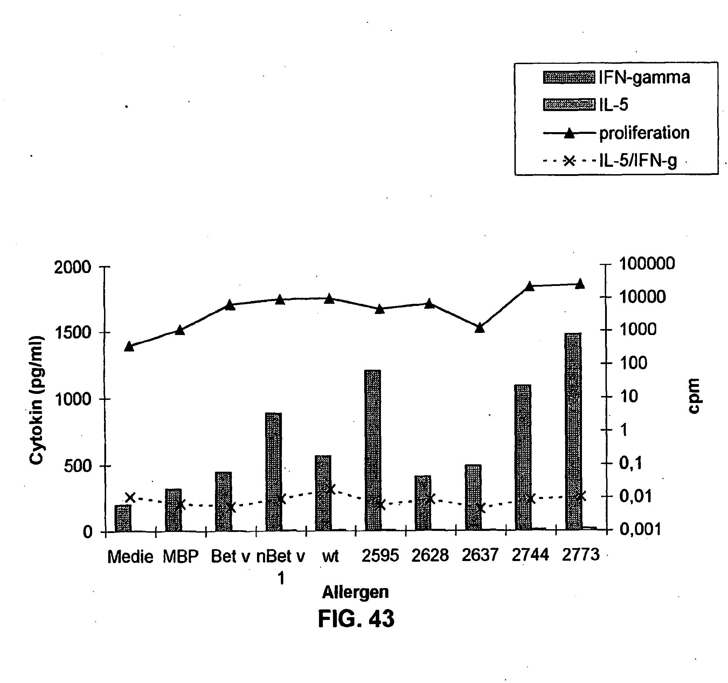

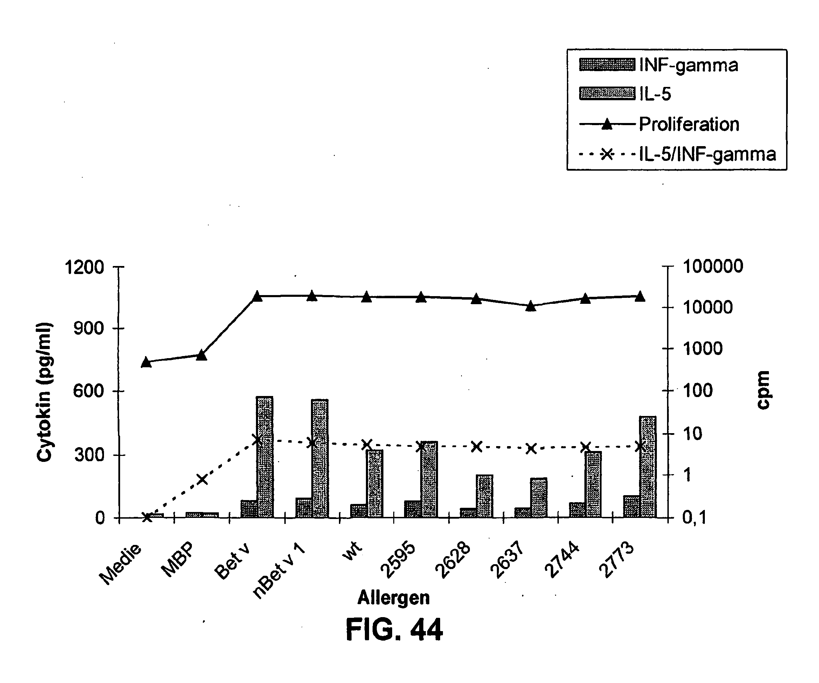

Cytokine production was dominated by IFN-gamma and increased proportionally with PBL proliferation. No signs of a Th1/Th2 shift were apparent (Fig. 42-44). Figure 42 shows a patient with a Th0 profile, Figure 43 a Th1 profile and Figure 44 a Th2 profile. Cytokine production is measured in pg/ml indicated as the bars and the ratio between IL-5/IFN-gamma is the lower dashed line (Y-axis to the right). Proliferation is measured in cpm seen on the Y-axis to the right as a solid line measured in cpm. Medium and MBP (maltose bindig protein) are included as background controls.

Eight T-cell lines established on nBet v 1 and all, except one, proliferated equally well to all birch samples. Four T-cell lines were secreting Th0 like cytokines based on the IL-5 and IFN-gamma ratio (Th2 > 5, 5 > Th0 > 0.2, 0.2 > Th1). Three T-cell lines were secreting Th1 cytokines and one T-cell line was secreting Th2 cytokines. The IL-5/IFN-gamma ratio was not affected by the different birch samples.

### Conclusion:

All PBL cultures and 7/8 T-cell lines that showed specific stimulation to nBet v 1 did also respond to rBet v 1 and the mutants. These data suggests that for T-cell stimulation a single isoform of Bet v 1 or these 4 mutants can substitute for the mixture of individual isoforms found in the natural allergen preparations. Thus, vaccines based on recombinant allergens or these 4 mutants will address the existing Bet v 1 specific T-cell population.

### EXAMPLE 10

### Induction of Bet v 1 specific IgG antibodies and blocking antibodies following immunization with recombinant and mutant Bet v 1 proteins:

In this section the term "blocking antibodies" is defined as antibodies, different from human IgE antibodies, that are able to bind to an antigen and prevent the binding of human IgE antibodies to that antigen.

The ability of recombinant Bet v1 2227 wild type protein (rBet v 1) and Bet v 1 2595, 2628, 2744 and 2773 mutant proteins to induce Bet v 1 specific IgG antibodies and blocking antibodies was tested in immunization experiments in mice.

BALB/cA mice (8 in each group) were immunized by intraperitoneal injections with recombinant Bet v1 2227 wild type protein or the four mutant proteins. The mice were immunized four times with a dose interval of 14 days. The different proteins were conjugated to 1,25 mg/ml Alhydrogel, (Aluminium Hydroxide gel, 1,3 % pH 8.0 - 8.4, Superfos Biosector). The mice were immunized with either 1 ug protein/dose or 10 ug protein/dose. Blood samples were drawn by orbital bleed at day 0,14,35, 21, 49 and 63.

Specific IgG antibody levels was analyzed by direct ELISA using rBet v 1 coated microtiterplates and biotinylated rabbit anti mouse IgG antibodies (Jackson) as detection antibody. Immunization with recombinant Bet v1 2227 wild type protein or the four mutant proteins induced a strong r Bet v 1 specific IgG response. This finding demonstrates that the four mutated proteins are able to induce antibodies that are highly cross reactive to the Bet v 1 2227 wild type protein

To assess the induction of blocking antibodies, serum samples from birch pollen allergic patients were incubated with paramagnetic beads coated with a monoclonal mouse anti-human IgE antibody. After incubation, the beads were washed and resuspended in buffer or diluted samples (1:100) of mouse serum from unimmunized mice (control) or mice immunized as described above. Biotinylated r Bet v 1 was then added to this mixture of beads and mouse serum antibodies. After incubation, the beads were washed and bound biotinylated rBet v 1 was detected using acridinium labeled streptavidine. Incubation of beads with serum from unimmunized mice did not change the binding of r Bet v 1 to the beads. In contrast, incubation of the beads with serum from mice immunized with the recombinant Bet v1 2227 wild type protein or the four mutant proteins significantly reduced binding of r Bet v 1 to the beads demonstrating the presence of Bet v 1 specific blocking antibodies in the serum samples. Thus, at day 63 one or more serum samples from all high dose (10 ug/dose) immunization groups were able to reduce binding of r Bet v1 to the beads with more than 80%. These findings demonstrate that the four mutated proteins are able to induce antibodies that can act as Bet v 1 specific blocking antibodies.

### REFERENCES

1. WO 97/30150 (Pangenetics B.V., Molecules for the induction of immunological tolerance)
2. WO 92/02621 (Biomay Biotechnik Produktions- und Handelsgesellschaft mbH, Allergens of Alder pollen and applications thereof)
3. WO 90/11293 (Immunologic Pharmaceutical Corporation, The University of North Carolina at Chapel Hill, Allergenic proteins from ragweed and uses thereof)
4. Takai T, Yokota T, Yasue M, Nishiyama C, Yuuki T, Mori A, Okudaira H, Okumura Y: "Engineering of the major house dust mite allergen Der f 2 for allergen-specific immunotherapy". Nat Biotechnol 15, 754-758 (1997).
5. Smith AM, Chapman MD: "Localization of antigenic sites on Der p 2 using oligonucleotide-directed mutagenesis targeted to predicted surface residues". Clin Exp Allergy 27, 593-599 (1997).
6. Aki T, Ono K, Hidaka Y, Shimonishi Y, Jyo T, Wada T, Yamashita M, Shigeta S, Murooka Y, Oka S: "Structure of IgE epitopes on a new 39-kD allergen molecule from the house dust mite, Dermatophagoides farinae". Int Arch Allergy Immunol 103, 357-364 (1994).
7. Förster E, Dudler T, Gmachl M, Aberer W, Urbanek R, Suter M: "Natural and recombinant enzymatically active or inactive bee venom phospholipase A2 has the same potency to release histamine from basophils in patients with Hymenoptera allergy". J Allergy Clin Immunol 95, 1229-1235 (1995).
8. Burks AW, Shin D, Cockrell G, Stanley JS, Helm RM, Bannon GA: "Mapping and mutational analysis of the IgE-binding epitopes on Ara h 1, a legume vicilin protein and a major allergen in peanut hypersensitivity". Eur J Biochem 245, 334-339 (1997).
9. Stanley JS, King N, Burks AW, Huang SK, Sampson H, Cockrell G, Helm RM, West CM, Bannon GA: "Identification and mutational analysis of the immunodominant IgE binding epitopes of the major peanut allergen Ara h 2". Arch Biochem Biophys 342, 244-253 (1997).
10. Ferreira F, Rohlfs A, Hoffmann-Sommergruber K, Schenk S, Ebner C, Briza P, Jilek A, Kraft D, Breitenbach M, Scheiner O: "Modulation of IgE-binding properties of tree pollen allergens by site-directed mutagenesis". Adv Exp Med Biol 409, 127-135 (1996).
11. Ferreira F, Ebner C, Kramer B, Casari G, Briza P, Kungl AJ, Grimm R, Jah-Schmid B, Breiteneder H, Kraft D, Breitenbach M, Rheinberger H-J, Scheiner O, "Modulation of IgE reactivity of allergens by site-directed mutagenesis: Potential use of hypeallergenic variants for immunotherapy", FASEB Journal for Experimental Biology Vol. 12, No. 2, February 1998, 231-242 (1998).
12. Wiedemann P, Giehl K, Almo SC, Fedorov AA, Girvin M, Steinberger P, Rüdiger M, Ortner M, Sippl M, Dolecek C, Kraft D, Jockusch B, Valenta R: "Molecular and structural analysis of a continuous birch profilin epitope defined by a monoclonal antibody". J Biol Chem 271, 29915-29921 (1996).
13. Alvarez AM, Fukuhara E, Nakase M, Adachi T, Aoki N, Nakamura R, Matsuda T: "Four rice seed cDNA clones belonging to the alpha-amylase/trypsin inhibitor gene family encode potential rice allergens". Biosci Biotechnol Biochem 59, 1304-1308 (1995).
14. Colombo P, Kennedy D, Ramsdale T, Costa MA, Djro G, Izzo V, Salvadori S, Guerrini R, Cocchiara R, Mirisola MG, Wood S, Geraci D, Journal of Immunology Vol. 160, No. 6, 15 March 1998, 2780-2875.
15. Spangfort MD, Ipsen H, Sparholt SH, Aasmul-Olsen S, Larsen MR, Mørtz E, Roepstorff P, Larsen JN: "Characterization of Purified Recombinant Bet v 1 with Authentic N-terminus, Cloned in Fusion with Maltose-Binding Protein". Prot Exp Purification 8, 365-373 (1996a).
16. Ipsen H, Wihl J-Å, Petersen BN, Løwenstein H: "Specificity mapping of patients IgE response towards the tree pollen major allergens Aln g I, Bet v I and Cor a I." Clin. Exp. Allergy 22, 391-9, (1992)
17. Gajhede M, Osmark P, Poulsen FM, Ipsen H, Larsen JN, Joost van Neerven RJ, Schou C, Løwnstein H, and Spangfort MD: "X-ray and NMR structure of Bet v 1, the origin of birch pollen allergy". Nature structural biology 3, 1040-1045 (1996).
18. Altschul SF, Gish W, Miller W, Myers EW, and Lipman DJ: "Basic local alignment search tool". J. Mol. Biol. 215, 403-410 (1990).
19. Higgins D, Thompson J, Gibson T, Thompson JD, Higgins DG, and Gibson TJ: "CLUSTAL W: improving the sensitivity of progressive multiple sequence alignment through sequence weighting, position-specific gap penalties and weight matrix choice". Nucleic Acids Res. 22, 4673-4680 (1994).
20. Saiki RK, Gelfand DH, Stoffel S, Scharf SJ, Higuchi R, Horn GT, Mullis KB, Erlich HA: "Primer-directed enzymatic amplification of DNA with a thermostable DNA polymerase". Science 239, 487-491 (1988).
21. Spangfort MD, Larsen JN, Gajhede M: "Crystallization and Preliminary X-ray Investigation at 2.0 Å Resolution of Bet v 1, a Birch Pollen Protein Causing IgE-Mediated Allergy". PROTEINS, Struc Func Genet 26, 358-360 (1996b).
22. Monsalve RI, Lu G, and King TP: "Recombinant venom allergen, antigen 5 of yellowjacket (Vespula vulgaris) and paper wasp (Polistes annularis) by expression in bacteria or yeast" (1999) Submitted.
23. Fang KSF, Vitale M, Fehlner P and King TP: "cDNA cloning and primary structure of a white-face hornet venom allergen, antigen 5". Proc. Natl. Acad. Sci. USA 85, 895 (1988).
24. Lu G, Villalba M, Coscia MR, Hoffman DR and King TP: "Sequence Analysis and Antigenic Cross-reactivity of a Venom Allergen, Antigen 5, from Hornets, Wasps, and Yellow Jackets". Journal of Immunology 150, 2823-2830 (1993).
25. Punnonen J: "Molecular Breeding of Allergy Vaccines and Antiallergic Cytokines". Int Arch Allergy Immunol 2000; 121:173-182.
26. P.A. Würtzen, M. Wissenbach, H. Ipsen, A. Bufe, J. Arnved, and R. J. J. van Neerven. J Allergy Clin Immunol, 1999; 104: 115-23.
27. Sparholt SH, Larsen JN, Ipsen H, Schou C, van Neerven RJ. Clin Exp Allergy 1997 Aug; 27(8):932-41.

## Claims

1. A recombinant allergen that is a mutant of a naturally occurring allergen, wherein the mutant allergen has at least four mutations, which each reduce the specific IgE binding capability of the mutated allergen as compared to the IgE binding capability of the said naturally occurring allergen, wherein each mutation is a substitution of one surface-exposed amino acid residue with another residue, which does not occur in the same position in the amino acid sequence of any known homologous protein within the taxonomic species from which said naturally occurring allergen originates, **characterized in that** at least four mutations (primary mutations) are mutually spaced by at least 15 Å and wherein said primary mutations are placed in such a manner that at least one circular surface region with a area of 800 Å² comprises no mutation.

2. A recombinant allergen according to claim 1, wherein the primary mutations are spaced 20 Å.

3. A recombinant allergen according to claim 1 or 2 comprising a number of secondary mutations, which each reduce the specific IgE binding capability of the mutated allergen as compared to the binding capability of the said naturally occurring allergen, wherein each secondary mutation is a substitution of one surface-exposed amino acid residue with another residue, which does not occur in the same position in the amino acid sequence of any known homologous protein within the taxonomic species from which said naturally occurring allergen originates, wherein the secondary mutations are placed outside the said circular region.

4. A recombinant allergen according to any of claims 1-3, wherein at least one of the surface-exposed amino acids to be substituted in the naturally occurring allergen has a solvent accessibility of above 20 %.

5. A recombinant allergen according to any of claims 1-4, wherein at least one of the surface-exposed amino acids to be substituted in the naturally occurring allergen is conserved with more than 70 %identity in all known homologous proteins within the species from which said naturally occurring allergen originates.

6. A recombinant allergen according to any of claims 1-5, which has essentially the same α-carbon backbone tertiary structure as said naturally occurring allergen.

7. A recombinant allergen according to any of claims 1-6, wherein each amino acid residue to be incorporated into the mutant allergen does not occur in the same position in the amino acid sequence of any known homologous protein within the taxonomic genus, such as the subfamily, the family, the superfamily, the legion, the suborder or the order from which said naturally occurring allergen originates.

8. A recombinant allergen according to any of claims 1-7, **characterised in that** the specific IgE binding to the mutated allergen is reduced by at least 5%.

9. A recombinant allergen according to claim 6, **characterised in that** when comparing the α-carbon backbone tertiary structures of the mutant and the naturally occurring allergen molecules, the average root mean square deviation of the atomic coordinates is below 2Å.

10. A recombinant allergen according to any of claim 1-9, **characterised in that** said circular surface region comprises atoms of 15-25 amino acid residues.

11. A recombinant allergen according to any one of claims 1-10, **characterised in that** the surface-exposed amino acid residues are ranked with respect to solvent accessibility, and that one or more amino acids among the more solvent accessible ones are substituted.

12. A recombinant allergen according to any one of claims 1-11, **characterised in that** the surface-exposed amino acid residues are ranked with respect to degree of conversation in all known homologous proteins within the species from which said naturally occurring allergen originates, and that one or more amino acids among the more conserved ones are substituted.

13. A recombinant allergen according to any of claims 1-12 comprising from 5 to 20 primary mutations.

14. A recombinant allergen according to any of claims 1-12 comprising from 7 to 12 primary mutations.

15. A recombinant allergen according to any one of claims 1-14 **characterised in that** the mutant allergen comprises from 1 to 4 secondary mutations per primary mutation.

16. A recombinant allergen according to any one of claims 1-15, **characterised in that** one or more of the substitutions is carried out by site-directed mutagenesis.

17. A recombinant allergen according to any one of claims 1-16, **characterised in that** one or more of the substitutions is carried out by DNA shuffling.

18. A recombinant allergen according to any one of claims 1-17 **characterised in that** it is a mutant of an inhalation allergen.

19. A recombinant allergen according to claim 18, **characterised in that** it is a mutant of a pollen allergen.

20. A recombinant allergen according to claim 19 **characterised in that** it is a mutant of a pollen allergen originating from the taxonomic order of *Fagales*, *Oleales* or *Pinales*.

21. A recombinant allergen according to claim 20, **characterised in that** it is a mutant of *Bet v* 1.

22. A recombinant allergen according to claim 21, **characterised in that** one or more of the substitutions is selected from the group consisting of V2, D72, E87, K-129, E-60, N-47, K-65, P-108, N-159, D-93, K-123, K-32, D-125, R-145, D-109, E-127, Q-36, E-131, L-152, E-6, E-96, D-156, P-63, H-76, E-8, K-134, E-45, T-10, V-12, K-20, S-155, H-126, P-50, N-78, K-119, V-2, L-24, E-42, N-4, A-153, I-44, E-138, G-61 , A-130, R-70, N-28, P-35, S-149, K-103, Y-150, H-154, N-43, A-106, K-115, P-14, Y-5, K-137, E-141, E-87 and E-73.

23. A recombinant allergen according to claim 19, **characterised in that** it is a mutant of a pollen allergen originating from the taxonomic order of *Poales*.

24. A recombinant allergen according to claim 19, **characterised in that** it is a mutant of a pollen allergen originating from the taxonomic order of *Asterales* or *Urticales*.

25. A recombinant allergen according to claim 18, **characterised in that** it is a mutant of a house dust mite allergen.

26. A recombinant allergen according to claim 25, **characterised in that** it is a mutant of a mite allergen originating from *Dermatophagoides*.

27. A recombinant allergen according to claim 26, **characterised in that** it is a mutant of Der p 2.

28. A recombinant allergen according to claim 27, **characterised in that** one or more of the substitutions is selected from the group consisting of R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 and R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 and K-15.

29. A recombinant allergen according to claim 18, **characterised in that** it is a mutant of a cockroach allergen.

30. A recombinant allergen according to claim 18, **characterised in that** it is a mutant of an animal allergen.

31. A recombinant allergen according to claim 30, **characterised in that** it is a mutant of an animal allergen originating from cat, dog or horse.

32. A recombinant allergen according to any one of claims 1-17 **characterised in that** it is a mutant of a venom allergen.

33. A recombinant allergen according to claim 32, **characterised in that** it is a mutant of a venom allergen originating from the taxonomic order of *Hymenoptera.*

34. A recombinant allergen according to claim 33, **characterised in that** is a mutant of a venom allergen from the taxonomic order of Vespidae, Apidae and Formicoidae.

35. A recombinant allergen according to any one of claims 32-34 **characterised in that** it is a mutant of *Ves v* 5.

36. A recombinant allergen according to claim 35 **characterised in that** one or more of the substitutions is selected from the group consisting of K-16, K-185, K-11, K-44, K-210, R-63, K-13, F-6, K-149, K-128, E-184, K-112, F-157, E-3, K-29, N-203, N-34, K-78, K-151, L-15, L-158, Y-102, W-186, K-134, D-87, K-52, T-67, T-125, K-150, Y-40, Q-48, L-65, K-81, Q-101, Q-208, K-144, N-8, N-70, H-104, Q-45, K-137, K-159, E-205, N-82, A-111, D-131, K-24, -V-36, N-7, M-138, T-209, V-84, K-172, V-19, D-56, P-73, G-33, T-106, N-170, L-28, T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85 and I-182.

37. A recombinant allergen according to any of claims 1-36 for use as a pharmaceutical.

38. Use of the recombinant allergen according to any of claims 1-36 for preparing a pharmaceutical for preventing and/or treating allergy.

39. A composition comprising two or more recombinant mutant allergen variants according to any of claims 1-36, wherein each variant is defined by having at least one primary mutation, which is absent in at least one of the other variants, wherein for each variant no secondary mutation is present within a radius of 15 Å from each absent primary mutation.

40. A composition according to claim 39 comprising 2-12 variants.

41. A composition according to claim 39 or 40 for use as a pharmaceutical.

42. Use of a composition according to claim 39 or 40 for preparing a pharmaceutical for preventing and/or treating allergy.

43. A pharmaceutical composition, **characterised in that** it comprises a recombinant allergen according to any one of claims 1-36 or a composition according to claim 39 or 40, in combination with a pharmaceutically acceptable carrier and/or excipient.

44. A pharmaceutical composition according to claim 43, **characterised in that** it is in the form of a vaccine against allergic reactions elicited by a naturally occurring allergen in patients suffering from allergy.

45. Use of a recombinant allergen according to any one of claims 1-36 or a composition according to claim 39 or 40 for the manufacture of a medicament for generating an immune response in a subject.

46. Use of a recombinant allergen according to any one of claims 1-36 or a composition according to claim 35 or 40 for the manufacture of a medicament for vaccination or treatment of a subject.

47. A process for preparing a pharmaceutical composition according to claim 43 or 44 comprising mixing a recombinant allergen according to any one of claims 1-36 or a composition according to claim 39 or 40 with pharmaceutically acceptable substances and/or excipients.

48. A pharmaceutical composition obtainable by the process according to claim 47.

49. Use of a recombinant allergen according to any one of claims 1-36 or a composition according to claim 39 or 40 for the manufacture of a medicament for the treatment, prevention or alleviation of allergic reactions in a subject.

50. A method of preparing a recombinant allergen according to any one of claims 1-36, **characterised in**
a) identifying a number of amino acid residues in a naturally occurring allergen, which has a solvent accessibility of at least 20 %;
b) selecting at least four of the identified amino acid residues in such a manner that each selected amino acid is spaced from each other selected amino acid by at least 15 Å, and that the selected amino acids are placed in such a manner that at least one circular surface region with a area of 800 Å² comprises no selected amino acid; and
c) effecting for each of the selected amino acids a primary mutation, which reduce the specific IgE binding capability of the mutated allergen as compared to the binding capability of the said naturally occurring allergen,
wherein each primary mutation is a substitution of a selected amino acid residue with another amino acid, which does not occur in the same position in the amino acid sequence of any known homologous protein within the taxonomic species from which said naturally occurring allergen originates.

51. A method according to claim 50, **characterised in** ranking the said identified amino acid residues with respect to solvent accessibility and substituting one or more amino acids among the more solvent accessible ones.

52. A method according to claim 50 or 51, **characterised in** selecting identified amino acid residues, which are conserved with more than 70 % identity in all known homologous proteins within the species from which said naturally occurring allergen originates.

53. A method according to claim 52, **characterised in** ranking the said identified amino acid residues with respect to degree of conversation in all known homologous proteins within the species from which said naturally occurring allergen originates and substituting one or more amino acids among the more conserved ones.

54. A method according to any of claims 50-53 comprising selecting the identified amino acids so as to form a mutant allergen, which has essentially the same α-carbon backbone tertiary structure as said naturally occurring allergen.

55. A method according to any of claims 50-54 **characterised in that** the substitution of amino acid residues is carried out by site-directed mutagenesis.

56. A method of preparing a recombinant allergen according to any one of claims 1-36, **characterised in that** the allergen is produced from a DNA sequence obtained by DNA shuffling (molecular breeding) of the DNA encoding the corresponding naturally occurring allergen.

57. A DNA sequence encoding a recombinant allergen according to any of claims 1-36, a derivative thereof, a partial sequence thereof, a degenerated sequence thereof or a sequence, which hybridises thereto under stringent conditions, wherein said derivative, partial sequence, degenerated sequence or hybridising sequence encodes a peptide having at least one B cell epitope.

58. A DNA sequence according to claim 57, which is a derivative of the DNA sequence encoding the naturally occurring allergen.

59. A DNA sequence according to claim 58, wherein the derivative is obtained by site-directed mutagenesis of the DNA encoding the naturally occurring allergen.

60. A DNA sequence according to any of claims 57-59, wherein the sequence is a derivative of the sequence shown in Fig. 3, wherein the DNA sequence is mutated so as to encode an allergen having at least four mutations selected from the group consisting of V2, D72, E87, K-129, E-60, N-47, K-65, P-108, N-159, D-93, K-123, K-32, D-125, R-145, D-109, E-127, Q-36, E-131, L-152, E-6, E-96, D-156, P-63, H-76, E-8, K-134, E-45, T-10, V-12, K-20, S-155, H-126, P-50, N-78, K-119, V-2, L-24, E-42. N-4, A-153, I-44, E-138, G-61, A-130, R-70, N-28, P-35, S-149, K-103, Y-150, H-154, N-43, A-106, K-115, P-14, Y-5, K-137, E-141, E-87 and E-73.

61. A DNA sequence according to any of claims 57-59, wherein the sequence is a derivative of the sequence shown in Fig. 13, wherein the DNA sequence is mutated so as to encode an allergen having at least four mutations selected from the group consisting of K-16, K-185, K-11, K-44, K-210, R-63, K-13, F-6, K-149, K-128, E-184, K-112, F-157, E-3, K-29, N-203, N-34, K-78, K-151, L-15, L-158, Y-102, W-186, K-134, D-87, K-52, T-67, T-125, K-150, Y-40, Q-48, L-65, K-81, Q-101, Q-208, K-144, N-8, N-70, H-104, Q-45, K-137, K-159, E-205, N-82, A-111, D-131, K-24, V-36, N-7, M-138, T-209, V-84, K-172, V-19, D-56, P-73, G-33, T-106, N-170, L-28, T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85 and I-182.

62. A DNA sequence according to any of claims 57-59, wherein the sequence is a derivative of the sequence shown in Fig. 16, wherein the DNA sequence is mutated so as to encode an allergen having at least four mutations selected from the group consisting of R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 and R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, 1-68, P-79, K-109 and K-15.

63. An expression vector comprising the DNA according to any of claims 57-62.

64. A host cell comprising the expression vector of claim 63.

65. A method of producing a recombinant mutant allergen comprising the step of cultivating the host cell according to claim 64.

66. A recombinant allergen according to any of claims 1-36 or encoded by the DNA sequence according to any of claims 57-62 comprising at least one T cell epitope capable of stimulating a T cell clone or T cell line specific for the naturally occurring allergen.

67. A diagnostic assay for assessing relevance, safety or outcome of therapy of a subject using a recombinant mutant allergen according to any of claims 1-36 or a composition according to claim 39 or 40, wherein an IgE containing sample of the subject is mixed with said mutant or said composition and assessed for the level of reactivity between the IgE in said sample and said mutant.

## Patentansprüche

1. Rekombinantes Allergen, das ein Mutant eines natürlich auftretenden Allergens ist, wobei das mutante Allergen zumindest vier Mutationen aufweist, von welchen jede die spezifische IgE-Bindungsfähigkeit des mutierten Allergens, verglichen mit der IgE-Bindungsfähigkeit des natürlich auftretenden Allergens, verringert, wobei jede Mutation eine Substitution eines an der Oberfläche exponierten Aminosäure-Rests durch einen anderen Rest ist, der in der Aminosäuresequenz irgendwelcher bekannten homologen Proteine in der taxonomischen Spezies, aus der das natürlich auftretende Allergen stammt, nicht an der selben Stelle auftritt, **dadurch gekennzeichnet, dass** zumindest vier Mutationen (primäre Mutationen) voneinander durch zumindest 15 Å beabstandet sind, und wobei diese primären Mutationen in einer solchen Weise angeordnet sind, dass zumindest eine runde Oberflächenregion mit einer Fläche von 800 Å² keine Mutation aufweist.

2. Rekombinantes Allergen nach Anspruch 1, wobei die primären Mutationen 20 Å voneinander beabstandet sind.

3. Rekombinantes Allergen nach Anspruch 1 oder 2, welches eine Anzahl sekundärer Mutationen aufweist, von welchen jede die spezifische IgE-Bindungsfähigkeit des mutierten Allergens, verglichen mit der IgE-Bindungsfähigkeit des natürlich auftretenden Allergens, verringern wobei jede sekundäre Mutation eine Substitution eines an der Oberfläche exponierten Aminosäure-Rests durch einen anderen Rest ist, der in der Aminosäuresequenz irgendwelcher bekannten homologen Proteine in der taxonomischen Spezies, aus der das natürlich auftretende Allergen stammt, nicht an der selben Stelle auftritt, wobei die sekundären Mutationen außerhalb der besagten runden Region angeordnet sind.

4. Rekombinantes Allergen nach einem der Ansprüche 1 bis 3, wobei zumindest eine der an der Oberfläche exponierten Aminosäuren, die in dem natürlich auftretenden Allergen zu substituieren sind, eine Lösungsmittel-Zugänglichkeit von über 20% aufweist.

5. Rekombinantes Allergen nach einem der Ansprüche 1 bis 4, wobei zumindest eine der an der Oberfläche exponierten Aminosäuren, die in dem natürlich auftretenden Allergen zu substituieren sind, mit einer Identität von mehr als 70 % in allen bekannten homologen Proteinen in der Spezies, aus der das natürlich auftretende Allergen stammt, konserviert ist.

6. Rekombinantes Allergen nach einem der Ansprüche 1 bis 5, welches im Wesentlichen die selbe α-Kohlenstoff-Hauptketten-Tertiärstruktur wie das natürlich auftretende Allergen aufweist.

7. Rekombinantes Allergen nach einem der Ansprüche 1 bis 6, wobei jeder Aminosäurerest, der in das mutante Allergen eingebaut werden soll, nicht an der selben Stelle in der Aminosäuresequenz irgendwelcher bekannter homologer Proteine in der taxonomischen Gattung, wie etwa der Unterfamilie, der Familie, der Überfamilie, der Legion, der Unterordnung oder der Ordnung, von der das natürlich auftretende Allergen stammt, auftritt.

8. Rekombinantes Allergen nach einem der Ansprüche 1 bis 7, **dadurch gekennzeichnet, dass** die spezifische IgE-Bindung an das mutierte Allergen um zumindest 5% verringert wird.

9. Rekombinantes Allergen nach Anspruch 6, **dadurch gekennzeichnet, dass** bei einem Vergleich der α-Kohlenstoff-Hauptketten-Tertiärstrukturen der mutanten und der natürlich auftretenden Allergenmolekülen, die durchschnittliche Abweichung des quadratischen Mittelwerts der Atomkoordinaten weniger als 2 Å beträgt.

10. Rekombinantes Allergen nach einem der Ansprüche 1 bis 9, **dadurch gekennzeichnet, dass** die besagte runde Oberflächenregion Atome von 15-25 Aminosäureresten aufweist.

11. Rekombinantes Allergen nach einem der Ansprüche 1 bis 10, **dadurch gekennzeichnet, dass** die an der Oberfläche exponierten Aminosäurereste hinsichtlich ihrer Lösungsmittel-Zugänglichkeit rangmäßig geordnet sind, und dass eine oder mehrere der Aminosäuren, die zu denen mit einer höheren Lösungsmittel-Zugänglichkeit zählen, substituiert sind.

12. Rekombinantes Allergen nach einem der Ansprüche 1 bis 11, **dadurch gekennzeichnet, dass** die an der Oberfläche exponierten Aminosäurereste hinsichtlich ihres Konservierungsgrades in allen bekannten homologen Proteinen innerhalb der Spezies, aus der das natürlich auftretende Allergen stammt, rangmäßig geordnet sind, und dass eine oder mehrere der Aminosäuren, die zu denen mit einem höheren Konservierungsgrad zählen, substituiert sind.

13. Rekombinantes Allergen nach einem der Ansprüche 1 bis 12, welches von 5 bis 20 primäre Mutationen aufweist.

14. Rekombinantes Allergen nach einem der Ansprüche 1 bis 12, welches von 7 bis 12 primäre Mutationen aufweist.

15. Rekombinantes Allergen nach einem der Ansprüche 1 bis 14, **dadurch gekennzeichnet, dass** das mutante Allergen von 1 bis 4 sekundäre Mutationen pro primärer Mutation aufweist.

16. Rekombinantes Allergen nach einem der Ansprüche 1 bis 15, **dadurch gekennzeichnet, dass** eine oder mehrere der Substitutionen durch ortsspezifische Mutagenese ausgeführt sind.

17. Rekombinantes Allergen nach einem der Ansprüche 1 bis 16, **dadurch gekennzeichnet, dass** eine oder mehrere der Substitutionen durch DNA-Shuffling ausgeführt sind.

18. Rekombinantes Allergen nach einem der Ansprüche 1 bis 17, **dadurch gekennzeichnet, dass** es ein Mutant eines Inhalations-Allergens ist.

19. Rekombinantes Allergen nach Anspruch 18, **dadurch gekennzeichnet, dass** es ein Mutant eines Pollen-Allergens ist.

20. Rekombinantes Allergen nach Anspruch 19, **dadurch gekennzeichnet, dass** es ein Mutant eines Pollen-Allergens ist, das aus der taxonomischen Ordnung der Fagales, Oleales oder Pinales stammt.

21. Rekombinantes Allergen nach Anspruch 20, **dadurch gekennzeichnet, dass** es ein Mutant von Bet v 1 ist.

22. Rekombinantes Allergen nach Anspruch 21, **dadurch gekennzeichnet, dass** eine oder mehrere der Substitutionen ausgewählt ist/sind aus der Gruppe bestehend aus V2, D72, E87, K-129, E-60, N-47, K-65, P-108, N-159, D-93, K-123, K-32, D-125, R-145, D-109, E-127, Q-36, E-131, L-152, E-6, E-96, D-156, P-63, H-76, E-8, K-134, E-45, T-10, V-12, K-20, S-155, H-126, P-50, N-78, K-119, V-2, L-24, E-42, N-4, A-153, I-44, E-138, G-61, A-130, R-70, N-28, P-35, S-149, K-103, Y-150, H-154, N-43, A-106, K-115, P-14, Y-5, K-137, E-141, E-87 und E-73.

23. Rekombinantes Allergen nach Anspruch 19, **dadurch gekennzeichnet, dass** es ein Mutant eines Pollen-Allergens ist, das aus der taxonomischen Ordnung der Poales stammt.

24. Rekombinantes Allergen nach Anspruch 19, **dadurch gekennzeichnet, dass** es ein Mutant eines Pollen-Allergens ist, das aus der taxonomischen Ordnung der Asterales oder Urticales stammt.

25. Rekombinantes Allergen nach Anspruch 18, **dadurch gekennzeichnet, dass** es ein Mutant eines Hausstaubmilben-Allergens ist.

26. Rekombinantes Allergen nach Anspruch 25, **dadurch gekennzeichnet, dass** es ein Mutant eines Milben-Allergens ist, das von Dermatophagoiden abstammt.

27. Rekombinantes Allergen nach Anspruch 26, **dadurch gekennzeichnet, dass** es ein Mutant von Der p 2 ist.

28. Rekombinantes Allergen nach Anspruch 27, **dadurch gekennzeichnet, dass** eine oder mehrere der Substitutionen ausgewählt ist/sind aus der Gruppe bestehend aus R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 und R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 und K-15

29. Rekombinantes Allergen nach Anspruch 18, **dadurch gekennzeichnet, dass** es ein Mutant eines Schaben-Allergens ist.

30. Rekombinantes Allergen nach Anspruch 18, **dadurch gekennzeichnet, dass** es ein Mutant eines Tier-Allergens ist.

31. Rekombinantes Allergen nach Anspruch 30, **dadurch gekennzeichnet, dass** es ein Mutant eines von der Katze, dem Hund oder dem Pferd stammenden Tier-Allergens ist.

32. Rekombinantes Allergen nach einem der Ansprüche 1 bis 17, **dadurch gekennzeichnet, dass** es ein Mutant eines Gift-Allergens ist.

33. Rekombinantes Allergen nach Anspruch 32, **dadurch gekennzeichnet, dass** es ein Mutant eines aus der taxonomischen Ordnung der Hymenoptera stammenden Gift-Allergens ist.

34. Rekombinantes Allergen nach Anspruch 33, **dadurch gekennzeichnet, dass** es ein Mutant eines Gift-Allergens von der taxonomischen Ordnung der Vespidae, Apidae und Formicoidae ist.

35. Rekombinantes Allergen nach einem der Ansprüche 32 bis 34, **dadurch gekennzeichnet, dass** es ein Mutant von Ves v 5 ist.

36. Rekombinantes Allergen nach Anspruch 35, **dadurch gekennzeichnet, dass** eine oder mehrere der Substitutionen ausgewählt ist/sind aus der Gruppe bestehend aus K-16, K-185, K-11, K-44, K-210, R-63, K-13, F-6, K-149, K-128, E-184, K-112, F-157, E-3, K-29, N-203, N-34, K-78, K-151, L-15, L-158, Y-102, W-186, K-134, D-87, K-52, T-67, T-125, K-150, Y-40, Q-48, L-65, K-81, Q-101, Q-208, K-144, N-8, N-70, H-104, Q-45, K-137, K-159, E-205, N-82, A-111, D-131, K-24, --V-36, N-7, M-138, T-209, V-84, K-172, V-19, D-56, P-73, G-33, T-106, N-170, L-28, T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85 und 1-182.

37. Rekombinantes Allergen nach einem der Ansprüche 1 bis 36 für die Verwendung als ein Pharmazeutikum.

38. Verwendung eines rekombinanten Allergens nach einem der Ansprüche 1 bis 36 zur Herstellung eines Pharmazeutikums zur Vorbeugung und/oder Behandlung von Allergien.

39. Zusammensetzung, welche zwei oder mehrere rekombinante mutante Allergenvarianten nach einem der Ansprüche 1 bis 36 aufweist, wobei jede Variante **dadurch** definiert ist, dass sie zumindest eine primäre Mutation, die in zumindest einer der anderen Varianten nicht vorhanden ist, aufweist, wobei für jede Variante in einem Radius von 15 Å von jeder fehlenden primären Mutation keine sekundäre Mutation vorhanden ist.

40. Zusammensetzung nach Anspruch 39, welche 2 bis 12 Varianten aufweist.

41. Zusammensetzung nach Anspruch 39 oder 40 zur Verwendung als Pharmazeutikum.

42. Verwendung einer Zusammensetzung nach Anspruch 39 oder 40 zur Herstellung eines Pharmazeutikums zur Vorbeugung und/oder Behandlung von Allergien.

43. Pharmazeutische Zusammensetzung, **dadurch gekennzeichnet, dass** sie ein rekombinantes Allergen nach einem der Ansprüche 1-36 oder eine Zusammensetzung nach Anspruch 39 oder 40 in Kombination mit einem pharmazeutisch zulässigem Träger und/oder Hilfsstoff aufweist.

44. Pharmazeutische Zusammensetzung nach Anspruch 43, **dadurch gekennzeichnet, dass** sie in der Form eines Impfstoffs gegen allergische Reaktionen, die bei an Allergien leidenden Patienten durch ein natürlich auftretendes Allergen ausgelöst werden, vorliegt.

45. Verwendung eines rekombinanten Allergens nach einem der Ansprüche 1 bis 36, oder einer Zusammensetzung nach Anspruch 39 oder 40 zur Herstellung eines Medikaments zur Erzeugung einer Immunantwort in einem Subjekt.

46. Verwendung eines rekombinanten Allergens nach einem der Ansprüche 1 bis 36, oder einer Zusammensetzung nach Anspruch 39 oder 40 zur Herstellung eines Medikaments zur Impfung oder Behandlung eines Subjekts.

47. Verfahren zur Herstellung einer pharmazeutischen Zusammensetzung nach Anspruch 43 oder 44, welches das Mischen eines rekombinanten Allergens nach einem der Ansprüche 1 bis 36, oder einer Zusammensetzung nach Anspruch 39 oder 40 mit pharmazeutisch zulässigen Substanzen und/oder Hilfsstoffen aufweist.

48. Pharmazeutische Zusammensetzung, die durch das Verfahren gemäß Anspruch 47 erhältlich ist.

49. Verwendung eines rekombinanten Allergens nach einem der Ansprüche 1 bis 36, oder einer Zusammensetzung nach Anspruch 39 oder 40 zur Herstellung eines Medikaments zur Behandlung, Vorbeugung oder Linderung allergischer Reaktionen in einem Subjekt.

50. Verfahren zur Herstellung eines rekombinanten Allergens nach einem der Ansprüche 1 bis 36, **gekennzeichnet durch**:
a. Identifizieren einer Anzahl an Aminosäure-Resten in einem natürlich auftretenden Allergen, das eine Lösungsmittel-Zugänglichkeit von zumindest 20% aufweist;
b. Auswählen von zumindest vier der identifizierten Aminosäureresten in solcher Weise, dass jede ausgewählte Aminosäure von jeder anderen ausgewählten Aminosäure **durch** zumindest 15 Å beabstandet ist, und dass die ausgewählten Aminosäuren in solcher Weise angeordnet sind, dass zumindest eine runde Oberflächenregion mit einer Fläche von 800 Å² keine ausgewählten Aminosäuren aufweist; und
c. Bewirken für jede der ausgewählten Aminosäuren eine primäre Mutation, welche die spezifische IgE-Bindungsfähigkeit des mutierten Allergens, verglichen mit der Bindungsfähigkeit des natürlich auftretenden Allergens, verringert, wobei jede primäre Mutation eine Substitution eines ausgewählten Aminosäure-Rests **durch** eine andere Aminosäure ist, die in der Aminosäuresequenz irgendwelcher bekannten homologen Proteine in der taxonomischen Spezies, aus der das natürlich auftretende Allergen stammt, nicht an der selben Stelle auftritt.

51. Verfahren nach Anspruch 50, **gekennzeichnet durch** ein rangordnungsmäßiges Reihen der identifizierten Aminosäurereste hinsichtlich der Lösungsmittel-Zugänglichkeit und Substituieren einer oder mehrerer Aminosäuren, die zu denen mit einer höheren Lösungsmittel-Zugänglichkeit zählen.

52. Verfahren nach Anspruch 50 oder 51, **gekennzeichnet durch** ein Auswählen identifizierter Aminosäurereste, die mit einer Identität von mehr als 70 % in allen bekannten homologen Proteinen in der Spezies, aus der das natürlich auftretende Allergen stammt, konserviert sind.

53. Verfahren nach Anspruch 52, **gekennzeichnet durch** ein Reihen der identifizierten Aminosäurereste hinsichtlich ihres Konservierungsgrades in allen bekannten homologen Proteinen innerhalb der Spezies, aus der das natürlich auftretende Allergen stammt, und Substituieren von einer oder mehreren der Aminosäuren, die zu denen mit einem höheren Konservierungsgrad zählen.

54. Verfahren nach einem der Ansprüche 50 bis 53, welches beinhaltet, die identifizierten Aminosäuren so auszuwählen, dass ein mutantes Allergen gebildet wird, das im Wesentlichen die gleiche α-Kohlenstoff-Hauptketten-Tertiärstruktur wie das natürlich auftretende Allergen aufweist.

55. Verfahren nach einem der Ansprüche 50 bis 54, **dadurch gekennzeichnet, dass** die Substitution von Aminosäureresten durch eine ortsspezifische Mutagenese durchgeführt wird.

56. Verfahren zur Herstellung eines rekombinanten Allergens nach einem der Ansprüche 1 bis 36, **dadurch gekennzeichnet, dass** das Allergen aus einer DNA-Sequenz hergestellt wird, die durch DNA-Shuffling (molekulares Züchten) der DNA, welche das entsprechende natürlich auftretende Allergen kodiert, erhalten wird.

57. DNA-Sequenz, die ein rekombinantes Allergen nach einem der Ansprüche 1 bis 36 kodiert, ein Derivat davon, eine Teilsequenz davon, eine degenerierte Sequenz davon oder eine Sequenz, welche unter stringenten Bedingungen dazu hybridisiert, wobei das Derivat, die Teilsequenz, die degenerierte Sequenz oder die hybridisierende Sequenz ein Peptid kodiert, welches zumindest ein B-Zellen-Epitop aufweist.

58. DNA-Sequenz nach Anspruch 57, die ein Derivat der DNA-Sequenz ist, welche das natürlich auftretende Allergen kodiert.

59. DNA-Sequenz nach Anspruch 58, wobei das Derivat durch ortsspezifische Mutagenese der DNA erhalten ist, die das natürlich auftretende Allergen kodiert.

60. DNA-Sequenz nach einem der Ansprüche 57 bis 59, wobei die Sequenz ein Derivat der in Fig. 3 gezeigten Sequenz ist, wobei die DNA-Sequenz so mutiert ist, dass sie ein Allergen kodiert, welches zumindest vier Mutationen aufweist, die ausgewählt sind aus der Gruppe, bestehend aus V2, D72, E87, K-129, E-60, N-47, K-65, P-108, N-159, D-93, K-123, K-32, D-125, R-145, D-109, E-127, Q-36, E-131, L-152, E-6, E-96, D-156, P-63, H-76, E-8, K-134, E-45, T-10, V-12, K-20, S-155, H-126, P-50, N-78, K-119, V-2, L-24, E-42, N-4, A-153, 1-44, E-138, G-61, A-130, R-70, N-28, P-35, S-149, K-103, Y-150, H-154, N-43, A-106, K-115, P-14, Y-5, K-137, E-141, E-87 und E-73

61. DNA-Sequenz nach einem der Ansprüche 57 bis 59, wobei die Sequenz ein Derivat der in Fig. 13 gezeigten Sequenz ist, wobei die DNA-Sequenz so mutiert ist, dass sie ein Allergen kodiert, welches zumindest vier Mutationen aufweist, die ausgewählt sind aus der Gruppe, bestehend aus K-16, K-185, K-11, K-44, K-210, R-63, K-13, F-6, K-149, K-128, E-184, K-112, F-157, E-3, K-29, N-203, N-34, K-78, K-151, L-15, L-158, Y-102, W-186, K-134, D-87, K-52, T-67, T-125, K-150, Y-40, Q-48, L-65, K-81, Q-101, Q-208, K-144, N-8, N-70, H-104, Q-45, K-137, K-159, E-205, N-82, A-111, D-131, K-24, V-36, N-7, M-138, T-209, V-84, K-172, V-19, D-56, P-73, G-33, T-106, N-170, L-28, T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85 und I-182

62. DNA-Sequenz nach einem der Ansprüche 57 bis 59, wobei die Sequenz ein Derivat der in Fig. 16 gezeigten Sequenz ist, wobei die DNA-Sequenz so mutiert ist, dass sie ein Allergen kodiert, welches zumindest vier Mutationen aufweist, die ausgewählt sind aus der Gruppe, bestehend aus R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 und R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, 1-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 und K-15.

63. Expressionsvektor, der die DNA nach einem der Ansprüche 57 bis 62 aufweist.

64. Wirtszelle, die den Expressionsvektor nach Anspruch 63 aufweist.

65. Verfahren zur Herstellung eines rekombinanten mutanten Allergens, welches Verfahren den Schritt aufweist, die Wirtszelle nach Anspruch 64 zu kultivieren.

66. Rekombinantes Allergen nach einem der Ansprüche 1 bis 36, oder eines, das durch die DNA-Sequenz nach einem der Ansprüche 57-62 kodiert wird, welches zumindest ein T-Zellen-Epitop aufweist, das in der Lage ist, einen T-Zellen-Klon oder eine T-Zelllinie, die für das natürlich auftretende Allergen spezifisch sind, zu stimulieren.

67. Diagnostische Untersuchung zur Untersuchung der Relevanz, Sicherheit oder des Ausgangs einer Therapie an einem Subjekt, unter Verwendung eines rekombinanten, mutanten Allergens nach einem der Ansprüche 1 bis 36 oder einer Zusammensetzung nach Anspruch 39 oder 40, wobei eine IgE enthaltende Probe des Subjekts mit dem Mutanten oder der Zusammensetzung gemischt wird, und das Reaktivitätsniveau zwischen dem IgE in der Probe und dem Mutanten untersucht wird.

## Revendications

1. Allergène recombiné qui est un mutant d'un allergène d'origine naturelle, dans lequel l'allergène mutant a au moins quatre mutations, dont chacune réduit la capacité de liaison à l'IgE spécifique de l'allergène muté quand on compare à la capacité de liaison à l'IgE, dudit allergène d'origine naturelle, dans lequel chaque mutation est une substitution d'un résidu d'acide aminé exposé à la surface par un autre résidu, qui n'apparaît pas à la même position dans la séquence des acides aminés d'une quelconque protéine homologue connue au sein de l'espèce taxonomique dont ledit allergène d'origine naturelle provient, **caractérisé en ce qu'**au moins quatre mutations (mutations primaires) sont mutuellement espacées d'au moins 15 Å et où lesdites mutations primaires sont placées de telle manière qu'au moins une région de surface circulaire avec une aire de 800 Å² ne comprenne pas de mutation.

2. Allergène recombiné selon la revendication 1, dans lequel les mutations primaires sont espacées de 20 Å.

3. Allergène recombiné selon la revendication 1 ou 2 comprenant un nombre de mutations secondaires, dont chacune réduit la capacité de liaison à l'IgE spécifique de l'allergène muté quand on compare à la capacité de liaison dudit allergène d'origine naturelle, dans lequel chaque mutation secondaire est une substitution d'un résidu d'acide aminé exposé à la surface par un autre résidu, qui n'apparaît pas à la même position dans la séquence des acides aminés d'une quelconque protéine homologue connue au sein de l'espèce taxonomique dont ledit allergène d'origine naturelle provient, où les mutations secondaires sont placées en dehors de ladite région circulaire.

4. Allergène recombiné selon l'une quelconque des revendications 1-3, dans lequel au moins un des acides aminés exposés à la surface qui doit être remplacé dans l'allergène d'origine naturelle a une accessibilité au solvant de plus de 20%.

5. Allergène recombiné selon l'une quelconque des revendications 1-4, dans lequel au moins un des acides aminés exposés à la surface qui doit être remplacé dans l'allergène d'origine naturelle est conservé avec plus de 70% d'identité dans toutes les protéines homologues connues au sein de l'espèce dont l'allergène d'origine naturelle provient.

6. Allergène recombiné selon l'une quelconque des revendications 1-5, qui a essentiellement la même structure tertiaire du squelette d'alpha-carbone que ledit allergène d'origine naturelle.

7. Allergène recombiné selon l'une quelconque des revendications 1-6, dans lequel chaque résidu d'acide aminé qui doit être incorporé dans l'allergène mutant n'apparaît pas à la même position dans la séquence des acides aminés d'une quelconque protéine homologue connue au sein du genre taxonomique, comme la sous-famille, la famille, la superfamille, la légion, le sous-ordre ou l'ordre dont ledit allergène d'origine naturelle provient.

8. Allergène recombiné selon l'une quelconque des revendications 1-7, **caractérisé en que** la liaison de l'IgE spécifique à l'allergène muté est réduite d'au moins 5%.

9. Allergène recombiné selon la revendication 6, **caractérisé en ce que** quand on compare les structures tertiaires du squelette d'alpha-carbone du mutant et les molécules d'allergène d'origine naturelle, la racine carrée moyenne de la déviation des coordonnées atomiques est inférieure à 2 Å.

10. Allergène recombiné selon l'une quelconque des revendications 1-9, **caractérisé en ce que** ladite région de surface circulaire comprend des atomes de 15-25 résidus des acides aminés.

11. Allergène recombiné selon l'une quelconque des revendications 1-10, **caractérisé en ce que** les résidus d'acide aminé exposés à la surface sont classés par rapport à l'accessibilité au solvant, et qu'un ou plusieurs acides aminés parmi ceux les plus accessibles au solvant sont substitués.

12. Allergène recombiné selon l'une quelconque des revendications 1-11, **caractérisé en ce que** les résidus d'acide aminé exposés à la surface sont classés par rapport au degré de conservation dans toutes les protéines homologues connues au sein de l'espèce dont l'allergène d'origine naturelle provient, et qu'un ou plusieurs acides aminés parmi ceux les plus conservés sont substitués.

13. Allergène recombiné selon l'une quelconque des revendications 1-12 comprenant de 5 à 20 mutations primaires.

14. Allergène recombiné selon l'une quelconque des revendications 1-12 comprenant de 7 à 12 mutations primaires.

15. Allergène recombine selon l'une quelconque des revendications 1-14 **caractérisé en ce que** l'allergène mutant comprend de 1 à 4 mutations secondaires par mutation primaire.

16. Allergène recombiné selon l'une quelconque des revendications 1-15, **caractérisé en ce qu'**une ou plusieurs des substitutions sont effectuées par mutagénèse dirigée.

17. Allergène recombiné selon l'une quelconque des revendications 1-16, **caractérisé en ce qu'**une ou plusieurs des substitutions sont effectuées par réarrangement d'ADN.

18. Allergène recombiné selon l'une quelconque des revendications 1-17, **caractérisé en ce que** c'est un mutant d'un allergène d'inhalation.

19. Allergène recombiné selon la revendication 18, **caractérisé en ce que** c'est un mutant d'un allergène de pollen.

20. Allergène recombiné selon la revendication 19, **caractérisé en ce que** c'est un mutant d'un allergène de pollen provenant de l'ordre taxonomique des *Fagales,* des *Oleales* ou des *Pinales*.

21. Allergène recombiné selon la revendication 20, **caractérisé en ce que** c'est un mutant de *Bet v* 1.

22. Allergène recombiné selon la revendication 21, **caractérisé en ce qu'**une ou plusieurs des substitutions sont choisies parmi le groupe constitué de V-2, D-72, E-87, K-129, E-60, N-47, K-65, P-108, N-159, D-93, K-123, K-32, D-125, R-145, D-109, E-127, Q-36, E-131, L-152, E-6, E-96, D-156, P-63, H-76, E-8, K-134, E-45, T-10, V-12, K-20, S-155, H-126, P-50, N-78, K-119, V-2, L-24, E-42, N-4, A-153, 1-44, E-138, G-61, A-130, R-70, N-28, P-35, S-149, K-103, Y-150, H-154, N-43, A-106, K-115, P-14, Y-5, K-137, E-141, E-87 et E-73.

23. Allergène recombiné selon la revendication 19, **caractérisé en ce que** c'est un mutant d'un allergène de pollen provenant de l'ordre taxonomique des *Poales*.

24. Allergène recombiné selon la revendication 19, **caractérisé en ce que** c'est un mutant d'un allergène de pollen provenant de l'ordre taxonomique des *Asterales* ou des *Urticales*.

25. Allergène recombiné selon la revendication 18, **caractérisé en ce que** c'est un mutant d'un allergène d'acarien de poussières de maison.

26. Allergène recombiné selon la revendication 25, **caractérisé en ce que** c'est un mutant d'un allergène d'acarien provenant des *Dermatophagoides*.

27. Allergène recombiné selon la revendication 26, **caractérisé en ce que** c'est un mutant de *Der p* 2.

28. Allergène recombiné selon la revendication 27, **caractérisé en ce qu'**une ou plusieurs des substitutions sont choisies parmi le groupe constitué de R-128, D-129, H-11, H-30, S-1, K-77, Y-75, R-31, K-82, K-6, K-96, K-48, K-55, K-89, Q-85, W-92, 1-97, H-22, V-65, S-24, H-74, K-126, L-61, P-26, N-93, D-64, 1-28, K-14, K-100, E-62, 1-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 et R-128, D-129, H-11, H-30, S-1, K-77, Y-75, R-31, K-82, K-6, K-96, K-48, K-55, K-89, Q-85, W-92, 1-97, H-22, V-65, S-24, H-74, K-126, L-61, P-26, N-93, D-64, 1-28, K-14, K-100, E-62, 1-127, E-102, E-25, P-66, L-17, G-60, P-95, E-53, V-81, K-51, N-103, Q-2, N-46, E-42, T-91, D-87, N-10, M-111, C-8, H-124, 1-68, P-79, K-109 et K-15.

29. Allergène recombiné selon la revendication 18, **caractérisé en ce que** c'est un mutant d'un allergène de blatte.

30. Allergène recombiné selon la revendication 18, **caractérisé en ce que** c'est un mutant d'un allergène d'animal.

31. Allergène recombiné selon la revendication 30, **caractérisé en ce que** c'est un mutant d'un allergène d'animal provenant d'un chat, d'un chien ou d'un cheval.

32. Allergène recombiné selon l'une quelconque des revendications 1-17, **caractérisé en ce que** c'est un mutant d'un allergène de venin.

33. Allergène recombiné selon la revendication 32, **caractérisé en ce que** c'est un mutant d'un allergène de venin provenant de l'ordre taxonomique des *Hymenoptera*.

34. Allergène recombiné selon la revendication 33, **caractérisé en ce que** c'est un mutant d'un allergène de venin provenant de l'ordre taxonomique des *Vespidae*, des *Apidae* et des *Formicoidae*.

35. Allergène recombiné selon l'une quelconque des revendications 32-34 **caractérisé en ce que** c'est un mutant de *Ves v* 5.

36. Allergène recombiné selon la revendication 35 **caractérisé en ce qu'**une ou plusieurs des substitutions sont choisies parmi le groupe constitué de K-16, K-185, K-11, K-44, K-210, R-63, K-13, F-6, K-149, K-128, E-184, K-112, F-157, F-3, K-29, N-203, N-34, K-78, K-151, L-15, L-158, Y-102, W-186, K-134, D-87, K-52, T-67, T-125, K-150, Y-40, Q-48, L-65, K-81, Q-101, Q-208, K-144, N-8, N-70, H-104, Q-45, K-137, K-159, E-205, N-82, A-111, D-131, K-24, V-36, N-7, M-138, T-209, V-84, K-172, V-19, D-56, P-73, G-33, T-106, N-170, L-28, T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85 et 1-182.

37. Allergène recombiné selon l'une quelconque des revendications 1-36 pour l'utilisation comme produit pharmaceutique.

38. Utilisation de l'allergène recombiné selon l'une quelconque des revendications 1-36 pour préparer un produit pharmaceutique pour prévenir et/ou traiter l'allergie.

39. Composition comprenant deux variants d'allergènes mutants recombinés ou plus selon l'une quelconque des revendications 1-36, dans laquelle chaque variant est défini comme ayant au moins une mutation primaire, qui est absente dans au moins un des autres variants, où pour chaque variant il n'y a pas de mutation secondaire présente dans un rayon de 15 Å de chaque mutation primaire absente.

40. Composition selon la revendication 39 comprenant 2-12 variants.

41. Composition selon les revendications 39 ou 40 pour l'utilisation comme produit pharmaceutique.

42. Utilisation d'une composition selon les revendications 39 ou 40 pour préparer un produit pharmaceutique pour prévenir et/ou traiter l'allergie.

43. Composition pharmaceutique, **caractérisée en ce qu'**elle comprend un allergène recombiné selon l'une quelconque des revendications 1-36 ou une composition selon les revendications 39 ou 40, en combinaison avec un support et/ou un excipient pharmaceutiquement acceptable.

44. Composition pharmaceutique selon la revendication 43, **caractérisée en ce qu'**elle est sous la forme d'un vaccin contre les réactions allergiques provoquée par un allergène d'origine naturelle chez des patients souffrant d'allergie.

45. Utilisation d'un allergène recombiné selon l'une quelconque des revendications 1-36 ou d'une composition selon les revendications 39 ou 40 pour la fabrication d'un médicament générant une réaction immunitaire chez un sujet.

46. Utilisation d'un allergène recombiné selon l'une quelconque des revendications 1-36 ou d'une composition selon les revendications 39 ou 40 pour la fabrication d'un médicament pour la vaccination ou le traitement d'un sujet.

47. Procédé de préparation d'une composition pharmaceutique selon les revendications 43 ou 44 comprenant le mélange d'un allergène recombiné selon l'une quelconque des revendications 1-36 ou d'une composition selon les revendications 39 ou 40 avec des substances et/ou des excipients pharmaceutiquement acceptables.

48. Composition pharmaceutique qu'on peut obtenir par le procédé selon la revendication 47.

49. Utilisation d'un allergène recombiné selon l'une quelconque des revendications 1-36 ou d'une composition selon les revendications 39 ou 40 pour la fabrication d'un médicament pour le traitement, la prévention ou le soulagement de réactions allergiques chez un sujet.

50. Procédé de préparation d'un allergène recombiné selon l'une quelconque des revendications 1-36, **caractérisé par**
a) l'identification d'un nombre de résidus d'acide aminé dans un allergène d'origine naturelle, qui a une accessibilité au solvant d'au moins 20% ;
b) la sélection d'au moins quatre des résidus d'acide aminé identifiés de telle sorte que chaque acide aminé choisi soit espacé de chaque autre acide aminé choisi d'au moins 15 Å, et que les acides aminés choisis soient placés de telle sorte qu'au moins une région de surface circulaire avec une aire de 800 Å² ne comprenne pas d'acide aminé choisi ; et
c) la réalisation pour chacun des acides aminés choisis d'une mutation primaire, qui réduit la capacité de liaison à l'IgE spécifique de l'allergène muté quand on compare à la capacité de liaison dudit allergène d'origine naturelle, où chaque mutation primaire est une substitution d'un résidu d'acide aminé choisi par un autre acide aminé, qui n'apparaît pas à la même position dans la séquence des acides aminés de toute protéine homologue connue au sein de l'espèce taxonomique dont ledit allergène d'origine naturelle provient.

51. Procédé selon la revendication 50, **caractérisé par** le classement desdits résidus d'acide aminé identifiés en ce qui concerne l'accessibilité au solvant et par la substitution d'un ou plusieurs acides aminés parmi ceux les plus accessibles au solvant.

52. Procédé selon les revendications 50 ou 51, **caractérisé par** la sélection des résidus d'acide aminé identifiés, qui sont conservés avec plus de 70% d'identité dans toutes les protéines homologues connues dans l'espèce dont ledit allergène d'origine naturelle provient.

53. Procédé selon la revendication 52, **caractérisé par** le classement desdits résidus d'acide aminé en ce qui concerne le degré de conservation dans toutes les protéines homologues connues dans l'espèce dont l'allergène d'origine naturelle provient et par la substitution d'un ou plusieurs acides aminés parmi ceux les plus conservés.

54. Procédé selon l'une quelconque des revendications 50-53 comprenant la sélection des acides aminés identifiés de manière à former un allergène mutant, qui a essentiellement la même structure tertiaire du squelette α-carbone que ledit allergène d'origine naturelle.

55. Procédé selon l'une quelconque des revendications 50-54 **caractérisé en ce que** la substitution des résidus d'acide aminé est effectuée par mutagénèse dirigée.

56. Procédé de préparation d'un allergène recombiné selon l'une quelconque des revendications 1-36, **caractérisé en ce que** l'allergène est produit à partir d'une séquence d'ADN obtenue par réarrangement d'ADN (amélioration génétique moléculaire) de l'ADN codant l'allergène d'origine naturelle.

57. Séquence d'ADN codant un allergène recombiné selon l'une quelconque des revendications 1-36, un dérivé de celui-ci, une séquence partielle de celui-ci, une séquence dégénérée de celui-ci ou une séquence, qui s'hybride sous des conditions stringentes, où ledit dérivé, séquence partielle, séquence dégénérée ou séquence hybridant code un peptide ayant au moins un épitope de cellule B.

58. Séquence d'ADN selon la revendication 57, qui est un dérivé de la séquence d'ADN codant l'allergène d'origine naturelle.

59. Séquence d'ADN selon la revendication 58, où le dérivé est obtenu par mutagénèse dirigée de l'ADN codant l'allergène d'origine naturelle.

60. Séquence d'ADN selon l'une quelconque des revendications 57-59, dans laquelle la séquence est un dérivé de la séquence montrée dans la figure 3, où la séquence d'ADN est mutée de manière à coder un allergène ayant au moins quatre mutations choisies parmi le groupe constitué de V-2, D-72, E-87, K-129, E-60, N-47, K-65, P-108, N-159, D-93, K-123, K-32, D-125, R-145, D-109, E-127, Q-36, E-131, L-152, E-6, E-96, D-156, P-63, H-76, E-8, K-134, E-45, T-10, V-12, K-20, S-155, H-126, P-50, N-78, K-119, V-2, L-24, E-42, N-4, A-153, I-44, E-138, G-61, A-130, R-70, N-28, P-35, S-149, K-103, Y-150, H-154, N-43, A-106, K-115, P-14, Y-5, K-137, E-141, E-87 et E-73.

61. Séquence d'ADN selon l'une quelconque des revendications 57-59, dans laquelle la séquence est un dérivé de la séquence montrée dans la figure 13, où la séquence d'ADN est mutée de manière à coder un allergène ayant au moins quatre mutations choisies parmi le groupe constitué de K-16, K-185, K-11, K-44, K-210, R-63, K-13, F-6, K-149, K-128, E-184, K-112, F-157, E-3, K-29, N-203, N-34, K-78, K-151, L-15, L-158, Y-102, W-186, K-134, D-87, K-52, T-67, T-125, K-150, Y-40, Q-48, L-65, K-81, Q-101, Q-208, K-144, N-8, 14-70, H-104, Q-45, K-137, K-159, E-205, N-82, A-111, D-131, K-24, V-36, N-7, M-138, T-209, V-84, K-172, V-19, D-56, P-73, G-33, T-106, N-170, L-28, T-43, Q-114, C-10, K-60, N-31, K-47, E-5, D-145, V-38, A-127, D-156, E-204, P-71, G-26, Y-129, D-141, F-201, R-68, N-200, D-49, S-153, K-35, S-39, Y-25, V-37, G-18, W-85 et I-182.

62. Séquence d'ADN selon l'une quelconque des revendications 57-59, dans laquelle la séquence est un dérivé de la séquence montrée dans la figure 16, où la séquence d'ADN est mutée de manière à coder un allergène ayant au moins quatre mutations choisies parmi le groupe constitué de R-128, D-129, H-11, H-30, S-1, K-77, Y-75, R-31, K-82, K-6, K-96, K-48, K-55, K-89, Q-85, W-92, I-97, H-22, V-65, S-24, H-74, K-126, L-61, P-26, N-93, D-64, I-28, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 et R-128, D-129, H-11, H-30, S-1, K-77, Y-75, R-31, K-82, K-6, K-96, K-48, K-55, K-89, Q-85, W-92, 1-97, H-22, V-65, S-24, H-74, K-126, L-61, P-26, N-93, D-64, I-2.8, K-14, K-100, E-62, I-127, E-102, E-25, P-66, L-17, G-60, P-95, E-53, V-81, K-51, N-103, Q-2, N-46, E-42, T-91, D-87, N-10, M-111, C-8, H-124, I-68, P-79, K-109 et K-15.

63. Vecteur d'expression comprenant l'ADN selon l'une quelconque des revendications 57-62.

64. Cellule hôte comprenant le vecteur d'expression selon la revendication 63.

65. Procédé de production d'un allergène mutant recombiné comprenant l'étape de culture de la cellule hôte selon la revendication 64.

66. Allergène recombiné selon l'une quelconque des revendications 1-36 ou codé par la séquence d'ADN selon l'une quelconque des revendications 57-62 comprenant au moins un épitope de cellule T capable de stimuler un clone de cellule T ou une lignée de cellule T spécifique de l'allergène d'origine naturelle.

67. Test de diagnostic pour évaluer la pertinence, la sureté ou le résultat du traitement d'un sujet utilisant un allergène mutant recombiné selon l'une quelconque des revendications 1-36 ou une composition selon les revendications 39 ou 40, où un échantillon contenant l'IgE du sujet est mélangé avec ledit mutant ou ladite composition et évalué quant au niveau de réactivité entre l'IgE dans ledit échantillon et ledit mutant.
